Europäisches Patentamt
European Patent Office
Office européen des brevets

(19)

(11) Publication number: **0 318 093**
**A2**

(12) **EUROPEAN PATENT APPLICATION**

(21) Application number: **88202606.5**

(22) Date of filing: **21.11.88**

(51) Int. Cl.[4]: **C07D 215/18 , C07D 401/12 , C07D 409/10 , A61K 31/47**

The title of the invention has been amended (Guidelines for Examination in the EPO, A-III, 7.3).

(30) Priority: **25.11.87 US 125050**

(43) Date of publication of application: **31.05.89 Bulletin 89/22**

(84) Designated Contracting States: **AT BE CH DE ES FR GB GR IT LI LU NL SE**

(71) Applicant: **MERCK FROSST CANADA INC.**
**16711 Trans-Canada Highway**
**Kirkland Quebec(CA)**

(72) Inventor: **Young, Robert N.**
**216 Senneville Road**
**Senneville Quebec H0X 3L2(CA)**
Inventor: **Zamboni, Robert**
**233 boul. Jacques Cartier Est**
**Longueuil Quebec J4L 1E1(CA)**
Inventor: **Gauthier, Jacques Y.**
**540 Odette Oligny**
**Apt. 2, Laval Quebec H7N 5X3(CA)**
Inventor: **Belley, Michel L.**
**175 Deguire Blvd.**
**Apt. 911 St. Laurent H4N 1P1(CA)**

(74) Representative: **Hesketh, Alan, Dr.**
**European Patent Department Merck & Co., Inc. Terlings Park Eastwick Road**
**Harlow Essex, CM20 2QR(GB)**

(54) **Diarylquinoline diacids and their use as medicaments.**

(57) Compounds having the formula:

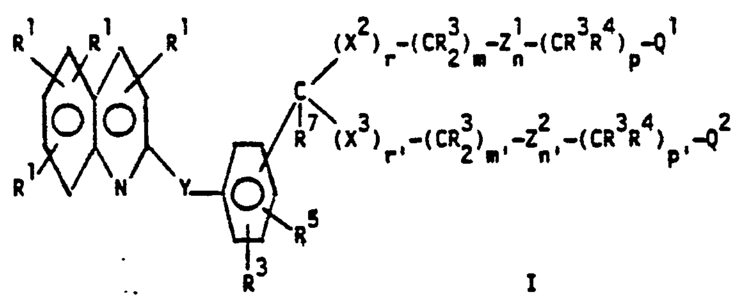

I

are leukotriene antagonists and nhibtors of leukotriene biosynthesis. These compounds are useful as anti-asthmatic anti-allergic, anti-inflammatory and cytoprotective agents.

EP 0 318 093 A2

# DIARYLSTYRYLQUINOLINE DIACIDS

## BACKGROUND OF THE INVENTION

The leukotrienes and their biological activities, especially their roles in various disease states and conditions have been described. For example, see U.S.P. 4,683,325 (July 28, 1987), which is incorporated herein by reference.

Several classes of compounds exhibit ability to antagonize the action of leukotrienes in mammals, especially humans. See for example: UK 2,058,785 and 2,094,301; and EP 56,172, 61,800, and 68,739.

EP 110,405 (June 13, 1984) describes anti-inflammatory and antiallergic substituted benzenes which are disclosed to be leukotriene inhibitors, i.e., inhibitors of the 5-lipoxygenase pathway.

## SUMMARY OF THE INVENTION

The present invention relates to compounds having activity as leukotriene and SRS-A antagonists or inhibitors of the biosynthesis of the leukotrienes, to methods for their preparation, to intermediates useful in their preparation and to methods and pharmaceutical formulations for using these compounds in mammals (especially humans).

Because of their activity as leukotriene antagonists or biosynthesis inhibitors, the compounds of the present invention are useful as anti-asthmatic, anti-allergic, and anti-inflammatory agents and are useful in treating allergic rhinitis and chronic bronchitis and for amelioration of skin diseases like psoriasis and atopic eczema. These compounds are also useful to antagonize or inhibit the pathologic actions of leukotrienes on the cardiovascular and vascular systems for example, actions such as result in angina. The compounds of the present invention are useful in the treatment of inflammatory and allergic diseases of the eye, including allergic conjunctivitis. The compounds are also useful as cytoprotective agents.

Thus, the compounds of the present invention may also be used to treat or prevent mammalian (especially, human) disease states such as erosive gastritis; erosive esophagitis; inflammatory bowel disease; ethanol induced hemorrhagic erosions; hepatic ischemic; noxious agent induced damage or necrosis of hepatic, pancreatic, renal, or myocardial tissue; liver parenchymal damage caused by hepatoxic agents such as $CCl_4$ and D-galactosamine; ischemic renal failure; disease induced hepatic damage; bile salt induced pancreatic or gastric damage; trauma- or stress-induced cell damage; and glycerol-induced renal failure.

## DETAILED DESCRIPTION

The compounds of this invention are best realized by Formula I:

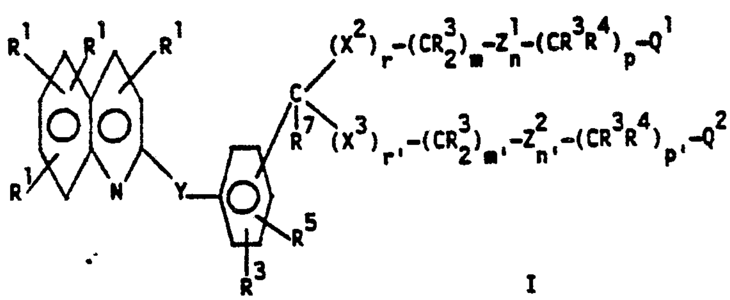

I

wherein:

$R^1$ is H, halogen, $C_1$-$C_8$ alkyl, $C_2$-$C_8$ alkenyl, $C_2$-$C_8$ alkynyl, $-CF_3$, $-SR^2$, $-S(O)R^2$, $-S(O)2R^2$, $-OR^3$, $-COOR^3$, $-(C=O)R^3$, $-C(OH)R^3R^3$, -CN, $-NO_2$, $-N_3$, substituted or unsubstituted phenyl, substituted or unsubstituted benzyl, substituted or unsubstituted 2-phenethyl, or substituted or unsubstituted pyridyl;

$R^2$ is $C_1$-$C_8$ alkyl, $C_2$-$C_8$ alkenyl, $C_2$-$C_8$ alkynyl, $-CF_3$, substituted or unsubstituted phenyl, substituted or unsubstituted benzyl, or substituted or unsubstituted 2-phenethyl;

$R^3$ is H or $R^2$;
$R^4$ is H, halogen, $-NO_2$, -CN, $-OR^3$, $SR^3$, $NR^3R^3$, or $C_1$-$C_8$ alkyl;
$CR^3R^4$ may be the radical of a naturally occurring amino acid;
$R^5$ is H, halogen, $-NO_2$, $N_3$, -CN, $-SR^2$, $-NR^3R^3$, $-OR^3$, $C_1$-$C_8$ alkyl, or $-(C=O)R^3$;
$R^6$ is $-(CH_2)_s-C(R^7R^7)-(CH_2)_s-R^8$ or $-CH_2CONR^{12}R^{12}$;
$R^7$ is H or $C_1$-$C_4$ alkyl;
$R^8$ is

A) a monocyclic or bicyclic heterocyclic radical containing from 3 to 12 nuclear carbon atoms and 1 or 2 nuclear heteroatoms selected from N, S or O and with each ring in the heterocyclic radical being formed of 5 or 6 atoms, or

B) the radical $W-R^9$;

$R^9$ contains up to 21 carbon atoms and is (1) a hydrocarbon radical or (2) an acyl radical of an organic acyclic or monocyclic carboxylic acid containing not more than 1 heteroatom in the ring;
$R^{10}$ is $-SR^{11}$, $-OR^{12}$, or $-NR^{12}R^{12}$;
$R^{11}$ is $C_1$-$C_6$ alkyl, $-(C=O)R^{14}$, unsubstituted phenyl, or unsubstituted benzyl;
$R^{12}$ is H, $R^{11}$, or two $R^{12}$ groups joined to the same N may form a ring of 5 or 6 members containing up to two heteroatoms chosen from O, S or N;
$R^{13}$ is $C_1$-$C_8$ alkyl, $C_2$-$C_8$ alkenyl, $C_2$-$C_8$ alkynyl, $-CF_3$, or unsubstituted phenyl, benzyl, or 2-phenethyl;
$R^{14}$ is H or $R^{13}$;
$R^{15}$ is $R^3$ or halogen;
$R^{16}$ is H, $C_1$-$C_4$ alkyl, or OH;
m and m′ are independently 0-8;
n and n′ are independently 0 or 1 but not both 0;
p and p′ are independently 0-8;
m + n + p is 1-10 when $X^2$ is O, S, S(O), or $S(O)_2$; m + n + p is 0-10 when $X^2$ is $CR^3R^{16}$;
m′ + n′ + p′ is 1-10 when $X^3$ is O, S, S(O), or $S(O)_2$; m′ + n′ + p′ is 0-10 when $X^3$ is $CR^3R^{16}$;
r is O or 1 when $Z^1$ is HET $(-R^3, -R^5)$;
r is 1 when $Z^1$ is $-CONR^3$;
r′ is O or 1 when $Z^2$ is $HET(-R^3,-R^5)$;
r′ is 1 when $Z^2$ is $CONR^3$;
s is 0-3;
$Q^1$ and $Q^2$ are independently $-COOR^3$, tetrazole, $-COOR^6$ $-CONHS(O)_2R^{13}$, -CN, $-CONR^{12}R^{12}$, -CHO, $-CH_2OH$, $-COCH^2OH$, $-NHS(O)_2R^{13}$; or if $Q^1$ or $Q^2$ is COOH and $R^4$ is -OH, -SH, or $-NHR^3$ then $Q^1$ or $Q^2$ and $R^4$ and the carbons through which they are attached may form a heterocyclic ring by loss of water;
W is O, S, or $NR^3$;
$X^1$ is O, S, -S(O)-, $-S(O)_2-$, $-NR^3$, or $-CR^3R^3-$;
$X^2$ and $X^3$ are independently O, S, S(O), $S(O)_2$, or $CR^3R^{16}$; Y is

$-CR^3=CR^3-$, $-C\equiv C-$, $-CR^3R^3-X^1-$,

$-X^1-CR^3R^3-$, $-CR^3R^3-X^1-CR^3R^3-$, 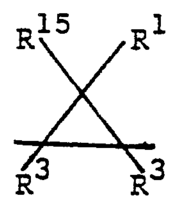 ,

C=O, $-NR^3-\overset{O}{C}-$, $-\overset{O}{C}-NR^3-$, O, S, or $NR^3$;

$Z^1$ and $Z^2$ are independently $-CONR^3-$ or $-HET(-R^3-R^5)-$provided that at least one of them is $-HET(-R^3, R^5)-$;
HET is

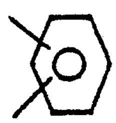 , 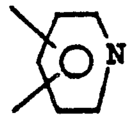 , or 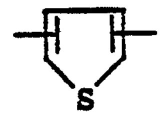 ;

and the pharmaceutically acceptable salts thereof.

Alkyl, alkenyl, and alkynyl are intended to include linear, branched, and cyclic structures and combinations thereof.

As used herein, the term "alkyl" includes "loweralkyl" and extends to cover carbon fragments having up to 20 carbon atoms. Examples of alkyl groups include octyl, nonyl, norbornyl, undecyl, dodecyl, tridecyl, tetradecyl, pentadecyl, eicosyl, 3,7-ethyl-2,2-methyl-4 propylnonyl, cyclododecyl, adamantyl, and the like.

As used herein, the term "loweralkyl" includes those alkyl groups of from 1 to 7 carbon atoms. Examples of loweralkyl groups include methyl, ethyl, propyl, isopropyl, butyl, sec- and tert-butyl, pentyl, hexyl, heptyl, cyclopropyl, cyclobutyl, cyclopentyl, cyclohexyl, cycloheptyl, 2-methylcyclopropyl, cyclopropylmethyl, and the like.

Alkenyl groups include vinyl, allyl, isopropenyl, pentenyl, hexenyl, heptenyl, cyclopropenyl, cyclobutenyl, cyclopentenyl, cyclohexenyl, 1-propenyl, 2-butenyl, 2-methyl-2-butenyl and the like.

As used herein, the term "alkoxy" includes those alkoxy groups of from 1 to 3 carbon atoms of either a straight, branched, or cyclic configuration. Examples of alkoxy groups include methoxy, ethoxy, propoxy, isopropoxy, cyclopropyloxy, and the like.

Substituted phenyl, benzyl, 2-phenethyl and pyridyl include 1 or 2 substituents on the aromatic ring selected from $C_1$-$C_6$ alkyl, $R^{10}$, $NO_2$, $SCF_3$, halogen, $-COR^7$, $-COR^{10}$, CN, and $CF_3$.

Halogen includes F, Cl, Br and I.

The prodrug esters of Q (i.e., when Q = $-COOR^6$) are intended to include the esters such as are described by Saari et al., J. Med. Chem., 21, No. 8, 746-753 (1978), Sakamoto et al., Chem. Pharm. Bull., 32, No. 6, 2241-2248 (1984) and Bundgaard et al., J. Med. Chem., 30, No. 3, 451-454 (1987).

When Q and $R^4$ and the carbons through which they are attached form a ring, the rings thus formed include lactones, lactams, and thiolactones.

It is intended that the definitions of any substituent (e.g., $R^1$, $R^2$, m, Q, X, etc.) in a particular molecule be independent of its definitions elsewhere in the molecule. Thus, $-NR^3R^3$ represents -NHH, $-NHCH_3$, $-NHC_6H_5$, etc.

The heterocycles formed when two $R^{12}$ groups join through N include pyrrolidine, piperidine, morpholine, thiamorpholine, piperazine, and N-methylpiperazine.

The naturally occurring amino acids, the radicals of which may be $CR^3R^4$, include alanine, asparagine, aspartic acid, arginine, cysteine, glutamic acid, glutamine, glycine, histidine, isoleucine, leucine, lysine methionine phenylalanine, proline, serine, threonine, tryptophan, tyrosine and valine.

Some of the compounds described herein contain one or more centers of asymmetry and may thus give rise to diastereoisomers and optical isomers. The present invention is meant to comprehend such possible diastereoisomers as well as their racemic and resolved, optically active forms. Optically active (R) and (S) isomers may be resolved using conventional techniques.

Some of the compounds described herein contain olefinic double bonds, and unless specified otherwise, are meant to include both E and Z geometric isomers.

Preferred compounds of Formula I are those wherein:

$R^1$ is H, halogen, $C_1$-$C_8$ alkyl, $-CF_3$, $-SR^2$, $-S(O)R^2$, $-S(O)_2R^2$, $-OR^3$, or -CN;

$R^2$ is $C_1$-$C_8$ alkyl or $-CF_3$;

$R^3$ is H or $R^2$;

$R^4$ is H, $-OR^3$, $-SR^3$, $NR^3R^3$, or $C_1$-$C_8$ alkyl;

$CR^3R^4$ may be the radical of a naturally occurring amino acid;

$R^5$ is H, halogen, -CN, $-SR^2$, $-OR^3$, $C_1$-$C_8$ alkyl, or $-(C=O)R^3$;

$R^6$ is $-(CH_2)_s-C(R^7R^7)-(CH_2)_s-R^8$ or $-CH_2CONR^{12}R^{12}$;

$R^7$ is H or $C_1$-$C_4$ alkyl;

$R^8$ is

A) a monocyclic or bicyclic heterocyclic radical containing from 3 to 12 nuclear carbon atoms and 1 or 2 nuclear heteroatoms selected from N, S or O and with each ring in the heterocyclic radical being formed of 5 or 6 atoms, or

B) the radical $W-R^9$;

$R^9$ contains up to 21 carbon atoms and is (1) a hydrocarbon radical or (2) an acyl radical of an organic acyclic or monocyclic carboxylic acid containing not more than l heteroatom in the ring;

$R^{10}$ is $-SR^{11}$, $-OR^{12}$, or $-NR^{12}R^{12}$;

$R^{11}$ is $C_1$-$C_6$ alkyl, $-(C=O)R^{14}$, unsubstituted phenyl, or unsubstituted benzyl;

$R^{12}$ is H, $R^{11}$, or two $R^{12}$ groups joined to the same N may form a ring of 5 or 6 members containing up to two heteroatoms chosen from O, S or N;

$R^{13}$ is $C_1$-$C_8$ alkyl, $-CF_3$, or unsubstituted phenyl, benzyl, or 2-phenethyl;

$R^{14}$ is H or $R^{13}$;

$R^{15}$ is $R^3$ or halogen;

$R^{16}$ is H, $C_1$-$C_4$ alkyl, or OH;

m and m′ are independently 0-4;

n and n′ are independently 0 or 1 but not both 0;

p and p′ are independently 0-4;

m + n + p is 1-10 when $X^2$ is O or S;

m + n + p is 0-10 when $X^2$ is $CR^3R^{16}$;

m′ + n′ + p′ is 1-10 when $X^3$ is O or S;

m′ + n′ + p′ is 0-10 when $X^3$ is $CR^3R^{16}$;

r is O or 1 when $Z^1$ is HET ($-R^3$, $-R^5$);

r is 1 when $Z^1$ is $-CONR^3$;

r′ is O or 1 when $Z^2$ is HET($-R^3$,$-R^5$);

r′ is 1 when $Z^2$ is $CONR^3$;

s is 0-3;

$Q^1$ and $Q^2$ are independently $-COOR^3$, tetrazole, $-COOR^6$, $-CONHS(O)_2R^{13}$, $-CONR^{12}R^{12}$, $-NHS(O)_2R^{13}$ ; or if $Q^1$ or $Q^2$ is COOH and $R^4$ is -OH, -SH, or $-NHR^3$ then $Q^1$ or $Q^2$ and $R^4$ and the carbons through which they are attached may form a heterocyclic ring by loss of water;

W is O, S, or NH;

$X^1$ is O, S, $-NR^3$, or $-CR^3R^3-$;

$X^2$ and $X^3$ are independently O, S, or $CR^3R^{16}$;

Y is $-CR^3=CR^3-$, $-C\equiv C-$, $-CR^3R^3-X^1-$, or $-X^1-CR^3R^3-$;

$Z^1$ and $Z^2$ are independently $-CONR^3$ or -HET($-R^3$-$R^5$)-provided that at least one of them is -HET($-R^3$,$-R^5$)-;

HET is

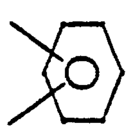 , 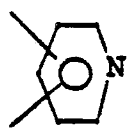 , or 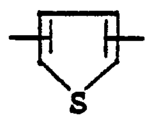 ;

and the pharmaceutically acceptable salts thereof.

More-preferred compounds of Formula I are those wherein:

$CR^3R^4$ is not the radical of a naturally occurring amino acid;

$Q^1$ and $Q^2$ are independently $-COOR^3$, tetrazole or $-CONR^{12}R^{12}$;

Y is $-CH=CH-$;

$Z^1$ and $Z^2$ are -HET($-R^3$-$R^5$)-;

and the remainder of the definitions are as in the above preferred embodiment.

Another more-preferred group of compounds of Formula I are those of Formula Ia:

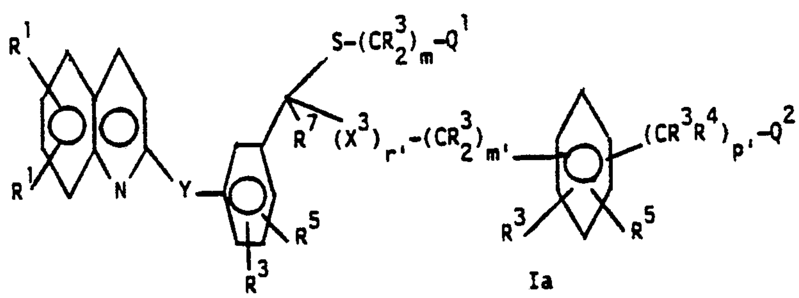

Ia

wherein:
$R^{6}$ is H or $C_1$-$C_8$ alkyl;
$CR^3R^4$ is not the radical of a naturally occurring amino acid;
m is 1-4;
m′ is 0-4;
p′ is 0-4;
r′ is 0 or 1;
$Q^1$ and $Q^2$ are independently $-COOR^3$, tetrazole, $-CONHS(O)_2R^{13}$, or $-CONR^{12}R^{12}$;
$X^3$ is S or $CR^3R^{16}$;
Y is $-CH=CH-$ or $-CH_2O-$;
and the remainder of the definitions are as in the above preferred embodiment.

It will be understood that in the discussion of methods of treatment which follows, references to the compounds of Formula I are meant to also include the pharmaceutically acceptable salts and the lactone, lactam and thiolactone forms.

The compounds of Formula I are active as antagonists of SRS-A and especially of leukotriene $D_4$. These compounds also have modest inhibitory activity on leukotriene biosynthesis but are primarily of therapeutic interest as antagonists. The activity of the compounds of Formula I can be detected and evaluated by methods known in the art. See for example, Kadin, U.S. Patent No. 4,296,129.

The ability of the compounds of Formula I to antagonize the effects of the leukotrienes and to inhibit the biosynthesis of the leukotrienes makes them useful for inhibiting the symptoms induced by the leukotrienes in a human subject. The compounds are valuable therefore in the prevention and treatment of such disease states in which the leukotrienes are the causative factor, e.g. skin disorders allergic rhinitis and obstructive airway diseases. The compounds are particularly valuable in the prevention and treatment of allergic bronchial asthma. They are also effective in the treatment of inflammatory diseases of the eye.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti inflammatory drugs on the gastrointestinal tract, animal studies show that cytoprotective compounds will prevent gastric lesions induced by oral administration of strong acids, strong bases, ethanol, hypertonic saline solutions and the like.

Two assays can be used to measure cytoprotective ability. These assays are; (A) an ethanol-induced lesion assay and (B) an indomethacin-induced ulcer assay and are described in U.S.P 4,683,325 (July 28, 1987).

The leukotriene antagonist properties of compounds of the present invention were evaluated using the following assays.

## Guinea-Pig Ileum Preparation for Evaluation of Antagonists of Leukotriene $D_4$ and Other Mediators

### Tissue:

Sections of ileum were taken from male Hartley strain guinea pigs (Charles River, U.S.A.) 300 to 500 g which were sacrificed by a blow to the head and exsanguinated. Terminal ileum was removed, cleaned with warm Tyrode's solution and then divided into segments of approximately 1.5-2.0 cm in each. The segments of ileum were then mounted under 1 g tension in a 20 ml organ bath containing 10 ml of Tyrode's solution with the following composition (mM): NaCl, 137; KCl, 2.7; $MgSO_4 \bullet 7H_2O$, 0.8; $CaCl_2$, 1.8; $NaH_2PO_4$, 0.42; $NaHCO_3$, 11.9; Dextrose, 5.6. The bathing solution was continuously aerated with 95% $O_2$ and 5% $CO_2$ and bath temperature was maintained at 37° C. The beta-adrenoceptor blocker, timolol (0.5 $\mu$g/ml) and the antimuscarinic agent atropine (1.0 $\mu$M) were present in the Tyrode's solution. Isometric tension changes were recorded using Grass FT03 force displacement transducers (Grass Instrument G., Quincy, Mass.) connected to a Beckman Type R Dynograph. The output (analog) signals from all channels of the Beckman Dynograph were converted to digital signals (DL-12 Data Logger, Buxco Electronics). These signals were subsequently fed into an IBM-XT computer for storage and subsequent analysis (Buxco Electronics Custom Software). In order to wash the tissue, the bath solution was automatically aspirated and replaced with a constant volume (10 ml) of fresh solution by means of timer controlled solenoid valves.

Antagonist Testing:

After the tissues were stable a standard dose of 0.3 ng/ml $LTD_4$ (100 µl) was repeatedly added (timer controlled Harvard Pump) to the bath every 4.5 minutes (1 minute contact, 30 second wash, 3 minute rest) until a consistent response was obtained (minimum of 4 responses). Addition of $LTD_4$ was performed automatically with two 4-channel Harvard Apparatus Syringe Pumps which delivered 100 µl (final bath concentration 0.3 ng/ml) of agonist simultaneously to all tissues every 4.5 minutes. Following each addition of $LTD_4$ the tissue was washed with Tyrode's solution until baseline tension was re established. After consistent responses were obtained the tissues were used to screen compounds.

Usually, 10 µl of a 10 mg/ml solution of the compound to be tested was added to the bath 30 seconds prior to the addition of $LTD_4$. The compound and $LTD_4$ remained in contact with the tissue until the maximum tension was developed (1 minute) after which the tissue was washed repeatedly until the baseline was re-established. Percent inhibition relative to the immediately preceding control response was computed on an IBM-XT for each dose of test compound (Buxco Electronics Custom Software). If the compound was active (greater than 50% inhibition) then tests were performed with 10 fold serial dilutions until inhibition was less than 50%. Provided the response was inhibited by less than 20%, the tissue was used immediately to evaluate another compound. When the response was inhibited by greater than 20%, cycles of $LTD_4$ alone were added until a consistent response was re-established.

In order to determine the specificity of the active compounds, they were tested against contractions induced by a standard dose of histamine (50 ng/ml) using a similar protocol to that described above (1'2 minute contact time, 30 seconds wash and 2 minutes rest).

$LTD_4$ Binding:

The results for $LTD_4$ binding were determined by the method of S.S. Pong and R.N. DeHaven, Proc. Nat. Acad. Sci. USA, 80, 7415-7419 (1983).

Compounds of Formula I were tested using the following assay to determine their mammalian leukotriene biosynthesis inhibiting activity.

Rat Peritoneal Polymorphonuclear (PMN) Leukocyte Assay

Rats under ether anesthesia are injected (i.p.) with 8 ml of a suspension of sodium caseinate (6 grams in ca. 50 ml water). After 15-24 hr. the rats are sacrificed ($CO_2$) and the cells from the peritoneal cavity are recovered by lavage with 20 ml of buffer (Eagles MEM containing 30 mM HEPES adjusted to pH 7.4 with NaOH). The cells are pelleted (350 x g, 5 min.), resuspended in buffer with vigorous shaking, filtered through lens paper, recentrifuged and finally suspended in buffer at a concentration of 10 cells/ml. A 500 µl aliquot of PMN suspension and test compound are preincubated for 2 minutes at 37° C, followed by the addition of 10 µM A-23187. The suspension is stirred for an additional 4 minutes then bioassayed for $LTB_4$ content by adding an aliquot to a second 500 µl portion of the PMN at 37° C. The $LTB_4$ produced in the first incubation causes aggregation of the second PMN, which is measured as a change in light transmission. The size of the assay aliquot is chosen to give a submaximal transmission change (usually -70%) for the untreated control. The percentage inhibition of $LTB_4$ formation is calculated from the ratio of transmission change in the sample to the transmission change in the compound-free control.

The following assays can be used to evaluate compounds which are either leukotriene antagonists or inhibitors of leukotriene biosynthesis or which possess a combination of these two properties.

Antigen Challenge 'in vitro' Assay

Male guinea pigs weighing 300-350 g are sensitized by injecting (intraperitoneally) 0.5 ml of a suspension containing 0.4 mg of egg albumin (Ovalbumin, Grade V, Sigma Chemical Co.) and 4.0 g of aluminum hydroxide in 19.6 ml of saline. Two weeks are permitted for sensitization to occur.

Three sensitized guinea pigs are stunned and exanguinated. The tracheas are removed, freed of adhering tissue and divided longitudinally by cutting through the cartilaginous tissue directly opposite the

muscle insertion. Each opened trachea is then transected between every second cartilage. Four of the cut sections are tied together, end to end, in a series with No.7 silk thread ensuring that the tracheal muscles are all in the same vertical plane. Thus, each chain consists of tissue from three different animals.

The chain so formed is then suspended under 1 g of tension (by silk ties at each end) in a 2 ml organ bath containing 1 ml of modified [1] Krebs-Henseleit buffer solution gassed with 95% $O_2$ and 5% $CO_2$ at 37° C. Mepyramine (7 x $10^{-6}$ M), atropine (1 x $10^{-7}$ M) and indomethacin (1.4 x $10^{-6}$ M) are added to the buffer to block the response to released histamine, acetylcholine, and cyclooxygenase products. To record responses, one end of the tracheal chain is attached to a Gould-Statham UC-2 force displacement transducer which is connected to a Beckman Type R Dynograph. The preparations are allowed to equilibrate for one hour during which time the tissues are automatically washed (10 ml volume displacement) every 6 minutes.

After the equilibration period the tissues are primed with methacholine (10 $\mu$g/ml), washed and allowed to recover to baseline. The tissues are treated again with a second dose of methacholine, washed, allowed to return to baseline and washed for an additional hour.

Two chains are used as a control. These are incubated in a concentration of egg albumin (0.1 $\mu$g/ml) sufficient to induce an average contraction of 50-80% of the methacholine response.

Each compound to be tested is added (at a final bath concentration of 10u $\mu$l/ml) 20 minutes prior to challenging the tissue with egg albumin.

The response of the challenged tissue is expressed as a percentage of the methacholine maximum. The percentage inhibition for each compound is then calculated. Compounds which at 10 $\mu$g/ml (final concentration) inhibit the egg albumin response by 50% or more are retested at a lower concentration.

## Asthmatic Rat Assay

Rats are obtained from an inbred line of asthmatic rats. Both female (190-250 g) and male (260-400 g) rats are used.

Egg albumin (EA), grade V, crystallized and lyophilized, is obtained from Sigma Chemical Co., St. Louis. Aluminum hydroxide is obtained from the Regis Chemical Company, Chicago. Methysergide bimaleate is supplied by Sandoz Ltd., Basel.

The challenge and subsequent respiratory recordings are carried out in a clear plastic box with internal dimensions 10 x 6 x 4 inches. The top of the box is removable; in use it is held firmly in place by four clamps and an airtight seal is maintained by a soft rubber gasket. Through the center of each end of the chamber a Devilbiss nebulizer (No. 40) is inserted via an airtight seal and each end of the box also has an outlet. A Fleisch No. 0000 pneumotachograph is inserted into one end of the box and coupled to a Grass volumetric pressure transducer (PT5-A) which is then connected to a Beckman Type R Dynograph through appropriate couplers. While aerosolizing the antigen, the outlets are open and the pneumotachograph is isolated from the chamber. The outlets are closed and the pneumotachograph and the chamber are connected during the recording of the respiratory patterns. For challenge, 2 ml of a 3% solution of antigen in saline is placed into each nebulizer and the aerosol is generated with air from a small Potter diaphragm pump operating at 10 psi and a flow of 8 liters/minute.

Rats are sensitized by injecting (subcutaneously) 1 ml of a suspension containing 1 mg EA and 200 mg aluminum hydroxide in saline. They are used between days 12 and 24 postsensitization. In order to eliminate the serotonin component of the response rats are pretreated intravenously 5 minutes prior to aerosol challenge with 3.0 $\mu$g/kg of methysergide. Rats are then exposed to an aerosol of 3% EA in saline for exactly 1 minute, then their respiratory profiles are recorded for a further 30 minutes. The duration of continuous dyspnea is measured from the respiratory recordings.

Compounds are generally administered either orally 1-4 hours prior to challenge or intraveneously 2 minutes prior to challenge. They are either dissolved in saline or 1% methocel or suspended in 1% methocel. The volume injected is 1 ml/kg (intravenously) or 10 ml/kg (orally). Prior to oral treatment rats are starved overnight. Their activity is determined in terms of their ability to decrease the duration of symptoms of dyspnea in comparison with a group of vehicle-treated controls. Usually, a compound is evaluated at a series of doses and an $ED_{50}$ is determined. This is defined as the dose (mg/kg) which would inhibit the

[1]modified Krebs solution in grams/liter and (mM): NaCl - 6.87 (120); glucose - 2.1 (11); $NaHCO_3$ -2.1 (25); KCl - 0.32 (4.72); $CaCl_2$ - 0.28 (2.5); $MgSO_4 \cdot 7H_2O$ - 0.11 (0.5); $KH_2PO_4$ - 0.16 (1.2); pH at bathing solution = 7.35 ± 0.05.

duration of symptoms by 50%.

The magnitude of a prophylactic or therapeutic dose of a compound of Formula I will, of course vary with the nature of the severity of the condition to be treated and with the particular compound of Formula I and its route of administration. It will also vary according to the age, weight and response of the individual patient. In general, the daily dose range for anti-asthmatic, anti-allergic or anti-inflammatory use and generally, uses other than cytoprotection, lie within the range of from about 0.001 mg to about 100 mg per kg body weight of a mammal, preferably 0.01 mg to about 10 mg per kg, and most preferably 0.1 to 1 mg per kg, in single or divided doses. On the other hand, it may be necessary to use dosages outside these limits in some cases.

The exact amount of a compound of the Formula I to be used as a cytoprotective agent will depend on, inter alia, whether it is being administered to heal damaged cells or to avoid future damage, on the nature of the damaged cells (e.g., gastrointestinal ulcerations vs. nephrotic necrosis), and on the nature of the causative agent. An example of the use of a compound of the Formula I in avoiding future damage would be co-administration of a compound of the Formula I with a non-steroidal anti-inflammatory drug (NSAID) that might otherwise cause such damage (for example, indomethacin). For such use, the compound of Formula I is administered from 30 minutes prior up to 30 minutes after administration of the NSAID. Preferably it is administered prior to or simultaneously with the NSAID, (for example, in a combination dosage form).

The effective daily dosage level for compounds of Formula I inducing cytoprotection in mammals, especially humans, will generally range from about 0.1 mg/kg to about 100 mg/kg, preferably from about 1 mg/kg to about 100 mg/kg. The dosage may be administered in single or divided individual doses.

The pharmaceutical compositions of the present invention comprise a compound of Formula I as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases or acids including inorganic bases or acids and organic bases or acids.

Salts derived from inorganic bases include aluminum, ammonium, calcium, copper, ferric, ferrous, lithium, magnesium, manganic, manganous, potassium, sodium, zinc salts and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines and basic ion exchange resins, such as arginine, betaine, caffeine, choline, N,N′-dibenzylethylenediamine, diethylamine, 2-diethylaminoethanol, 2-dimethylaminoethanol, ethanolamine, ethylenediamine, N-ethyl morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric and p-toluenesulfonic acid, and the like. Particularly preferred are hydrobromic, hydrochloric, phosphoric, and sulfuric acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

Dosage forms include tablets, troches, dispersions, suspensions, solutions, capsules, creams, ointments, aerosols, and the like.

For use where a composition for intravenous administration is employed, a suitable dosage range for anti-asthmatic, anti-inflammatory or anti-allergic use is from about 0.001 mg to about 10 mg (preferably from about 0.01 mg to about I mg) of a compound of Formula I per kg of body weight per day and for cytoprotective use from about 0.1 mg to about 100 mg (preferably from about 1 mg to about 100 mg and more preferably from about I mg to about 10 mg) of a compound of Formula I per kg of body weight per day.

In the case where an oral composition is employed, a suitable dosage range for anti-asthmatic, anti-inflammatory or anti-allergic use is, e.g. from about 0.01 mg to about 100 mg of a compound of Formula I per kg of body weight per day, preferably from about 0.1 mg to about 10 mg per kg and for cytoprotective

use from about 0 1 mg to about 100 mg (preferably from about 1 mg to about 100 mg and more preferably from about 10 mg to about 100 mg) of a compound of Formula I per kg of body weight per day.

For administration by inhalation, the compounds of teh present invention are conveniently delivered in the form of an aerosol spray presentation from pressurized packs or a nebuliser, or as a powder which may be formulated as a cartridge from which the powder composition may be inhaled with the aid of a suitable device. The preferred delivery system for inhalation is a metered dose inhalation (MDI) aerosol, which may be formulated as a suspension or solution in fluorocarbon propellants.

Suitable topical formulations of Compound I include transdermal devices, aerosols, creams, ointments, lotions, dusting powders, and the like.

For the treatment of diseases of the eye, ophthalmic preparations for ocular administration comprising 0.001-1% by weight solutions or suspensions of the compounds of Formula I in an acceptable ophthalmic formulation may be used.

In practical use, the compounds of formula I can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage from, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration tablets and capsules represent the most advantageous oral dosage unit form, in which case solid pharmaceutical carriers are obviously employed. If desired, tablets may be coated by standard aqueous or nonaqueous techniques.

In addition to the common dosage forms set out above, the compounds of Formula I may also be administered by controlled release means and/or delivery devices such as those described in U.S. Patent Nos. 3,845,770; 3,916,899; 3,536,809; 3,598,123; 3,630,200 and 4,008,719, the disclosures of which are hereby incorporated herein by reference.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Desirably, each tablet contains from about 2.5 mg to about 500 mg of the active ingredient and each cachet or capsule contains from about 2.5 to about 500 mg of the active ingredient.

The following are examples of representative pharmaceutical dosage forms for the compounds of Formula I:

| Injectable Suspension (I.M.) | mg/ml |
|---|---|
| Compound of Formula I | 10 |
| Methylcellulose | 5.0 |
| Tween 80 | 0.5 |
| Benzyl alcohol | 9.0 |
| Benzalkonium chloride | 1.0 |
| Water for injection to a total volume of 1 ml | |

| Tablet | mg/tablet |
|---|---|
| Compound of Formula I | 25 |
| Microcrystalline Cellulose | 415 |
| Providone | 14.0 |
| Pregelatinized Starch | 43.5 |
| Magnesium Stearate | 2.5 |
| | 500 |

| Capsule | mg/capsule |
|---|---|
| Compound of Formula I | 25 |
| Lactose Powder | 573.5 |
| Magnesium Stearate | 1.5 |
| | 600 |

In addition to the compounds of Formula I, the pharmaceutical compositions of the present invention can also contain other active ingredients, such as cyclooxygenase inhibitors, non-steroidal anti-inflammatory drugs (NSAIDS), peripheral analgesic agents such as zomepirac, diflunisal and the like. The weight ratio of the compound of the Formula to the second active ingredient may be varied and will depend upon the effective dose of each ingredient. Generally, an effective dose of each will be used. Thus, for example, when a compound of the Formula I is combined with an NSAID the weight ratio of the compound of the Formula I to the NSAID will generally range from about 1000:1 to about 1:1000. Combinations of a compound of the Formula I and other active ingredients will generally also be within the aforementioned range, but in each case, an effective dose of each active ingredient should be used.

NSAIDS can be characterized into five groups:

(1) the propionic acid derivatives;
(2) the acetic acid derivatives;
(3) the fenamic acid derivatives;
(4) the biphenylcarboxylic acid derivatives;

and

(5) the oxicams

or a pharmaceutically acceptable salt thereof. NSAIDS which are within the scope of this invention are those disclosed in U.S.P. 4,683 325 (July 28,1987).

Pharmaceutical compositions comprising the Formula I compounds may also contain inhibitors of the biosynthesis of the leukotrienes such as are disclosed in U.S.P. 4,666,907 (April 19, 1987), U.S.P. 4,663,307 (May 5, 1987), U.S.P. 4,611,056 (September 9, 1986), and U.S.P. 4,634,766 (January 6, 1987), which are hereby incorporated herein by reference.

The compounds of the Formula I may also be used in combination with leukotriene antagonists such as those disclosed in EP 106,565 (April 25, 1984) and EP 104,885 (April 4, 1984) which are hereby incorporated herein by reference and others known in the art such as those disclosed in EP 56,172 (July 21, 1982) and U.S.P. 4,424,231 (January 3, 1984); and in U.K. Patent Specification No. 2,058,785, which are hereby incorporated herein by reference.

Pharmaceutical compositions comprising the Formula I compounds may also contain as the second active ingredient prostaglandin (including thromboxane) antagonists such as those disclosed in U.S.P. 4,536,507 (August 20, 1985), U.S.P. 4,237,160 (December 2, 1980), EP 166,597 (January 1, 1986), and EP 234,708 (September 2, 1987). They may also contain histidine decarboxylase inhibitors such as α-fluoromethylhistidine, described in U.S. 4,325,961. The compounds of the Formula I may also be advantageously combined with an $H_1$ or $H_2$-receptor antagonist, such as for instance benadryl, dramamine, histadyl, phenergan, terfenadine, acetamazole, cimetidine, ranitidine, famotidine, aminothiadiazoles disclosed in EP 40,696 (December 2, 1981) and like compounds, such as those disclosed in U.S. Patent Nos. 4,283,408; 4,362,736; and 4,394,508. The pharmaceutical compositions may also contain a $K^+/H^+$ ATPase inhibitor such as omeprazole, disclosed in U.S.P. 4,255,431, and the like. Another useful pharmaceutical composition comprises the Formula I compounds in combination with serotonin antagonists such as methysergide, the serotonin antagonists disclosed in Nature, vol. 316, pages 126-131, 1985, and the like.

Each of the references referred to in this paragraph is hereby incorporated herein by reference.

When the second active ingredient in compositions of this invention is a thromboxane synthetase inhibitor, such inhibitor can be as described in UK 2,038,821 (e.g., UK-37248 and dazoxiben hydrochloride), U.S.P. 4,217,357 (e.g., UK-34787), U.S.P. 4,444,775 (e.g., CGS 13080), U.S.P. 4,226,878 (e.g., ONO 046), U.S.P. 4,495,357 (e.g., U63557A) U.S.P. 4,273,782 (e.g., UK-38485), or EP 98,690 (e.g., CV-4151).

The combination compositions can be administered orally or other than orally; e.g., parenterally, by insufflation, topically, rectally, etc.; using appropriate dosage forms; e.g., tablets, capsules, suspensions, solutions, and the like, for oral administration; suspension emulsions, and the like, for parenteral administration; solutions for intravenous administration; and ointments, transdermal patches, and the like, for topical administration. These compositions are formulated similarly to the compositions discussed above.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The following compounds (formula I′) are within the scope of the invention:

TABLE 1

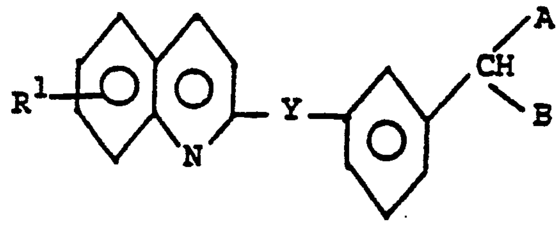

I'

| Example | $R^1$ | Y | A | B |
|---|---|---|---|---|
| 1 | 7-Cl | -CH=CH- | $-S(CH_2)_2CO_2H$ | $-CH_2CH_2$(1,2-Phe)$CO_2H$* |
| 2 | 7-Cl | -CH=CH- | $-S(CH_2)_2C(O)N(CH_3)_2$ | $-CH_2CH_2$(1,2-Phe)$CO_2H$ |
| 3 | 7-Cl | $-CH_2CH_2-$ | $-S(CH_2)_2C(O)N(CH_3)_2$ | -(1,3-Phe)$CO_2H$ |
| 4 | 7-Cl | -CH=CH- | $-S(CH_2)_2C(O)N(CH_3)_2$ | -(2,5-Thio)$CO_2H$ |
| 5 | 7-Cl | -CH=CH- | $-S(CH_2)_2C(O)N(CH_3)_2$ | -(1,3-Phe)$CO_2H$ |
| 6 | 7-Cl | -CH=CH- | $-S(CH_2)_2C(O)N(CH_3)_2$ | -(1,4-Phe)$CO_2H$ |
| 7 | 7-Cl | -CH=CH- | $-S(CH_2)_2CO_2H$ | -(1,3-Phe)$CO_2H$ |
| 8 | 7-Cl | -CH=CH- | -S-(1,3-Phe)$CO_2H$ | $-(CH_2)_2CH(CH_3)CH_2CO_2H$ |
| 9 | 7-Cl | $-CH_2O-$ | $-S(CH_2)_2C(O)N(CH_3)_2$ | -(1,3-Phe)$CO_2H$ |
| 10 | 7-Cl | -CH=CH- | -S(1,3-Phe)$CO_2H$ | -(1,3-Phe)$CO_2H$ |
| 11 | 7-Cl | $-CH_2O-$ | $-S(CH_2)_2C(O)NH$-t-Bu | -(1,3-Phe)$CO_2H$ |
| 12 | 7-Cl | $-CH_2O-$ | $-S(CH_2)_2C(O)N(CH_3)_2$ | -(3,5-Pye)$CO_2H$ |
| 13 | 7-Cl | -CH=CH- | $-S(CH_2)_2C(O)N(CH_3)_2$ | -(3,5-Pye)$CO_2H$ |
| 14 | 7-Cl | $-CH_2O-$ | -S-(1,3-Phe)$CO_2H$ | -S-(1,3-Phe)$CO_2H$ |
| 15 | 7-Cl | -CH=CH- | -S-(1,3-Phe)$CO_2H$ | -S-(1,3-Phe)$CO_2H$ |
| 16 | 7-Cl | -CH=CH- | -S-(1,4-Phe)$CO_2H$ | -S-(1,4-Phe)$CO_2H$ |
| 17 | 7-Cl | -CH=CH- | -S-(1,4-Phe)$CO_2H$ | -(2,6-Pye)$CO_2H$ |
| 18 | $7-OCH_3$ | -CH=CH- | $-S(CH_2)_2CO_2H$ | -(1,3-Phe)$CO_2H$ |
| 19 | $6-CF_3$ | -CH=CH- | $-S(CH_2)_2CO_2H$ | -(1,3-Phe)$CO_2H$ |
| 20 | $7-CF_3$ | -CH=CH- | $-S(CH_2)_2CO_2H$ | -(1,4-Phe)$CO_2H$ |
| 21 | $6-SO_2CH_3$ | -CH=CH- | $-S(CH_2)_2CO_2H$ | -(1,3-Phe)$CO_2H$ |
| 22 | H | -CH=CH- | $-S(CH_2)_2CO_2H$ | -(1,3-Phe)$CO_2H$ |

Table 1 (cont'd)

| Ex. | $R^1$ | Y | A | B |
|---|---|---|---|---|
| 23 | 7-Cl | -CH=CH- | $-S(1,4-Phe)CO_2H$ | $-(1,3-Phe)CO_2H$ |
| 24 | 7-Cl | -CH=CH- | $-S(1,4-Phe)C(O)N(CH_3)_2$ | $-(1,3-Phe)CO_2H$ |
| 25 | 7-Cl | $-CH_2O-$ | $-S(CH_2)_2CO_2H$ | $-CH_2(1,3-Phe)CO_2H$ |
| 26 | 7-Cl | -CH=CH- | $-S(4,2-Pye)CO_2H$ | $-(1,3-Phe)CO_2H$ |
| 27 | 7-Cl | -CH=CH- | $-S(1,2-Phe)CO_2H$ | $-(1,3-Phe)CO_2H$ |
| 28 | 7-Cl | $-CH_2O-$ | $-S(CH_2)_2CO_2H$ | $-(CH_2)_2(1,2-Phe)CO_2H$ |
| 29 | 7-Cl | $-CH_2O-$ | $-S(CH_2)_2C(O)NMe_2$ | $-(CH_2)_2(1,2-Phe)CO_2H$ |
| 30 | 7-Cl | $-CH_2O-$ | $-S(1,2-Phe)CO_2H$ | $-S(1,2-Phe)CO_2H$ |
| 31 | 7-Cl | $-CH_2O-$ | $-(CH_2)_2C(O)N(CH_3)_2$ | $-(1,3-(4-Cl-Phe))CO_2H$ |
| 32 | 7-Cl | $-CH_2O-$ | $-SCH_2(1,2-Phe)CO_2H$ | $-SCH_2(1,2-Phe)CO_2H$ |
| 33 | 7-Cl | $-CH_2O-$ | $-SCH_2(1,2-Phe)CO_2H$ | $-S(CH_2)_2C(O)N(CH_3)_2$ |
| 34 | 7-Cl | -CH=CH- | $-S(CH_2)_3C(O)N(CH_3)_2$ | $-(1,3-Phe)CO_2H$ |
| 35 | 7-Cl | $-CH_2O-$ | $-S(CH_2)_2C(O)NH-t-Bu$ | $-(CH_2)_2(1,2-Phe)CO_2H$ |
| 36 | 7-Cl | $-CH_2CH_2-$ | $-S(CH_2)_2C(O)NH-t-Bu$ | $-(CH_2)_2(1,2-Phe)CO_2H$ |
| 37 | 7-Cl | $-CH_2CH_2-$ | $-S(CH_2)_2C(O)N(CH_3)_2$ | $-(CH_2)_2(1,3-Phe)CO_2H$ |
| 38 | 7-Cl | $-CH_2O-$ | $-S(CH_2)_2C(O)N(CH_3)_2$ | $-(1,2-Phe)CO_2H$ |
| 39 | 7-Cl | $-CH_2O-$ | $-S(CH_2)_2C(O)NH(1-adamantyl)$ | $-(CH_2)_2(1,2-Phe)CO_2H$ |
| 40 | 7-Cl | $-CH_2O-$ | $-S(CH_2)_2C(O)N(CH_3)_2$ | $-(CH_2)_2(1,2-Phe)CN_4H$* |
| 41 | 7-Cl | $-CH_2O-$ | $-S(CH_2)_2C(O)N(CH_3)_2$ | $-(CH_2)_2(1,2-Phe)CH_2CN_4H$ |
| 42 | 7-Cl | $-CH_2O-$ | $-S(CH_2)_2C(O)N(CH_3)_2$ | $-(CH_2)_2(1,2-Phe)CH_2CO_2H$ |
| 43 | 7-Cl | $-CH_2O-$ | $-S(CH_2)_2CO_2H$ | $-(CH_2)_2(1,2-Phe)C(O)N(CH_3)_2$ |
| 44 | 7-Cl | $-CH_2O-$ | $-S(CH_2)_2C(O)N(CH_3)_2$ | $-(1,3-Phe)CH_2CN_4H$ |
| 45 | 7-Cl | $-CH_2O-$ | $-S(1,3-Phe)CO_2H$ | $-(1,3-Phe)CO_2H$ |
| 46 | 7-Cl | $-CH_2O-$ | $-S(CH_2)_2CO_2H$ | $-(1,3-Phe)CH_2C(O)N(CH_3)_2$ |
| 47 | 7-Cl | $-CH_2O-$ | $-S(CH_2)_2CO_2H$ | $-(1,3-Phe)CH_2C(O)NH-t-Bu$ |
| 48 | 7-Cl | $-CH_2O-$ | $-S(CH_2)_2CN_4H$ | $-(1,3-Phe)CH_2C(O)N(CH_3)_2$ |
| 49 | 7-Cl | $-CH_2O-$ | $-S(CH_2)_2CN_4H$ | $-(1,3-Phe)CH_2C(O)NH-t-Bu$ |
| 50 | 7-Cl | $-CH_2O-$ | $-SCH_2C(O)N(CH_3)_2$ | $-(1,3-Phe)CH_2CN_4H$ |
| 51 | 7-Cl | $-CH_2O-$ | $-SCH_2C(O)NH-t-Bu$ | $-(1,3-Phe)CH_2CN_4H$ |
| 52 | 7-Cl | $-CH_2O-$ | $-S(CH_2)_2C(O)N(CH_3)_2$ | $-(1,2-Phe)CH_2CN_4H$ |
| 53 | 7-Cl | $-CH_2O-$ | $-S(CH_2)_2C(O)NH-t-Bu$ | $-(1,2-Phe)CH_2CN_4H$ |
| 54 | 7-Cl | $-CH_2O-$ | $-SCH_2CO_2H$ | $-(CH_2)_2(1,2-Phe)C(O)N(CH_3)_2$ |
| 55 | 7-Cl | $-CH_2O-$ | $-SCH_2CO_2H$ | $-(CH_2)_2(1,2-Phe)C(O)NH-t-Bu$ |
| 56 | 7-Cl | $-CH_2O-$ | $-S(CH_2)_2CO_2H$ | $-(CH_2)_2(1,3-Phe)C(O)N(CH_3)_2$ |
| 57 | 7-Cl | $-CH_2O-$ | $-S(CH_2)_2CO_2H$ | $-(CH_2)_2(1,3-Phe)C(O)NH-t-Bu$ |
| 58 | 7-Cl | $-CH_2O-$ | $-SCH_2CO_2H$ | $-(CH_2)_2(1,3-Phe)C(O)N(CH_3)_2$ |
| 59 | 7-Cl | $-CH_2O-$ | $-SCH_2CO_2H$ | $-(CH_2)_2(1,3-Phe)C(O)NH-t-Bu$ |
| 60 | 7-Cl | $-CH_2O-$ | $-SCH_2CN_4H$ | $-(CH_2)_2(1,3-Phe)C(O)N(CH_3)_2$ |
| 61 | 7-Cl | $-CH_2O-$ | $-SCH_2CN_4H$ | $-(CH_2)_2(1,3-Phe)C(O)NH-t-Bu$ |
| 62 | H | $-CH_2O-$ | $-S(CH_2)_2CO_2H$ | $-(CH_2)_2(1,2-Phe)C(O)N(CH_3)_2$ |
| 63 | 6,7-diCl | $-CH_2O-$ | $-S(CH_2)_2CO_2H$ | $-(CH_2)_2(1,2-Phe)C(O)N(CH_3)_2$ |
| 64 | $7-S(O)_2Me$ | $-CH_2O-$ | $-S(CH_2)_2CO_2H$ | $-(CH_2)_2(1,2-Phe)C(O)N(CH_3)_2$ |
| 65 | $6-OCH_3$ | $-CH_2O-$ | $-S(CH_2)_2CO_2H$ | $-(CH_2)_2(1,2-Phe)C(O)N(CH_3)_2$ |

Table 1 (cont'd)

| Ex. | $R^1$ | Y | A | B |
|---|---|---|---|---|
| 66 | 6-$CH(CH_3)_2$ | $-CH_2O-$ | $-S(CH_2)_2CO_2H$ | $-(CH_2)_2$(1,2-Phe)C(O)$N(CH_3)_2$ |
| 67 | 7-Cl | $-CH_2O-$ | $-S(CH_2)_2CO_2H$ | $-(CH_2)_2$(1,2-Phe)C(O)NH-t-Bu |
| 68 | 7-Cl | $-CH_2O-$ | $-S(CH_2)_2CO_2H$ | $-(CH_2)_2$(1,2-Phe)C(O)$N(CH_2)_5$* |
| 69 | 7-Cl | $-CH_2O-$ | $-S(CH_2)_2CO_2H$ | $-(CH_2)_2$(1,2-Phe)C(O)$N((CH_2)_2O(CH_2)_2)$* |
| 70 | 7-Cl | $-CH_2O-$ | $-S(CH_2)_2CO_2H$ | $-(CH_2)_2$(1,2-Phe)C(O)NH-1-adamantyl |
| 71 | 7-Cl | $-CH_2O-$ | $-S(CH_2)_2CO_2H$ | $-(CH_2)_2$(1,2-Phe)C(O)$NHCH_2Ph$* |
| 72 | 7-Cl | $-CH_2O-$ | $-S(CH_2)_2CO_2H$ | $-(CH_2)_2$(1,2-Phe)C(O)H-1-naphthyl |
| 73 | 7-Cl | $-CH_2O-$ | $-S(CH_2)_2CN_4H$ | $-(CH_2)_2$(1,2-Phe)C(O)$N(CH_3)_2$ |
| 74 | 7-Cl | $-CH_2O-$ | $-S(CH_2)_2CN_4H$ | $-(CH_2)_2$(1,2-Phe)C(O)NH-t-Bu |
| 75 | 7-Cl | $-CH_2O-$ | $-SCH_2CH(CH_3)CO_2H$ | $-(CH_2)_2$(1,2-Phe)C(O)$N(CH_3)_2$ |
| 76 | 7-Cl | $-CH_2O-$ | $-SCH_2CH(CH_3)CO_2H$ | $-(CH_2)_2$(1,2-Phe)C(O)NH-t-Bu |
| 77 | 7-Cl | $-CH_2O-$ | $-S(CH_2)_2CO_2H$ | $-(CH_2)_2$(1,2-(4-Br-Phe))C(O)$N(CH_3)_2$ |
| 78 | 7-Cl | $-CH_2O-$ | $-S(CH_2)_2CO_2H$ | $-(CH_2)_2$(1,2-(4-$SCH_3$-Phe))C(O)$N(CH_3)_2$ |
| 79 | 7-Cl | $-CH_2O-$ | $-S(CH_2)_2CO_2H$ | $-(CH_2)_2$(1,2-(4-$S(O)_2CH_3$-Phe))$(CH_3)_2$ |
| 80 | 7-Cl | $-CH_2O-$ | $-S(CH_2)_2CO_2H$ | $-(CH_2)_2$(1,2-(5-Br-Phe))C(O)$N(CH_3)_2$ |
| 81 | 7-Cl | $-CH_2O-$ | $-S(CH_2)_2CO_2H$ | $-(CH_2)_2$(1,2-(5-$SCH_3$-Phe))C(O)$N(CH_3)_2$ |
| 82 | 7-Cl | $-CH_2O-$ | $-S(CH_2)_2CO_2H$ | $-(CH_2)_2$(1,2-(5-$S(O)_2CH_3$-Phe))C(O)$N(CH_3)_2$ |
| 83 | 7-Cl | $-CH_2O-$ | $-S(CH_2)_2C(O)N(CH_3)_2$ | $-(CH_2)_2$(1,2-Phe)C(O)$NHS(O)_2CH_3$ |
| 84 | 7-Cl | $-CH_2O-$ | $-S(CH_2)_2C(O)NHS(O)_2CF_3$ | $-(CH_2)_2$(1,2-Phe)C(O)$N(CH_3)_2$ |
| 85 | 7-Cl | $-CH_2O-$ | $-S(CH_2)_2C(O)N(CH_3)_2$ | -(1,3-Phe)$CH_2C(O)NHS(O)_2Ph$ |
| 86 | 7-Cl | $-CH_2O-$ | $-OCH_2CN_4N$ | $-(CH_2)_2$(1,2-Phe)C(O)$N(CH_3)_2$ |
| 87 | 7-Cl | $-CH_2O-$ | $-OCH_2CO_2H$ | $-(CH_2)_2$(1,2-Phe)C(O)$N(CH_3)_2$ |
| 88 | 7-Cl | $-CH_2O-$ | $-OCH(CH_3)CO_2H$ | $-(CH_2)_2$(1,2-Phe)C(O)$N(CH_3)_2$ |
| 89 | 7-Cl | $-CH_2O-$ | $-O(CH_2)_2C(O)N(CH_3)_2$ | -(1,3-Phe)$CH_2CN_4H$ |
| 90 | 7-Cl | $-CH_2O-$ | $-O(CH_2)_2C(O)N(CH_3)_2$ | -(1,3-Phe)$CH(CH_3)CN_4H$ |
| 91 | 7-Cl | $-CH_2CH_2-$ | $-S(CH_2)_2CO_2H$ | $-(CH_2)_2$(1,2-Phe)C(O)$N(CH_3)_2$ |
| 92 | 7-Cl | $-CH_2CH_2-$ | $-SCH_2CH(CH_3)CO_2H$ | $-(CH_2)_2$(1,2-Phe)C(O)$N(CH_3)_2$ |
| 93 | 7-Cl | $-CH_2CH_2-$ | $-SCH_2CO_2H$ | $-(CH_2)_2$(1,2-Phe)C(O)$N(CH_3)_2$ |
| 94 | 7-Cl | $-CH_2CH_2-$ | $-SCH(CH_3)CO_2H$ | $-(CH_2)_2$(1,2-Phe)C(O)$N(CH_3)_2$ |
| 95 | 7-Cl | $-CH_2O-$ | $-S(CH_2)_2CO_2H$ | $-(CH_2)_2$(1,2-Phe)C(O)$N(C_2H_5)_2$ |
| 96 | 7-Cl | $-CH_2O-$ | $-S(CH_2)_2CO_2H$ | $-(CH_2)_2$(1,2-Phe)C(O)$NHCH_3$ |

*Phe = 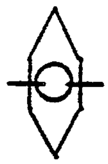 Thio = 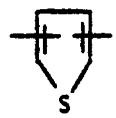 Pye = 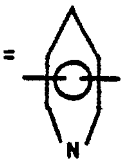 Ph = 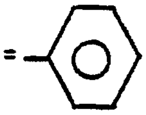

$N(CH_2)_5$ = 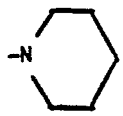 $N((CH_2)_2O(CH_2)_2)$ = 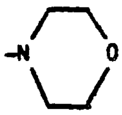

t-Bu = $-C(CH_3)_3$ $CN_4H$ = 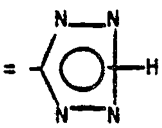

Compounds of the present invention can be prepared according to the following methods. Temperatures are in degrees Celcius.

METHOD A

Quinaldine derivative II is condensed with aldehyde IIa in the presence of a suitable catalyst like $ZnCl_2$ at temperatures greater than 120° or by heating with a dehydrating agent such as acetic anhydride to give adduct III. Bromo acid IV is treated with 2 equivalents of a base such as n-butyllithium in a suitable solvent such as THF at -100° then at -78° with III to afford alcohol V. Alcohol V is reacted with thiol VI in the presence of a suitable catalyst such as $BF_3$ or $AlCl_3$ to give adduct VII.

METHOD B

Alternatively, adduct V can be transformed to VIII, where Z is a suitable leaving group such as Cl, using reaction conditions such as $CCl_4$/trioctylphosphine. VIII is reacted with thiol VI in the presence of a suitable base such as $K_2CO_3$ to give adduct VII.

METHOD C

Referring to Method C, a quinoline derivative of structure IX is prepared from II using a suitable reagent such as N-bromosuccinimide. IX is then reacted with a compound of formula X in the presence of a suitable base such as NaOH, NaH, $K_2CO_3$ or NaOMe in an inert solvent such as THF with warming if necessary to provide the adduct XI. Using the reactions described in Methods A or B adduct XI is transformed to XII.

METHOD D

Referring to Method D, bromo derivative XIII can be treated with $PPh_3$ in a suitable solvent such as toluene or $CH_3CN$ with warming if necessary to provide phosphonium salt XIV. The phosphonium salt XIV is treated with n-butyllithium then with lactol XV to afford styrene adduct XVI. Alcohol XVI in transformed to ester XVII using conventional methods such as $CrO_3$/pyridine followed by $MnO_2$/NaCN/AcOH/MeOH. Styrene adduct XVII is condensed with thiol VI in the presence of a suitable catalyst such as $AlCl_3$ to give thiol ether XVIII.

When A = CN, XVIII is reduced with a reagent such as $SnCl_2$/HCl to give aldehyde XIX. Quinaldine derivative IX is treated with $PPh_3$ in a suitable solvent such as toluene to give phosphonium salt XX. The

phosphonium salt XX is treated with n-butyl lithium then with aldehyde XIX to give styryl quinoline XXI.

When A = OMe, XVIII is dimethylated using a suitable reagent such as $BBr_3$ to give phenol derivative XXII. Phenol XXII is condensed with quinaldine derivative IX using a suitable catalyst such as $K_2CO_3$ to afford adduct XXIII.

## METHOD E

Referring to Method E, quinaldine derivative II is first treated with LDA and then with bromo derivative XXIV to afford adduct XXV. Cyano derivative XXV is reduced to aldehyde XXVI with a reagent such as $SnCl_2$/HCl. Using the methodology described in Method A or B XXVI is converted to XXVII.

## METHOD F

Reaction of styrylaldehyde III with an alkanoic acid or tetrazole substituted with a thiol or hydroxy group in an inert solvent such as benzene in the presence of a suitable catalyst such as $BF_3$•OEt affords the styrylquinoline derivative XXX.

The groups $Q^1$ and $Q^2$ may be modified by hydrolysis of an ester group, removal of a blocking group, or conversion of a nitrile to an amide or tetrazole by heating with tributyltin azide, thus providing additional examples of the leukotriene antagonists of the present invention. Compound XXX is representative of the structure I compounds.

## METHOD G

Other compounds of Formula I can be prepared as indicated in Method G. Thus the ester derivative XXXI can be reduced to the alcohol XXXII by lithium aluminum hydride or other suitable reducing agents. Alcohol XXXII can then be oxidized to aldehyde XXXII by pyridinium chlorochromate or other suitable oxidizing agents. Carboxylic acids of Formula XXXIa can be converted to the acid chloride XXXIV (the acid bromide or a mixed carbonate anhydride could also be used) which when reacted with diazomethane yields the diazoketone XXXV. Compound XXXV, upon reaction with aqueous acid, preferably a nonnucleophilic acid such as sulfuric acid or p-toluenesulfonic acid, is converted to the hydroxymethyl ketone XXXVI.

Acid chloride XXXIV, upon reaction with a sulfonamide, $R^{13}SO_2NH_2$, in the presence of a weak base yields the acyl sulfonamide XLII. Reaction of XXXIV with an amine,$R^{13}R^{12}NH$, yields amide XXXVII. Amide XXXVII can be sequentially reduced, to amine XXXVIII, with diborane or lithium aluminum hydride, and sulfonylated with $R^{13}SO_2Cl$ to produce sulfonamide XXXIX. Amide XXXVII (when both $R^{12}$ substituents are hydrogen) can be dehydrated by standard reagents to nitrile XL, which is converted to tetrazole XLI by reaction with sodium azide, tri-n-butyltin azide or other suitable methods.

## METHOD H

Compound XI is converted to phosphonium salt XLII by the following sequence of reactions: 1) reduction of the carbonyl group to an alcohol by means of a suitable reducing agent such as $NaBH_4$ or $LiBH_4$; 2) conversion of the alcohol to a bromide with an appropriate reagent combination such as 1,2-bis-(diphenylphosphino)ethane/$CBr_4$; 3) reaction of the bromide with triphenylphosphine. A Wittig olefination reaction between XV and XLII, using a base such as potassium hexamethyldisilazide (KHMDS), yields compound XLIII. Alcohol XLIII is converted to amide XLIV by the sequence: 1) oxidation to the aldehyde using $MnO_2$ in EtOAC; 2) oxidative conversion of the aldehyde to the methyl ester using $MnO_2$/NaCN/AcOH/MeOH/THF; 3) treatment of the resulting ester with an amine $HNR^{12}R^{12}$ or an aluminum amide such as $(CH_3)_2AlNR^{12}R^{12}$ yields amide XLIV. Reaction of XLIV and VI as described in Method D then yields compound XLV.

## METHOD I

Compound XVII is converted to amide XLVI by one of the methods described in Methods G and H.

Hydration of the double bond in XLVI is effected by sequential treatment with $Hg(OAc)_2$ and $NaBH_4$ to yield compound XLVII. Reaction of XLVII with alcohol XLIX, using a catalyst such as $ZnCl_2$ then yields compound L. An alternate synthesis of XLVII involves, first hydration of XVII to XLVIII, followed by amide formation. Compound L can also be prepared by acid-catalyzed addition of XLIX to the double bond in XLVI. Methods of hydration and of alcohol addition to double bonds are described in J. March, Advanced Organic Chemistry, 3rd. ed., John Wiley & Sons, New York, 1985, pp. 681-687.

It is to be noted that intermediate XLVIII may form a seven-membered ring lactone between the alcohol group and the ester group. Such a lactone can also be used to form amide XLVII.

Compound L is transformed to compounds LI or LII by the methodology described in Method D.

METHOD J

The functional groups, representative of $Q^1$ or $Q^2$, which are present in intermediates V, XVI, XVII, XVIII, XIX, XXII, XLIII, XLIV, XLVI, XLVII, XLVIII and L can be transformed to other representatives of $Q^1$ or $Q^2$ by the methodology described in Method G. These transformed intermediates can also be employed, according to the above methods, to prepare compounds of Formula I.

It will be evident to one skilled in the art that the above described methods must be compatible with the other functional groups present in the molecules. Where necessary, such compatibility is achieved by suitable protection and deprotection techniques (see for example, T.W. Greene, Protective Groups in Organic Synthesis, John Wiley & Sons, New York, 1981).

In the following schema, Qu represents

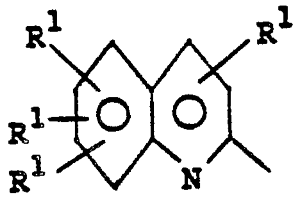 .

METHOD A

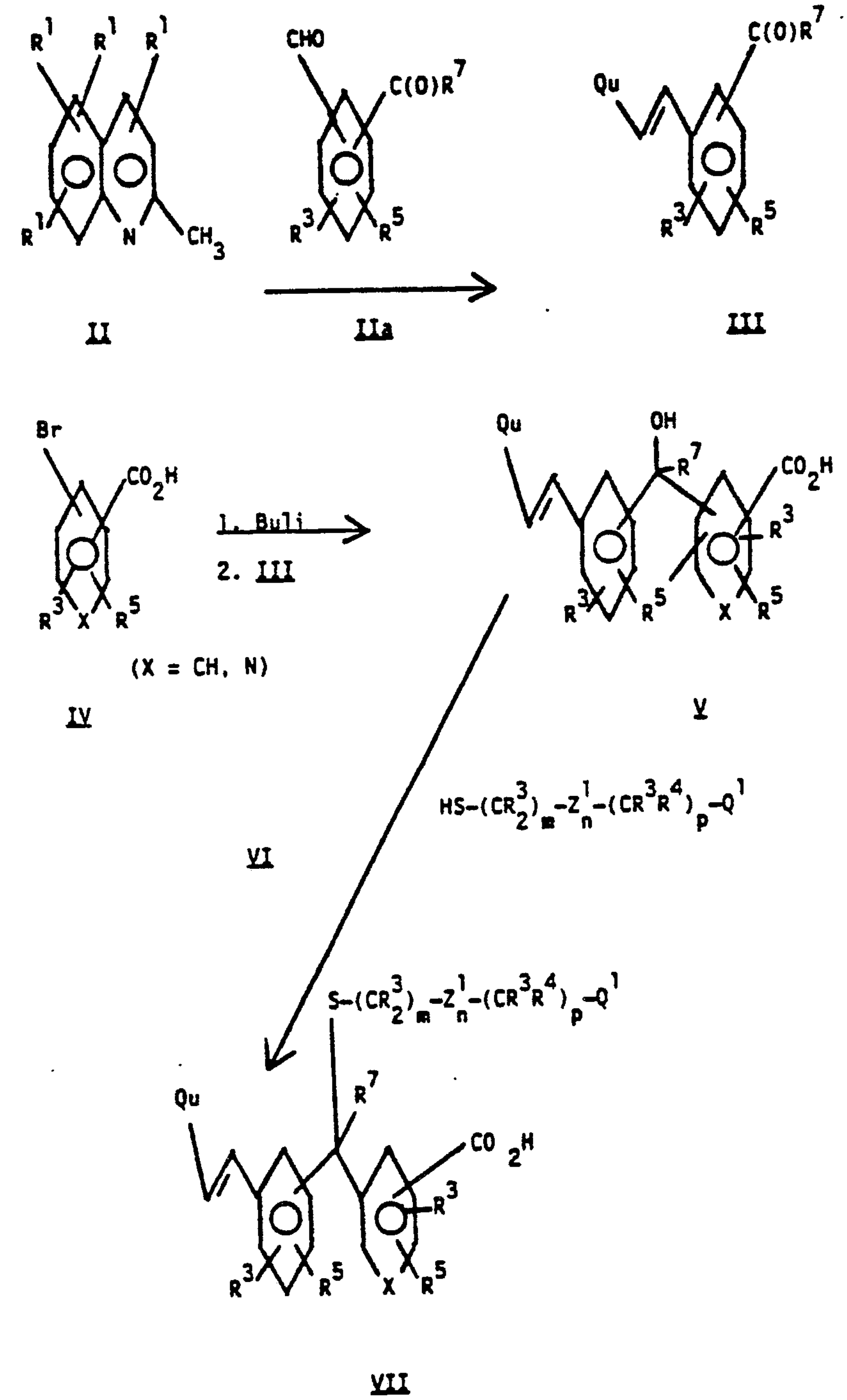

METHOD B

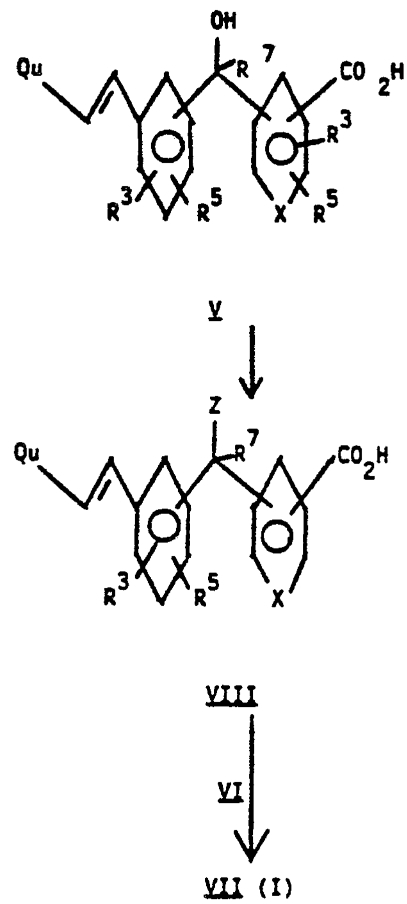

V

VIII

VI

VII (I)

METHOD C

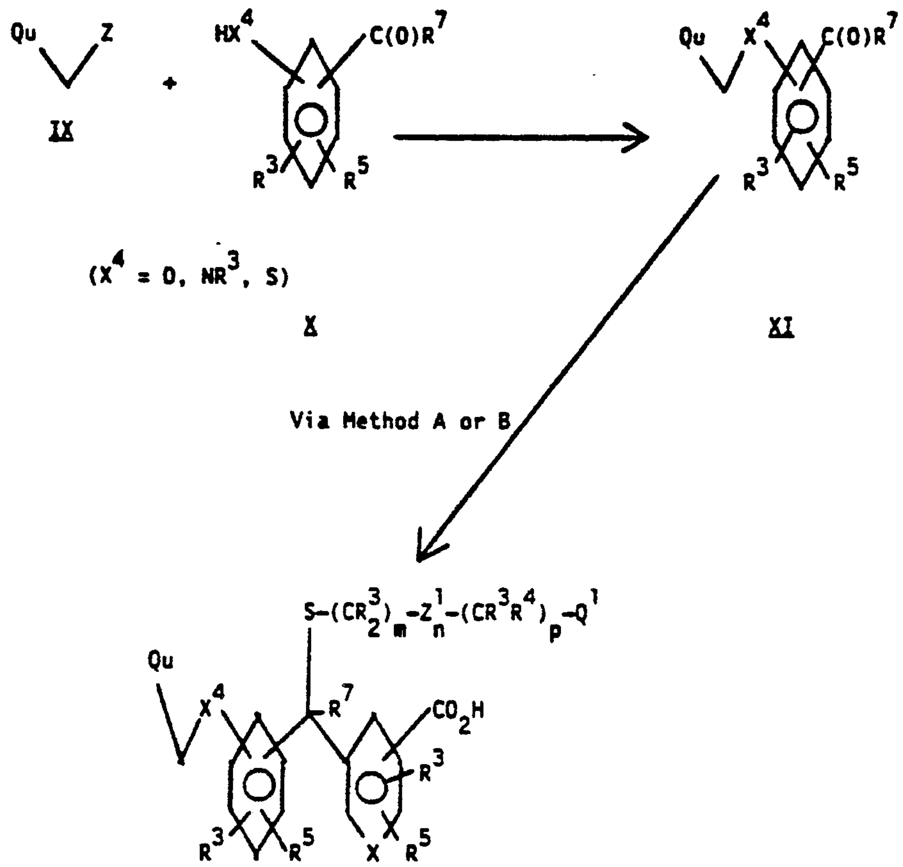


IX

($X^4$ = O, $NR^3$, S)

X

XI

XII (I)

METHOD D

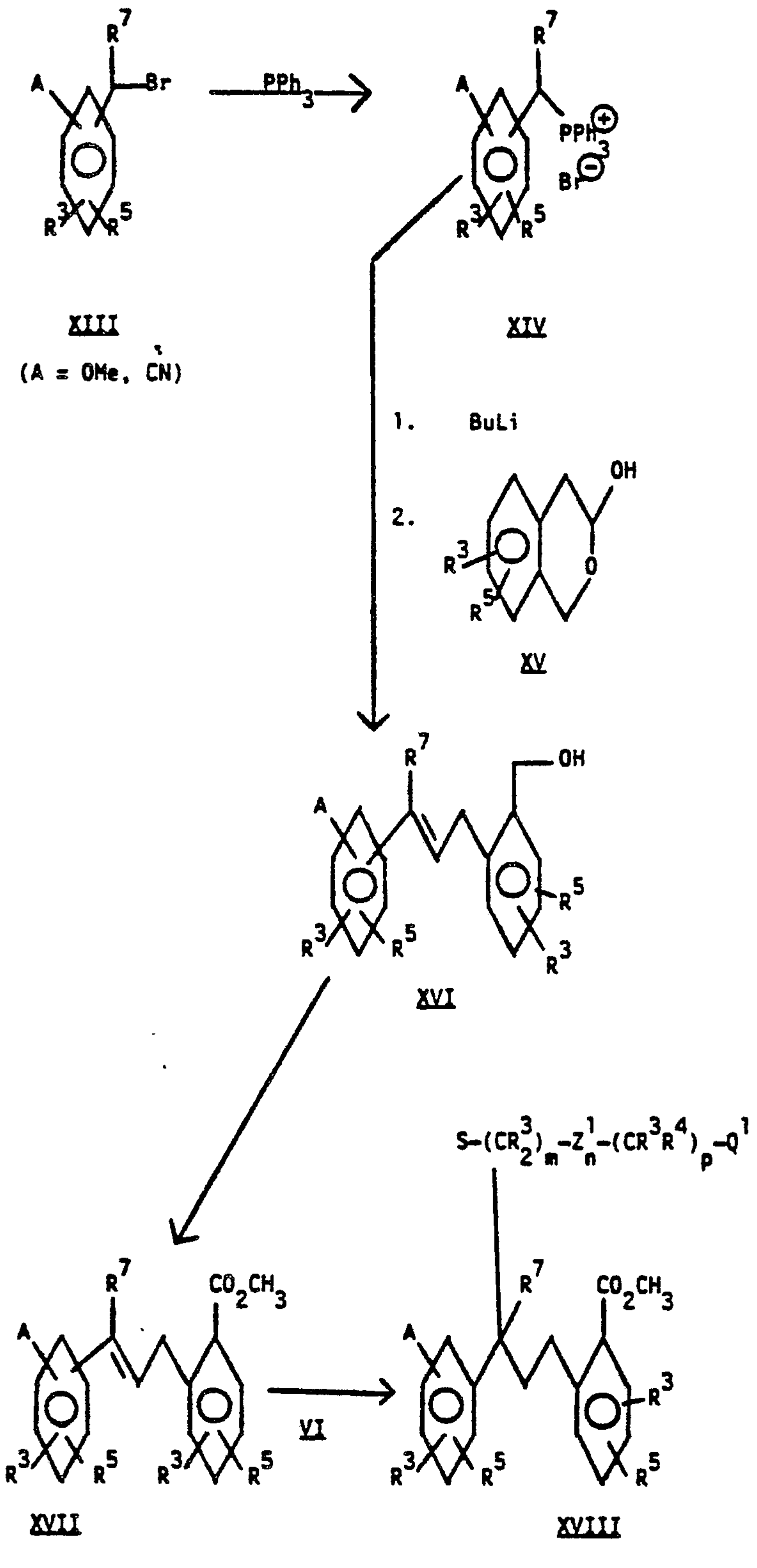


XIII (A = OMe, CN)

XIV

XV

XVI

XVII

XVIII

METHOD D (Cont.)

XVIII (A = CN)

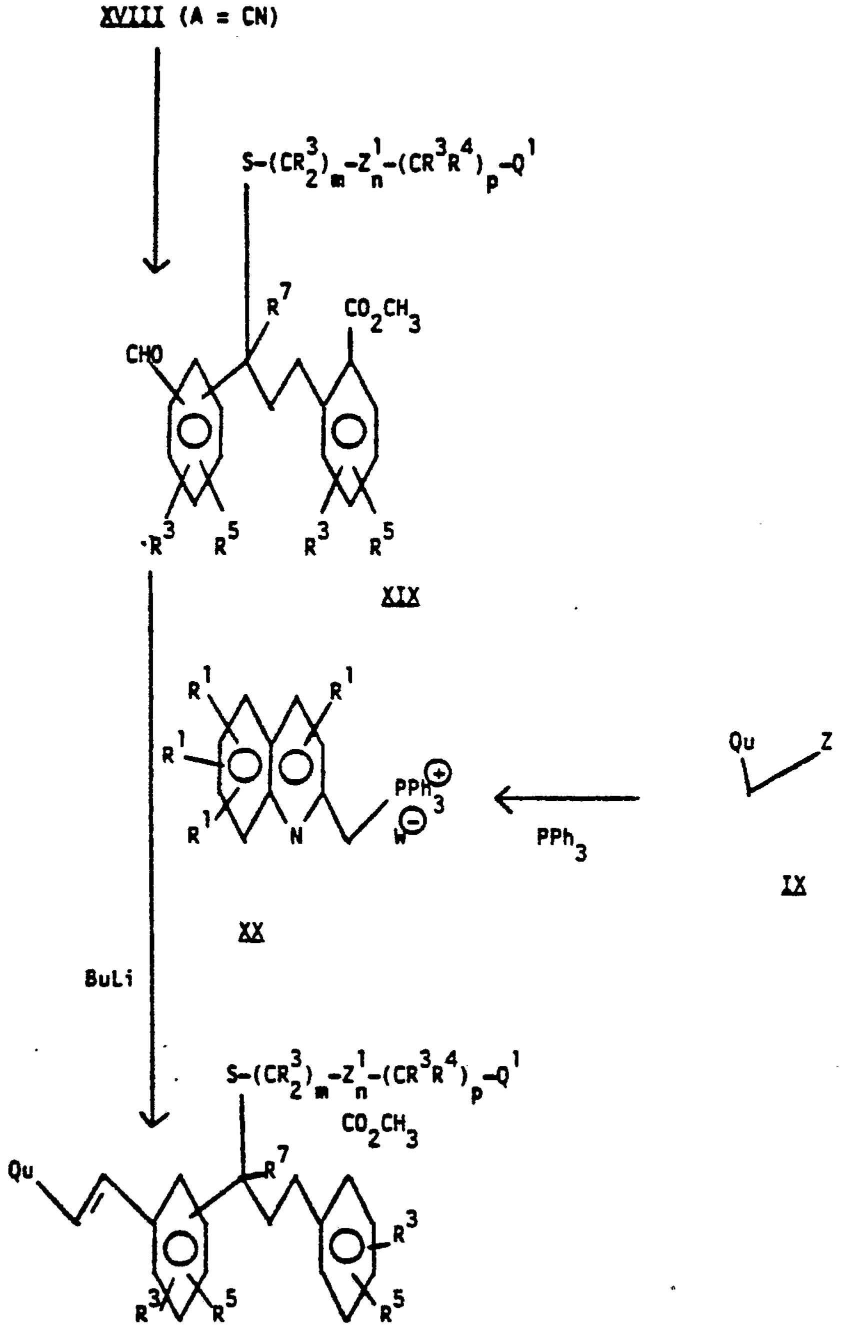


XIX

XX

IX

XXI (I)

## METHOD D (Cont.)

XVIII (A = OMe)

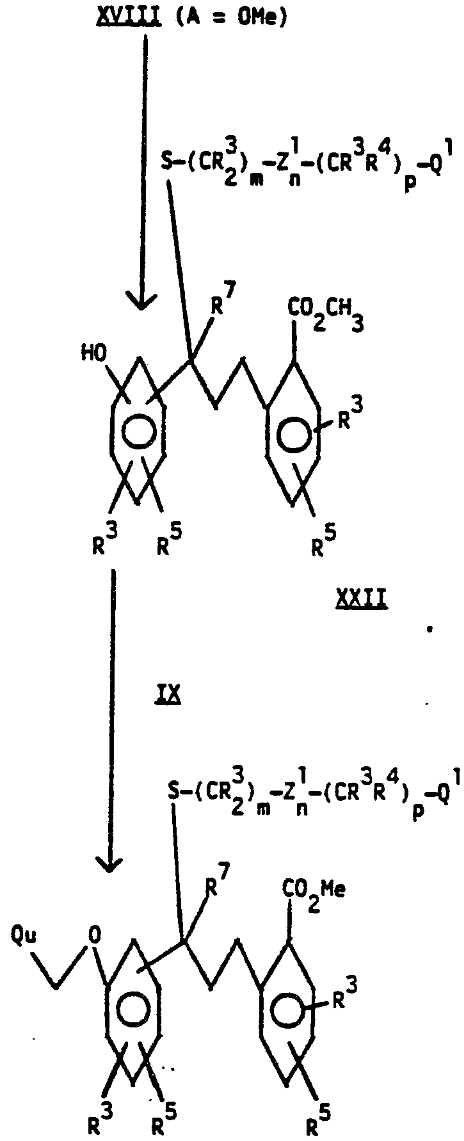


XXII

XXIII (I)

METHOD E

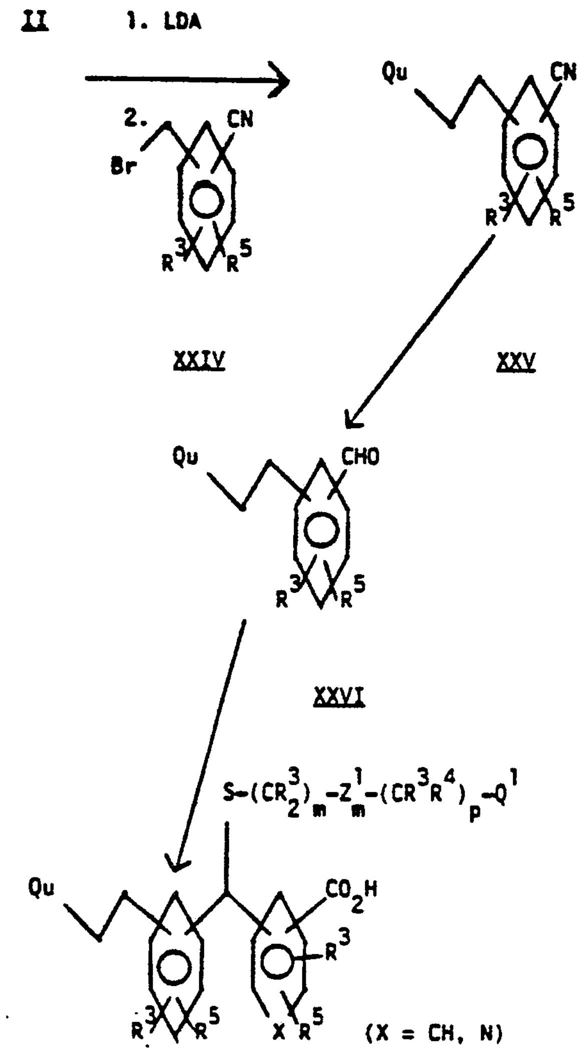


XXIV

XXV

XXVI

XXVII (I)

METHOD F

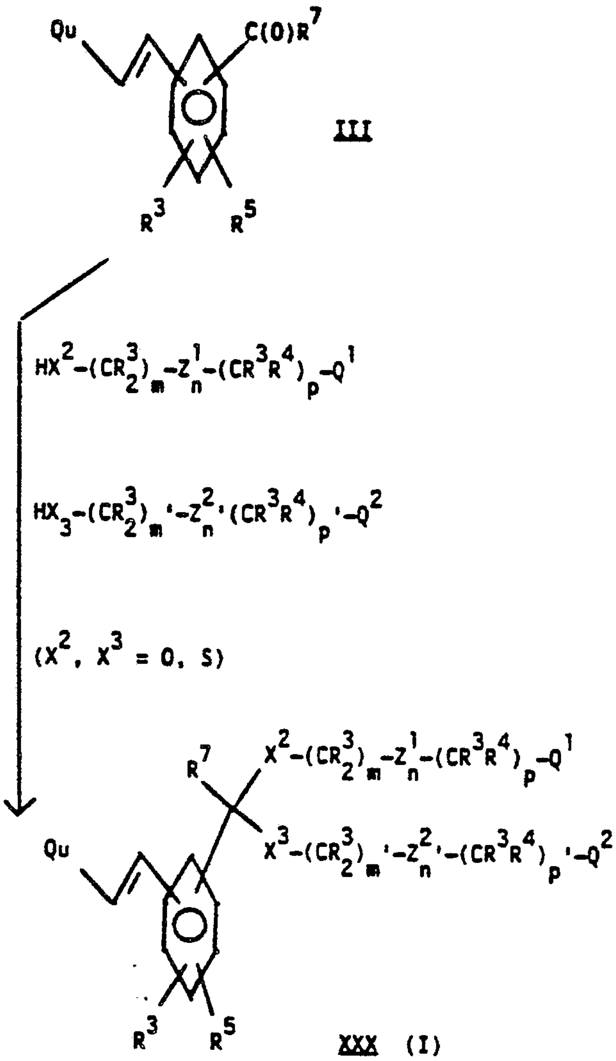

XXX (I)

METHOD G

QP below represents the residual structure of VII, XII, XXI, XXIII, XXVII or XXX in which $Q^1$ or $Q^2$ was $CO_2R^3$ (XXXI)

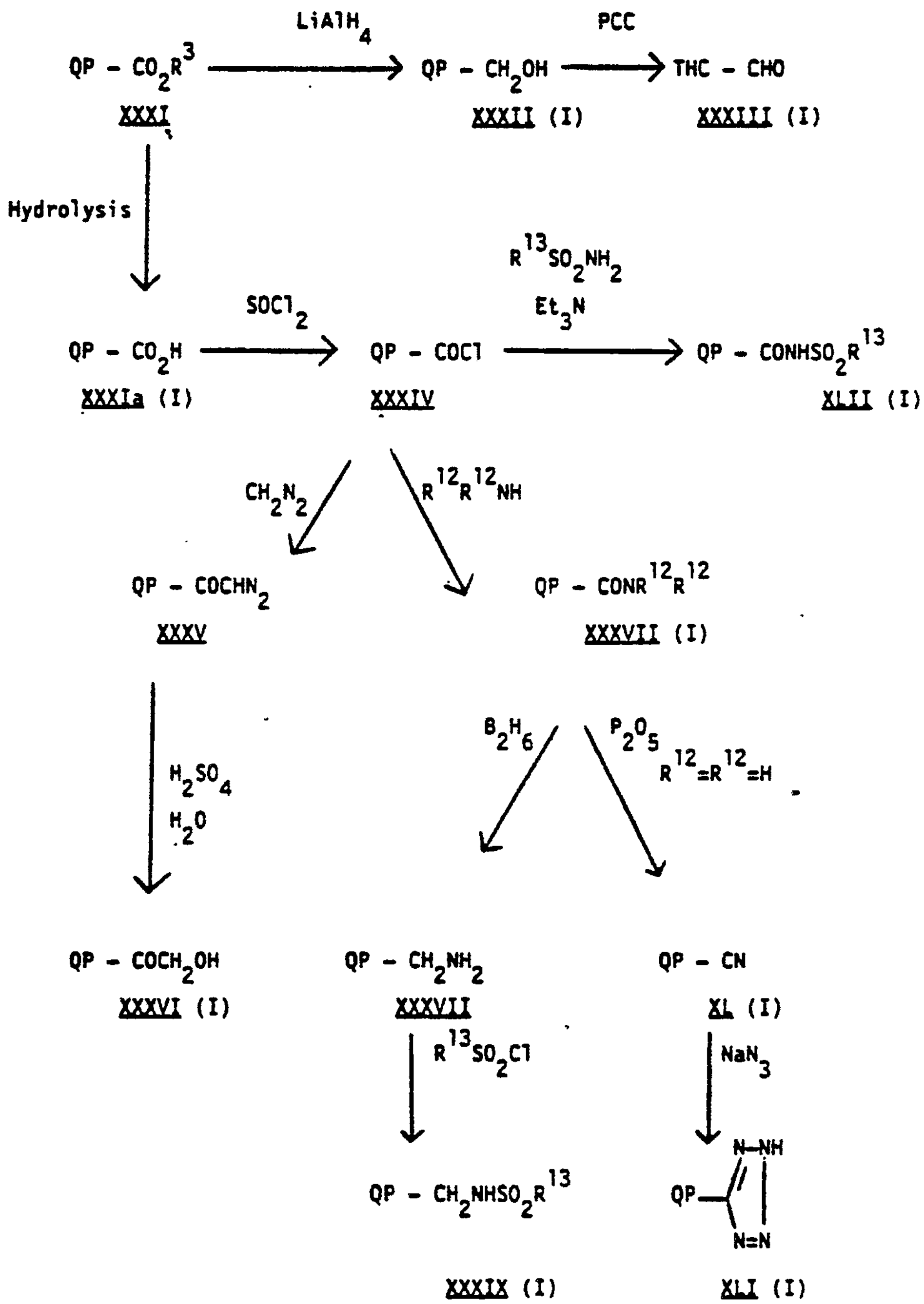
LiAlH4
PCC
XXXI
XXXII (I)
XXXIII (I)
Hydrolysis
R13SO2NH2
Et3N
SOCl2
XXXIa (I)
XXXIV
XLII (I)
CH2N2
R12R12NH
XXXV
XXXVII (I)
B2H6
P2O5
R12=R12=H
H2SO4
H2O
XXXVI (I)
XXXVII
XL (I)
R13SO2Cl
NaN3
XXXIX (I)
XLI (I)

METHOD H

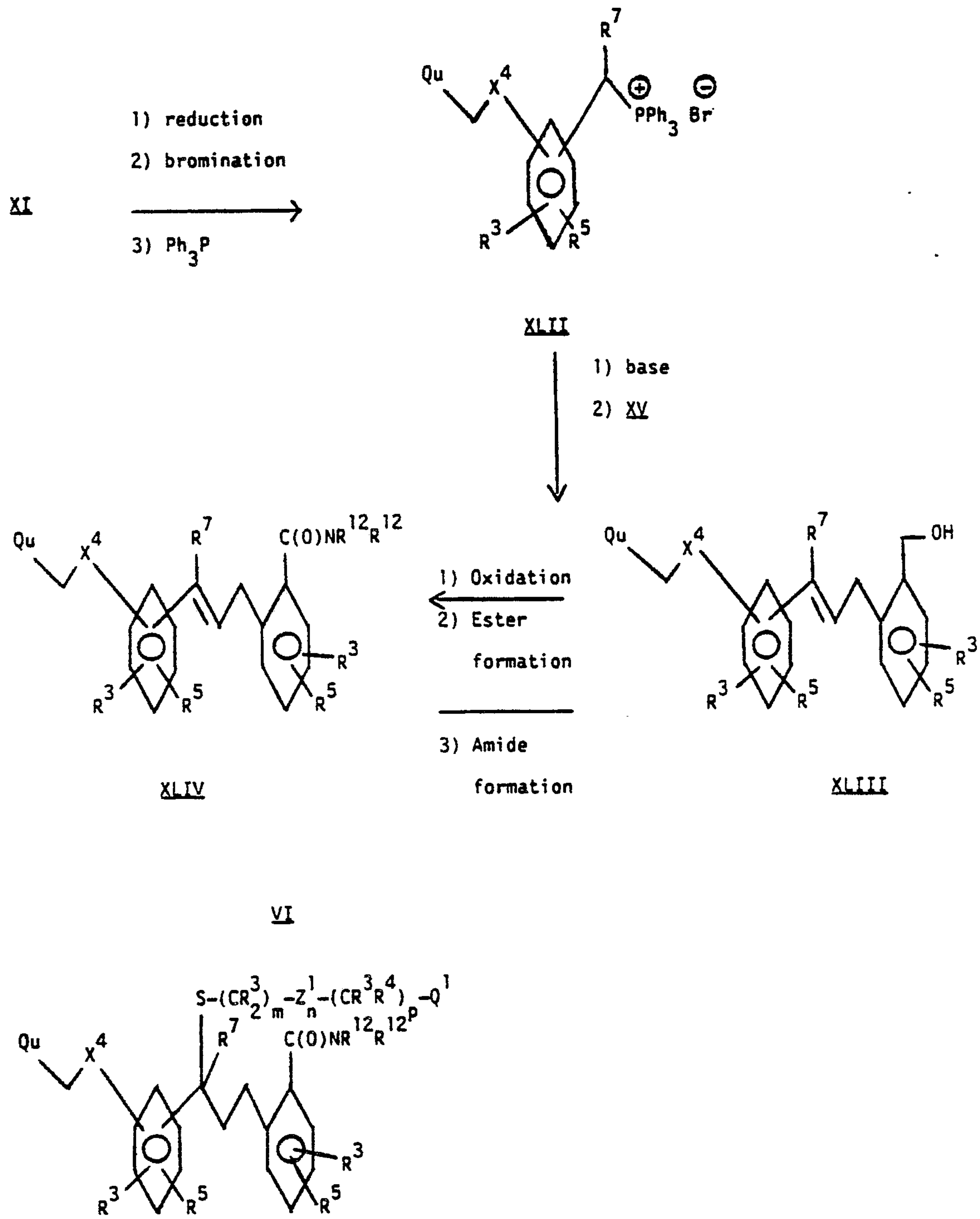


XI

XLII

XLIV

XLIII

VI

XLV (I)

METHOD I

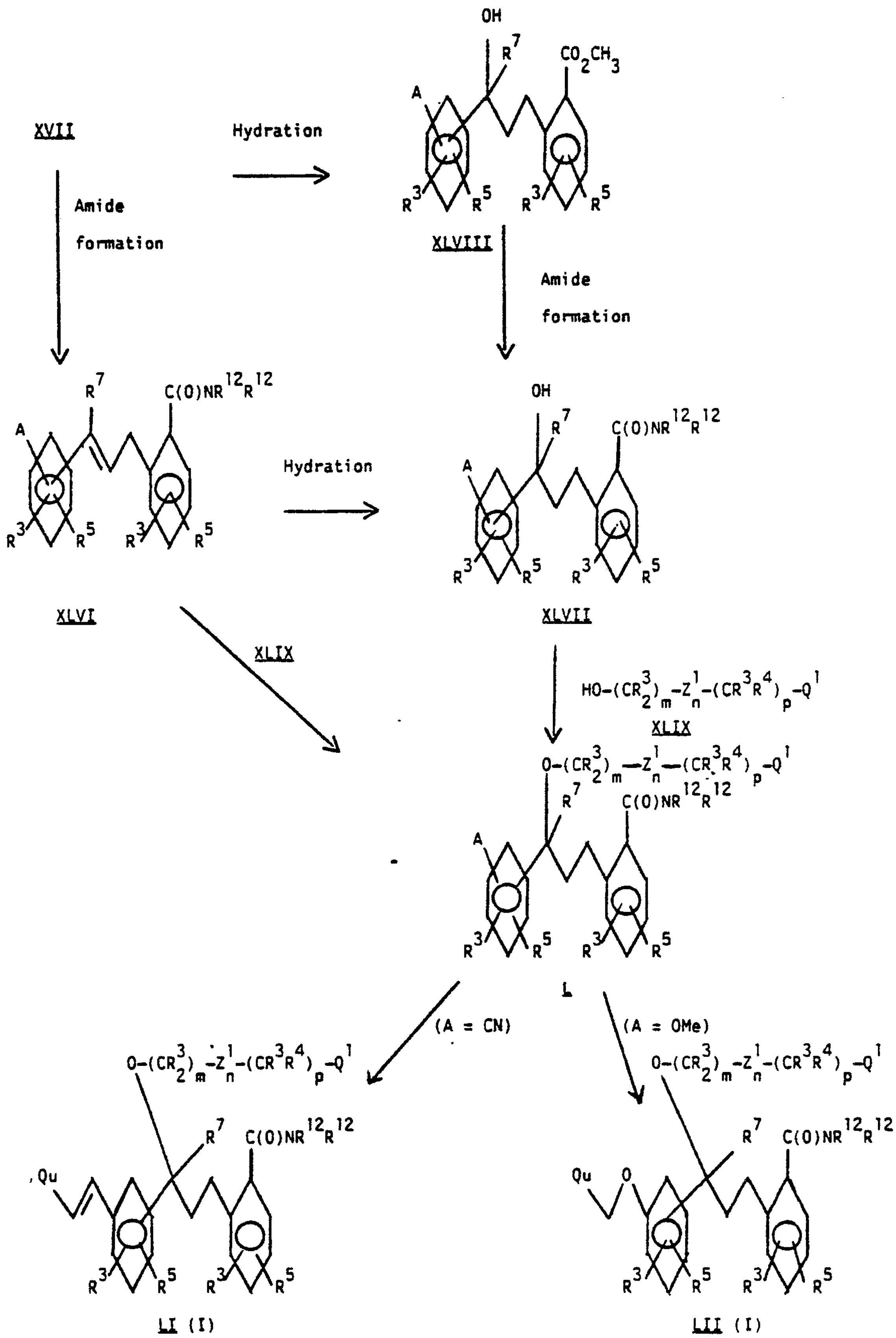
XVII
Hydration
Amide formation
XLVIII
Amide formation
Hydration
XLVI
XLVII
XLIX
$HO-(CR^3_2)_m-Z^1_n-(CR^3R^4)_p-Q^1$
XLIX
L
(A = CN)
(A = OMe)
LI (I)
LII (I)

The invention is further defined by reference to the following examples, which are intended to be illustrative and not limiting.

All temperatures are in degrees Celsius.

## Example 1

Preparation of 2-(3-(3-(2-(7-chloroquinolin-2-yl)ethenyl)phenyl)-3-(2-carboxyethylthio)propyl)benzoic acid, disodium salt

Step 1 Preparation of (3-cyanophenylmethyl) triphenylphosphonium bromide

To a solution of 3-bromomethylbenzonitrile (19.6 g) in $CH_3CN$ (500 ml) triphenylphosphine ($Ph_3P$) was added (30 g). The reaction mixture was stirred at 60° cooled and filtered. The title product thus obtained was dried and used as such for the next step.

Step 2 Preparation of 2-(1-(3-cyanophenyl)propen-3-yl)benzyl alcohol

To phosphonium salt (step 1) (13.4 g) in tetrahydrofuran (THF) (100 mL) at -78° was added potassium hexamethyldisilazide (KHMDS) (0.6 M in toluene) (50 mL). The reaction mixture was warmed to 0° for 1 hr. After cooling to -78°, 1H-3-hydroxy-3,4-dihydrobenzo(c)pyran (2 g) in THF (10 mL) was added. The mixture was warmed to room temperature (RT) for 1 hr, poured onto pH 7 buffer, extracted with ethyl acetate, dried and evaporated. Flash chromatography using 20% ethyl acetate in toluene afforded the title compound.
p.m.r. ($CDCl_3$) $\delta$ (ppm): 1.8 (m, 1H), 3.7 (m, 2H), 5.64 (d, 1H), 5.84 (d, 1H), 5.9-6.6 (m, 2H), 7.1-7.7 (m, 8H).

Step 3 Preparation of 2-(1-(3-cyanophenyl)propen-3-yl))benzaldehyde

To a suspension of pyridinium chlorochromate (PCC) (10 g) and 4A powdered molecular sieves in $CH_2Cl_2$ (200 mL) was added the alcohol from step 2. The mixture was stirred 1 hr at room temperature, ether was added and the mixture was filtered through a pad of $SiO_2$ using 30% ethyl acetate hexane as eluant. The filtrate was evaporated to afford the title compound which was used as such for the next step.

Step 4 Preparation of methyl-2-(1-(3-cyanophenyl)propen-3-yl)benzoate

To a solution of aldehyde (step 3) in MeOH (200 mL). AcOH (1.2 mL) and NaCN (4 g) was added $MnO_2$ (20 g). The mixture was stirred for 2 hrs and poured onto $H_2O$ (1 L). The aqueous phase was extracted with ethyl acetate (2 x 50 mL) and the combined organic phases were dried and evaporated. Flash chromatography of the residue using 5% ethyl acetate in toluene afforded the title compound as a mixture of cis and trans isomers.
p.m.r. ($CDCl_3$) $\delta$ (ppm): 3.4 and 3.6 (s, 3H). 3.7 and 3.9 (dd, 2H), 6.2-6.8 (m, 2H), 7.4-7.8 (m 7H), 8.1 (m. 1H).

Step 5 Preparation of methyl 2-(3-(2-(methoxycarbonyl)ethylthio)-3-(3-cyanophenyl)propyl)benzoate

To a solution of olefin (step 4) (367 mg) in $CH_2Cl_2$ (10 mL) was added methyl 3-mercaptopropionate (200 mg) and $AlCl_3$ (0.7 g). The mixture was stirred for 3 hrs. at room temperature, quenched with 25% aq. $NH_4OAc$ and extracted with ethyl acetate. The organic phase was dried and evaporated. Flash chromatography of the residue using 10% ethyl acetate in toluene afforded the title compound.
p.m.r. ($CDCl_3$) $\delta$ (ppm): 2.0-2.3 (m, 2H), 2.4-2.7 (m, 4H), 2.8-3.1 (m, 2H), 3.7 (s, 3H), 3.9 (s, 3H), 3.9 (t, 1H), 7.1-7.7 (m, 7H), 7.9 (d, 1H).

Step 6 Preparation of methyl 2-(3-(2-(methoxycarbonyl)ethylthio)-3-(3-formylphenyl)propyl)benzoate

HCl (gas) was bubbled into a suspension of $SnCl_2$ (1.2 g) in ether until 2 layers were formed. The cyano compound (350 mg) (step 5) was then added. The mixture was stirred 3 hours at room temperature and carefully quenched with $H_2O$ at 0°. The reaction mixture was poured onto pH 7 buffer (300 mL), extracted with ethyl acetate (200 mL), and the organic phase was dried and evaporated. Flash chromatography of the residue using 25% ethyl acetate in hexane afforded the title compound.
p.m.r. ($CDCl_3$) $\delta$ (ppm): 2.2-2.3 (m, 2H), 2.5-2.7 (m, 4H), 2.85-3.2 (m, 2H), 3.75 (s, 3H), 3.95 (s, 3H), 4.0 (s, 1H), 7.2-8.0 (m, 8H), 10.1 (2, 1H).

Step 7 Preparation of methyl 2-(3-(3-(2-(7-chloroquinolin-2-yl)ethenyl)phenyl)-3-(2-(methoxycarbonyl)-ethylthio)propyl)benzoate

To a suspension of ((7-chloroquinolin-2-yl)methyl)triphenylphosphonium bromide (EP 233,763, Example 4, step 2) (489 mg) in THF (5 ml) at -78° was added butyllithium (.51 ml of 1.6N). The reaction mixture was stirred at -78° 1 hr and aldehyde (350 mg) (step 6) in THF (2 ml) was added. The mixture was warmed to RT, poured onto buffer (pH 7), extracted with ethyl acetate, and the organic phase was dried and evaporated. Flash chromatography using 25% ethyl acetate/hexane afforded the title compound.
p.m.r. ($CD_3COCD_3$) $\delta$ (ppm): 2.3-2.4 (m, 2H), 2.5-2.7 (m, 4H), 2.85-3.15 (m 2H), 3.65 (s, 3H), 3.85 (s, 3H), 4.05 (t, 1H), 7.3-8.0 (m, 13H), 8.05 (d, 1H), 8.3 (d, 1H).

Steps 8

To the diester (step 7) in THF (5 mL) and MeOH (5 mL) was added LiOH (5 mL of 1N). The solution was stirred 3 days at RT partitioned between EtOAc/$H_2O$ (acidified with AcOH), and the organic phase was dried and evaporated to give the diacid. To the diacid was added 2 eq of NaOH and the solution was freeze dried to give the title compound.

| Anal. Calcd. for $C_{30}H_{24}NO_4SNa_2Cl\bullet 3H_2O$: | | | | | | | | |
|---|---|---|---|---|---|---|---|---|
| | C | 57.18; | H | 4.79; | N | 2.22; | Na | 7.28 |
| Found | C | 57.70; | H | 4.63; | N | 2.13; | Na | 7.52. |

Example 2

Preparation 2-(3-(3-(2-(7-chloroquinolin-2-yl)ethenyl)phenyl)-3-(2-(dimethylcarbamoyl)ethylthio) propyl)-benzoic acid, sodium salt

Using the procedure of Example 1 but replacing methyl 3-mercaptopropionate with 3-mercapto-N,N-dimethylpropionamide in step 5 and using 1 eq. of NaOH in step 8 instead of 2 eq. NaOH there was obtained the title compound.
p.m.r. ($CD_3COCD_3$) $\delta$ (ppm): 2.2-2.3 (m, 2H), 2.4-2.7 (m, 4H), 2.8 (s, 3H), 2.9 (s, 3H), 2.85-3.2 (m, 2H), 4.05 (t, 1H), 7.0-8.0 (m, 13H), 8.0 (d, 1H), 8.3 (d, 1H).

Example 3

3-(3-(2-(7-chloroquinolin-2-yl)ethyl)phenyl)-(2-(dimethylcarbamoyl)ethylthio)methyl)benzoic acid, sodium salt

Step 1 Preparation of 3-(2-(7-chloroquinolin-2-yl)ethyl)benzonitrile

To 7-chloroquinaldine (18 g) in THF (200 mL) at -78° was added lithium diisopropylamide (LDA) 0.1M). The reaction mixture was stirred for 30 min at -78° and added dropwise to a solution of 3-(bromomethyl)-benzonitrile (19.6 g) in THF (200 mL) at 0°. The mixture was stirred 2 hrs at 0°, quenched with 25% ag. $NH_4OAc$, extracted with ethyl acetate (500 mL) and the organic phase was dried and evaporated. Flash chromatography using 20% ethyl acetate/hexane afforded the title compound.
p.m.r. ($CDCl_3$) $\delta$ (ppm): 3.3-3.4 (m, 4H), 7.0-8.2 (m, 9H).

Step 2 Preparation of 3-(2-(7-chloquinolin-2-yl)ethyl)benzaldehyde

To a solution of nitrile (step 1) (10 g) in formic acid (150 mL) and $H_2O$ (50 mL) was added Ni-Al alloy (6 g). The reaction mixture was heated at 130° for 2 days, filtered and evaporated. The residue was partitioned between ethyl acetate (500 mL) and aqueous $NaHCO_3$, and the organic phase was dried and evaporated. Flash chromatography using 25% ethyl acetate hexane afforded the title aldehyde which was used as such for the next step.

Step 3 Preparation of 3-((3-(2-(7-chloroquinolin-2-yl)ethyl)phenyl)hydroxymethyl)benzoic acid

To a solution of 3-bromobenzoic acid (0.8 g) at -100° in THF (20 mL) was added dropwise 2 eq. of n-butyllithium in hexane. The mixture was warmed to -78° and aldehyde (1 g) (step 2) in THF (5 mL) was added dropwise over 15 min. After stirring 2 hrs at -78° the reaction mixture was quenched with buffer (25% aq. $NH_4OAc$), extracted with ethyl acetate, and the organic phase was dried and evaporated. Flash chromatography of the residue using 15% to 25% acetone/toluene/acetic acid 0.1% afforded the title compound.
p.m.r. ($CDCl_3$) $\delta$ (ppm): 3.1 (m, 2H), 3.3 (m, 2H), 5.8 (s, 1H), 6.0-7.0 (bs, 1H), 7.05-7.60 (m, 8H), 7.70 (d, 1H), 8.0 (m, 2H), 8.1-8.2 (m, 2H).

Step 4

To a solution of alcohol (step 3) (0.4 g) in $CH_2Cl_2$ (25 mL) was added 3-mercapto-N,N-dimethylpropionamide (0.2 mL) and $AlCl_3$ (800 mg). The reaction mixture was stirred 1 hr at RT, and quenched with 25% $NH_4OAc$ (100 mL)/AcOH (2 mL)/THF (50 mL) and EtOAc (200 mL). The organic phase was separated dried and evaporated. Flash chromatography of the residue using 25% to 40% acetone/toluene/acetic acid (0.1%) afforded the acid of the title compound.
p.m.r. ($CD_3COCD_3$) $\delta$ (ppm): 2.2-2.4 (m, 4H), 2.55 (s, 3H), 2.65 (s, 3H), 2.8-3.0 (m, 4H), 5.15 (s, 1H), 7.0-8.0 (m, 13H).

Step 5

The acid was treated with NaOH (1 eq.) in $H_2O$/EtOH, evaporated and freeze dried to give the title compound.

| Anal. Calcd. for $C_{30}H_{28}N_2SO_3ClNa\bullet H_2O$: | | | | | | | | |
|---|---|---|---|---|---|---|---|---|
| | C | 62.75; | H | 5.45; | N | 4.86; | Na | 4.00 |
| Found | C | 62.35; | H | 5.38; | N | 5.30; | Na | 3.53. |

Example 4

5-((3-(2-(7-chloroquinolin-2-yl)ethenyl)phenyl)(2-(dimethylcarbamoyl)ethylthio)methyl)thiophene-2-carboxylic acid

Step 1 Preparation of 5-((3-(2-(7-chloroquinolin-2-yl)ethenyl)phenyl)hydroxymethyl)thiophene-2-carboxylic acid

At -78° C, BuLi, 1.6M in hexanes (7.5 mL, 2.3 equiv.) was added dropwise to a solution of thiophene-2-carboxylic acid (0.784 g, 1.2 equiv.) in THF (20 mL) and the mixture was stirred at -78° C for 30 minutes. Then a solution of 3-(2-(7-chloro-2-quinolinyl)ethenyl) benzaldehyde (EP 233,763, Example 24, step 1) (1.515 g, 5.15 mmoles) in THF (25 mL) was added dropwise. Stirring was continued for an hour at -78° C and the reaction was quenched with 25% aqueous $NH_4OAc$. The mixture was acidified to pH 5 with acetic acid and extracted with EtOAc. The organic fraction was dried over $Na_2SO_4$ and evaporated. Flash chromatography on silica using EtOAc:toluene:AcOH 30:70:1 and 40:60:1 yielded the title compound.
$^1H$ NMR ($CD_3COCD_3$) $\delta$ (ppm): 6.80 (s, 1H), 7.32-7.65 (m, 6H), 7.70 (d, 1H), 7.82-8.04 (m, 5H), 8.33 (d, 1H).

Step 2

At -10° C, $AlCl_3$ (2.323 g, 8 equiv.) was added to a solution of the hydroxyacid of step 1 (915 mg, 2.17 mmoles) and 3-mercapto-N,N-dimethylpropion amide (587 mg, 2 equiv.) in $CH_2Cl_2$ (45 mL) and the mixture was stirred at 0° C for 1.5 hours. An oil separated, which was collected with a spatula and quenched with THF:25% aqueous $NH_4OAc$ 1:1. The remaining reaction mixture was stirred 30 minutes at room temperature and quenched at 0° C with 25% aqueous $NH_4OAc$. The solutions were combined, acidified with acetic acid and extracted with EtOAc. Drying the organic phase over $Na_2SO_4$ and flash chromatography of the residue using acetone:toluene:AcOH 20:80:1 and 30:70:1 afforded the title acid.
$^1H$ NMR ($CD_3COCD_3$) $\delta$ (ppm): 2.60 (m, 2H), 2.70 (m, 2H) 2.84 (s, 3H), 2.96 (s, 3H), 6.60 (s, 1H), 7.37-7.58 (m, 5H), 7.63 (d, 1H), 7.77 (d, 1H), 7.82-8.03 (m, 5H) 8.33 (d, 1H).

Example 5

3-((3-(2-(7-chloroquinolin-2-yl)ethenyl)phenyl)(2-(dimethylcarbamoyl)ethylthio)methyl)benzoic acid

Step 1 Preparation of 3-((3-(2-(7-chloroquinolin-2-yl)ethenyl)phenyl)hydroxymethyl)benzoic acid

To the dilithium salt (5.92 mmoles) obtained from 3-bromobenzoic acid (W.E. Parham and Y.A. Sayed, J. Org. Chem., 39, 2051 (1974)), a solution of 3-(2-(7-chloro-2-quinolinyl)ethenyl)benzaldehyde (1.503 g, 5.12 mmoles) in THF (25 mL) was added dropwise at -78° C. The mixture was stirred 2 hours at -78° C and was quenched with 25% aqueous $NH_4OAc$. The mixture was acidified to pH 5 with AcOH and extracted with EtOAc. The organic fractions were dried over $Na_2SO_4$ and evaporated. Flash chromatography on silica using EtOAc:toluene:AcOH 30:70:1 yielded the title compound.
$^1H$ NMR ($CD_3COCD_3$•DMSO-d6) $\delta$ (ppm): 5.90 (s, 1H), 6.00 (s, 1H, OH), 7.36-7.58 (m, 5H), 7.62 (d, 1H), 7.73 (d, 1H), 7.82-8.02 (m, 6H), 8.13 (s, 1H), 8.37 (d, 1H).

Step 2

At 0° C, $AlCl_3$ (1.182 g, 7.5 equiv.) was added to a suspension of the hydroxyacid of step 1 (492 mg, 1.183 mmoles) and 3-mercapto-N,N-dimethylpropionamide (327 mg, 2 equiv.) in $CH_2Cl_2$ (12 mL). The mixture was stirred at 0° C for 1.5 hours and was quenched with THF:25% aqueous $NH_4OAc$. Acidification to pH 5 with AcOH, extraction with EtOAc, drying the organic phase over $Na_2SO_4$ and flash chromatography on silica using acetone:toluene: AcOH 20:80:1 afforded the title acid.
$^1H$ NMR ($CD_3COCD_3$) $\delta$ (ppm): 2.63 (m, 2H), 2.73 (m, 2H), 2.83 (s, 3H), 2.95 (s, 3H), 5.62 (s, 1H), 7.40-7.56 (m, 5H), 7.65 (d, 1H), 7.79-8.03 (m, 7H), 8.22 (s, 1H), 8.34 (d, 1H).

## Example 6

### 4-((3-(2-(7-chloroquinolin-2-yl)ethenyl)phenyl)(2-(dimethylcarbamoyl)ethylthio)methyl)benzoic acid

Using the same procedure as for Example 5, but substituting 3-bromobenzoic acid by 4-bromobenzoic acid in step 1, the title compound was prepared.
$^1$H NMR ($CD_3COCD_3$) δ (ppm): 2.62 (m, 2H), 2.72 (m, 2H), 2.84 (s, 3H), 2.95 (s, 3H), 5.59 (s, 1H), 7.38-7.57 (m, 4H), 7.60-7.73 (m, 3H), 7.82-8.07 (m, 7H), 8.33 (d, 1H).

## Example 7

### 3-((2-carboxyethylthio)(3-(2-(7-chloroquinolin-2-yl)ethenyl)phenyl)methyl)benzoic acid

To a suspension of the hydroxyacid of Example 5, step 1, (193 mg, 464 µmoles) in $CH_2Cl_2$ (5 mL), 3 mercaptopropionic acid (45 µL, 1.1 equiv.) was added, followed by $AlCl_3$ (254 mg, 4 equiv.) and 2,6-di-tert-butyl-4-methylphenol (23 mg, 0.2 equiv.). The reaction mixture was stirred at room temperature 6.7 hours. Then, at 0°C, THF was added, followed by 25% aqueous $NH_4OAc$. The mixture was acidified to pH 5 with AcOH and was extracted with EtOAc. Drying of the organic phase over $Na_2SO_4$ and flash chromatography of the residue on silica using acetone:toluene:AcOH 10:90:1 afforded the title diacid.
$^1$H NMR ($CD_3COCD_3$•DMSO) δ (ppm): 2.57 (m, 2H), 2.69 (m, 2H), 5.62 (s, 1H), 7.40-7.58 (m, 5H), 7.66 (d, 1H), 7.81 (d, 1H), 7.85-8.04 (m, 6H), 8.20 (s, 1H), 8.35 (d, 1H).

## Example 8

### 6-(3-carboxyphenylthio)-6-(3-(2-(7-chloroquinolin-2-yl)ethenyl)phenyl)-3-methylhexanoic acid

#### Step 1 Preparation of methyl 3-methyl-5-(methylsulfonyloxy)pentanoate

To methyl 5-hydroxy-3-methylpentanoate (B. Lythgoe, J. Chem. Soc., Perkin Trans. I, 834 (1978)) (9.63 g, 65.9 mmoles) in 200 mL $CH_2Cl_2$ at -78°C, $Et_3N$ (14 mL, 1.5 equiv.) and methanesulfonyl chloride (5.6 mL, 1.1 equiv.) were added. After one hour of stirring at room temperature, 25% $NH_4OAc$ was added. Extraction with $CH_2Cl_2$, filtration of the organic phase through silica and evaporation afforded the title compound, which was used as such in the next step.

#### Step 2 Preparation of methyl 5-iodo-3-methylpentanoate

The mesylate (step 1, 14.4 g, 64.2 mmoles) and NaI (48 g, 5 equiv.) were heated to reflux in 200 mL acetone for 3 hours. The mixture was then filtered through celite and the solvent evaporated. The residue was partionned between water and $Et_2O$, the ether extract washed with 5% $Na_2S_2O_3$ and brine, dried and evaporated. Flash chromatography of the residue on silica with EtOAc:hexane 2.5:97.5 afforded the title compound.
$^1$H NMR (90 MHz, $CDCl_3$) δ (ppm): 1.00 (d, 3H), 1.70-2.43 (m, 5H), 3.20 (t, 2H), 3.67 (s, 3H).

#### Step 3 Preparation of (5-methoxy-3-methyl-5-oxo-pentyl)triphenylphosphonium iodide

Triphenylphosphine (14.6 g, 2 equiv.) and the iodide (step 2, 7.85 g, 27.6 mmoles) were heated to 80°C in 50 mL of toluene for 24 hours and at 100°C 6 hours. The mixture was allowed to cool to room

temperature, the toluene layer was discarded and the remaining oil heated in toluene for another hour. After cooling to room temperature, the toluene layer was removed. The remaining oil was heated for one hour in ether, the ether was removed at room temperature and the remaining oil dried under vacuum.
$^1$H NMR ($CDCl_3$) δ (ppm): 1.09 (d, 3H), 1.50-2.00 (m, 3H), 2.26-2.45 (m, 2H). 3.59 (s, 3H), 3.5-3.85 (m, 2H), 7.69-7.90 (m, 15H).

Step 4 Preparation of methyl 6-(3-cyanophenyl)-3-methyl-5-hexenoate

Under a continuous flow of $N_2$ at -78°C, KHMDS (0.684 M in toluene, 11 mL, 1.3 equiv.) was added dropwise to a solution of the phosphonium salt (step 3, 0.133 M in THF:HMPA 10:1, 62 mL, 1.4 equiv.) and 3-cyanobenzaldehyde (7.702 g, 58.7 mmoles) over a period of 30 minutes. The mixture was then allowed to warm to room temperature and was stirred. for a further 2 hours. 25% aqueous $NH_4OAc$ was added and the aqueous layer was extracted with EtOAc. The organic layer was washed twice with brine, dried over $Na_2SO_4$ and evaporated. The residue was purified by flash chromatography on silica using EtOAc:hexane 7.5:92.5 and 10:90 to afford the title product.
$^1$H NMR ($CDCl_3$) δ 0.98 (d, 3H), 2.07-2.42 (m, 5H), 3.66 (s, 3H), 5.79 (td, 1H), 6.48 (d, 1H). 7.40-7.59 (m, 4H) p.p.m.

Step 5 Preparation of O-(3-(methoxycarbonyl)phenyl)dimethylcarbamothioate

At 0°C, NaH (59.6% in oil, 2.975 g, 1.1 equiv.) was added portionwise to a solution of methyl 3-hydroxybenzoate (10.22 g, 67.2 mmoles) in dimethylformamide (70 mL) and the mixture was stirred 30 minutes at room temperature. Then, dimethylthiocarbamoyl chloride (11.95 g, 1.4 equiv.) was addded and stirring was continued for 3 hours. The reaction mixture was poured into buffer (700 mL) and extracted with EtOAc. The organic layer was dried over $Na_2SO_4$ and evaporated. Flash chromatography of the residue on silica using EtOAc:toluene 2.5:97.5 afforded the title compound.
$^1$H NMR ($CDCl_3$) δ (ppm): 3.37 (s, 3H), 3.48 (s, 3H), 3.93 (s, 3H), 7.29 (d, 1H), 7.48 (dd, 1H), 7.75 (broad s, 1H), 7.95 (d, 1H).

Step 6 Preparation of S-(3-methoxycarbonyl)phenyl)dimethylcarbamothioate

The product of step 5 (8.452 g, 35.3 mmoles) was heated to reflux for 5 days in dichlorobenzene (50 mL). Flash chromatography of the reaction mixture on silica using EtOAc:toluene 5:95 and 10:90 yielded the title product.
$^1$H NMR ($CDCl_3$) δ (ppm): 3.05 (broad s, 3H), 3.12 (broad s, 3H), 3.91 (s, 3H), 7.48 (dd, 1H), 7.70 (d, 1H). 8.08 (d, 1H), 8.18 (broad s, 1H).

Step 7 Preparation of methyl-3-mercaptobenzoate

To the product of step 6 (6.767 g, 28.3 mmoles) in MeOH (23 mL), MeONa (1.74 M in MeOH, 33 mL, 2 equiv.) was added and the solution was stirred at room temperature 8 hours and kept at 5°C for 3 days. $NH_4OAc$ (600 mL) was added, followed by HCl (1N,60 mL). Extraction with EtOAc, drying of the organic phase over $Na_2SO_4$ and flash chromatography of the residue on silica with EtOAc:hexane:AcOH 4:96:1 yielded the title thiol.
$^1$H NMR ($CDCl_3$) δ (ppm): 3.55 (s, 1H), 3.92 (s, 3H), 7.31 (d, 1H), 7.46 (d, 1H), 7.82 (d, 1H), 7.95 (broad s, 1H).

Step 8 Preparation of methyl 6-(3-cyanophenyl)-6-(3-(methoxycarbonyl)phenylthio)-3-methylhexanoate

The cyanostyrene of step 4 (506 mg, 2.08 mmoles) and the thiol of step 7 (449 mg, 1.3 equiv.) were mixed together in $CH_2Cl_2$ (20 mL). $AlCl_3$ (1.16 g, 4.2 equiv.) was added and the mixture was stirred at room temperature 1.5 hours. At 0°C, 25% aqueous $NH_4OAc$ was added. Extraction with EtOAc and flash chromatography of the residue from the dried organic phase on silica with EtOAc:hexane 15:85 and 20:80

afforded the title compound.
$^1H$ NMR ($CDCl_3$) δ (ppm): 0.93 (2d, 3H), 1.02-1.55 (m, 2H), 1.78-2.36 (m, 5H), 3.66 (2s, 3H), 3.94 (s, 3H), 4.13 (dd, 1H), 7.22-7.53 (m, 6H), 7.88 (broad s, 2H).

Step 9 Preparation of methyl 6-(3-formylphenyl)-6-(3-(methoxycarbonyl)phenylthio)-3-methyl hexanoate

HCl gas was bubbled into a suspension of $SnCl_2$ (2.769 g, 9 equiv.) in ether (16 mL) until obtention of 2 liquid phases. The nitrile of step 8 (670 mg, 1.628 mmoles) in toluene (3 mL) was then added. HCl was bubbled into the mixture for 30 minutes and occasionally during a further 5 hours. At 0° C, water ( 20 mL) was added to the flask and the mixture was stirred at room temperature until obtention of a clear reaction mixture ( 5-10 minutes). The reaction mixture was then poured into EtOAc:25% aqueous $NH_4OAc$ 1:1 ( 500 mL) and the resulting suspension was stirred overnight, filtered through celite and extracted with EtOAc. The organic layer was dried over $Na_2SO_4$ and evaporated. Flash chromatography of the residue on silica using EtOAc:hexane 15:85 and 20:80 yielded the title aldehyde.
$^1H$ NMR ($CDCl_3$) δ (ppm): 0.92 (2d, 3H), 1.04-1.55 (m, 2H), 1.84-2.34 (m, 5H), 3.62 (2s, 3H), 3.90 (s, 3H), 4.21 (t, 1H), 7.2-7.54 (m, 4H), 7.72 (broad s, 2H) 7.84 (d, 1H), 7.89 (s, 1H).

Step 10 Preparation of methyl 6-(3-(2-(7-chloroquinolin-2-yl)ethenyl)phenyl)-6-(3-(methoxycarbonyl)-phenylthio)-3-methylhexanoate

At -78° C, BuLi (1.6 M in hexanes, 1. mL, 1.2 equiv.) was added dropwise to a suspension of ((7-chloroquinolin-2-yl)methyl)triphenylphosphonium bromide (EP 233,763. Example 4, step 2) (926 mg, 1.3 equiv.) in THF (9 mL) and the suspension was stirred at this temperature for 1 hour. The benzaldehyde of step 9 (566 mg, 1.364 mmoles) in THF (5 mL) was then added dropwise. The mixture was stirred at -78° C for 15 minutes and at room temperature for 2 hours. It was quenched with 25% aqueous $NH_4OAc$ and extracted with EtOAc. The organic layer was dried over $Na_2SO_4$, filtered through silica and evaporated. Flash chromatography of the residue using EtOAc:hexane 15:85 and 20:80 yielded the title diester.
$^1H$ NMR ($CD_3COCD_3$) δ (ppm): 0.85 (2d, 3H), 1.07-1.56 (m, 2H), 1.83-2.30 (m, 5H), 3.53 (2s, 3H), 3.80 (s, 3H), 4.45 (t, 1H), 7.30-7.58 (m, 7H), 7.67-7.98 (m, 7H), 8.32 (d, 1H).

Step 11

To the diester of step 10 (722 mg, 1.26 mmoles) in THF:MeOH 1:1 (12 mL), LiOH 1.0 N (3.8 mL, 3 equiv.) was added and the mixture was stirred for 28 hours. 25% aqueous $NH_4OAc$ was then added and the mixture was acidified to pH 5 with HCl. Extraction with EtOAc, drying of the organic layer over $Na_2SO_4$ and flash chromatography of the residue on silica using EtOAc:toluene:AcOH 30:70:1 yielded the title diacid.
$^1H$ NMR ($CD_3COCD_3$) δ (ppm): 0.92 (2d, 3H), 1.14-1.65 (m, 2H), 1.88-2.16 (m, 4H), 2.21-2.38 (m, 1H), 4.50 (t, 1H), 7.30-7.62 (m, 7H), 7.70-8.03 (m, 7H), 8.32 (d, 1H).

## Example 9

Preparation of 3-((3-((7-chloroquinolin-2-ylmethyl)oxy)phenyl)(2-(dimethylcarbamoyl)ethylthio)methyl)benzoic acid, sodium salt

Step 1 Preparation of methyl 3-[(3-((7-chloroquinolin-2-ylmethyl)oxy)phenyl)hydroxymethyl] benzoate

To a suspension of 3-bromobenzoic acid (905 mg) in THF (25 cc) at -78° C was added 1.6M BuLi (6.25 cc) and the mixture was stirred for 1/2 hr at -78° C. A solution of 3-((7-chloroquinolin-2-ylmethyl)oxy) benzaldehyde (EP 233,763, Example 16, step 1) (1.49 g) in THF (25 cc) was added dropwise and allowed to react for 1 hr at -78°. 25% aqueous $NH_4OAc$ (25 cc) and acetic acid (3 cc) were added at -78° and the mixture allowed to warm to 25° C, at which temperature it was extracted with ethyl acetate (3x 25 cc). The

organic layer was washed with brine and the solvents were removed in vacuo. The residue was taken up in dry 10% HCl in MeOH for 16 hrs at room temperature. Most of the MeOH was removed in vacuo and the residue partitioned between 25% aqueous $NH_4OAc$ (10 cc) and ethyl acetate (3x 10 cc). The organic layer was washed with brine and the solvents removed in vacuo. The residue was purified by chromatography to afford the title compound.
p.m.r. ($CD_3COCD_3$) $\delta$ (ppm): 8.3-8.4 (d, 1H), 6.9-8.1 (m, 12H), 5.85-5.9 (d, 1H), 5.35 (s, 2H), 5.1 (d, 1H), 3.85 (s, 3H).

Step 2 Preparation of methyl 3-((3-((7-chloroquinolin-2-ylmethyl)oxy)phenyl)(2-(dimethylcarbamoyl)-ethylthio)methyl)benzoate

To a solution of the alcohol (step 1) (70 mg) at 25 °C and 3-mercapto-N,N-dimethylpropionamide (66 mg) in dichloroethane (2 cc) was added $AlCl_3$ (212 mg) and the gummy suspension stirred for 1/2 hr after which time 25% aqueous $NH_4OAc$ (5 cc) was added. Organic materials were extracted with ethyl acetate and the organic layer washed with brine and the solvents removed in vacuo. The residue was purified by chromatography to afford the title compound which was used as such in the next step.

Step 3

To a solution of the ester (step 2) (244 mg) in MeOH (1 cc) and THF (3 cc) was added 2N NaOH (750 µL) and the mixture stirred 3 hrs at 25 °C after which the solvents were removed in vacuo. The residue was partitioned between 25% aqueous $NH_4OAc$ (10 cc) containing AcOH (2 cc) and ethyl acetate (25 cc). The organic layer was washed with brine and the solvent removed to give a residue which was purified by chromatography. To the acid obtained was added 1 equivalent of NaOH and the mixture freeze dried to afford the title compound.
p.m.r. ($CD_3COCD_3$) $\delta$ (ppm): 6.9-8.4 (m, 13H), 5.5 (s, 1H), 5.35 (s, 2H), 2.9 (s, 3H), 2.8 (s, 3H), 2.5-2.7 (m, 4H).

## Example 10

3-((3-carboxyphenylthio)(3-(2-(7-chloroquinolin-2-yl)ethenyl)phenyl)methyl)benzoic acid

Step 1 Preparation of 3-((3-(2-(7-chloroquinolin-2-yl)ethenyl)phenyl)(3-methoxycarbonylphenylthio)methyl)-benzoic acid

To a suspension of the hydroxyacid of Example 5, step 1 (1.032 g, 2.48 mmoles) in $CH_2Cl_2$ (25 mL), methyl 3-mercaptobenzoate (Example 8, step 7, 545 mg, 1.3 equiv.) and 2,6-di-tert-butyl-4 methylphenol (106 mg, 0.2 equiv.) were added. At 0 °C, $AlCl_3$ (1.290 g, 3.9 equiv.) was added and the reaction mixture was stirred at 0 °C 1.3 hours. THF and 25% aqueous $NH_4OAc$ were then added and the mixture was stirred at room temperature for 3 hours. The mixture was acidified to pH 5 with acetic acid and was extracted with EtOAc. The organic layer was dried over $Na_2SO_4$ and evaporated. Flash chromatography of the residue on silica using EtOAc:toluene:AcOH 10:90:1 yielded the title compound (contaminated by a diaddition product), which was used as such in the next step.

Step 2

To the mixture of step 1 (400 mg containing 460 µmoles of monoaddition product and 153 µmoles of diaddition product) in THF:MeOH 1:1 (6 mL), LiOH 1.0N (2 mL) was added and the reaction mixture was stirred for 2 days. 25% aqueous $NH_4OAc$ was then added and the mixture was acidified with AcOH and extracted with EtOAc. Drying the organic phase over $Na_2SO_4$ and flash chromatography of the residue in silica using EtOAc:toluene:AcOH 20:80:1 and 30:70:1 yielded the title compound.

$^1H$ NMR ($CD_3COCD_3$) δ (ppm): 6.17 (s, 1H), 7.32-7.68 (m, 8H), 7.78-8.08 (m, 9H), 8.27 (s, 1H), 8.31 (d, 1H).

## Example 11

### Preparation of 3-((3-((7-chloroquinolin-2-ylmethyl)oxy)phenyl)(2-(t-butylcarbamoyl)ethylthio)methyl)benzoic acid, sodium salt

#### Step 1 Preparation of methyl 3-((3-((7-chloroquinolin-2-ylmethyl)oxy)phenyl)(2-carboxyethylthio)methyl)-benzoate

To a 0° C solution of the alcohol (Example 9, step 1) (475 mg) and 3 mercaptopropionoic acid (160 mg) in dichloroethane (15 cc). $AlCl_3$ (532 mg), was added portion-wise and the suspension was stirred for 1/2 hr at 0° C after which time 25% aqueous $NH_4OAc$ (10 cc) was added. The organic materials were extracted with ethyl acetate (3x 10 cc), the organic layer washed with brine and the solvents removed in vacuo to yield a residue which was purified by chromatography to afford the title compound.
p.m.r. ($CD_3COCD_3$) δ (ppm): 6.9-8.4 (m, 13H), 5.5 (s, 1H), 5.35 (s, 2H), 3.85 (s, 3H), 2.5-2.7 (m, 4H).

#### Step 2 Preparation of methyl 3-((3-((7-chloroquinolin-2-ylmethyl)oxy)phenyl)(2-(t-butylcarbamoyl)ethylthio)-methyl)benzoate

To a 0° solution of the acid (step 1) (522 mg) in dichloromethane (25 cc), acetonitrile (7 cc) and triethylamine (202 mg) was added 2-chloro-1-methylpyridinium iodide (511 mg) and the mixture left to react for 1.5 hrs at 0° C. Then t-butylamine (366 mg) was added and the mixture stirred at 25° C for 16 hrs. 25% aqueous $NH_4OAc$ (25 cc) was added and the mixture extracted with ethyl acetate (3x 25 cc). The combined organic layers were washed with brine and the solvents removed in vacuo. The residue was purified by chromatography to afford the title compound.
p.m.r. ($CD_3COCD_3$) δ (ppm): 6.9-8.4 (m, 13H), 6.75 (bs, 1H), 5.45 (s, 1H), 5.35 (s, 2H), 3.85 (s, 3H), 2.3-2.65 (m, 4H), 1.3 (s, 9H).

#### Step 3

To a solution of the ester (step 2) (327 mg) in MeOH (1 cc) and THF (3 cc) was added 2N NaOH (850 μL) and the reaction stirred for 16 hrs at 25° C. 25% aqueous $NH_4OAc$ (10 cc) and acetic acid (1 cc) were added and the organic material extracted with ethyl acetate (3x 10 cc). The organic layer was washed with brine and the solvents removed in vacuo to give the acid which was treated with one equivalent of NaOH and freeze dried to afford the title compound.
p.m.r. ($CD_3COCD_3$) δ (ppm): 6.8-8.3 (m, 14H), 5.25 (s, 2H), 5.2 (s, 1H), 2.2-2.6 (m, 4H), 1.25 (s, 9H).

## EXAMPLE 12

### Preparation of 5-((3-((7-chloro-2-quinolinyl)methoxy)phenyl)((3-dimethylamino-3-oxopropyl)thio)methyl)-3-pyridinecarboxylic acid, sodium salt

#### Step 1

Preparation of methyl 5-((3-((7-chloro-2-quinolinyl)methoxy)phenyl)-hydroxymethyl)-3-pyridinecarboxylate

To a -100 °C suspension of 3 bromonicotinic acid (1.21 g) in THF (25 c.c.) was added BuLi (1.6 M in hexanes, 12 mmoles); after 45 min there was added a solution of the aldehyde from EP 233,763, Example 16, Step 1 (1.48 g), in THF (25 c.c.) and the mixture was stirred 1.5 hr at -78 °C. It was then poured onto 25% aqueous $NH_4OAc$ and 1 c.c. of conc. AcOH was added; the product was extracted with ethyl acetate, washed with brine, dried with $MgSO_4$ and the solvents removed in vacuo. The residue was purified by chromatography to yield the carboxylic acid which was esterified with 10% HCl in MeOH at r.t. After removal of most of the MeOH, 25% aqueous $NH_4OAc$ was added and the product extracted with ethyl acetate, washed with brine and the solvents removed in vacuo to yield the title compound.
$^1H$ NMR ($CD_3COCD_3$) δ (ppm): 3.90 (s, 3H), 5.30 (s, 2H), 5.45 (d, 1H), 6.00 (bs, 1H), 6.90-9.00 (m, 12H).

Step 2

Preparation of methyl 5-((3-((7-chloro-2-quinolinyl)methoxy)phenyl)-chloromethyl)-3-pyridinecarboxylate

To a r.t. solution of the alcohol (Step 1) (217 mg) in dichloromethane (10 c.c.) and carbon tetrachloride (5 c.c.) was added tri-n-octyl phosphine (650 mg) and the reaction was stirred for 1.5 hr. The reaction products were preabsorbed on $SiO_2$ and the title compound purified by chromatography.
$^1H$ NMR ($CD_3COCD_3$) δ (ppm): 3.90 (s, 3H), 5.40 (s, 2H), 6.55 (s, 1H), 7.05-9.10 (m, 12H).

Step 3

Preparation of methyl 5-((3-((7-chloro-2-quinolinyl)methoxy)phenyl)((3-dimethylamino-3-oxopropyl)thio)-methyl)-3-pyridinecarboxylate

To a solution of the chloride (Step 2) (122 mg) and N,N-dimethyl 3-mercaptopropionamide (45 mg) in acetonitrile (10 c.c.) was added cesium carbonate (440 mg) and the mixture was heated to 80 °C for 3 hrs. The reaction products were pre-absorbed on $SiO_2$ and the title compound purified by chromatography.
$^1H$ NMR ($CD_3COCD_3$) δ (ppm) 2.50-2.70 (m, 4H), 2.80 (s, 3H), 2.90 (s, 3H), 3.90 (s, 3H), 5.40 (s, 2H), 5.60 (s, 2H), 6.95-9.00 (m, 12H).

Step 4

To a 0 °C solution of the ester (Step 3) (85 mg) in THF (3 c.c.) and MeOH (1 c.c.) was added 2N NaOH (0.25 c.c.) and the mixture was stirred at r.t. for 3 hrs. 25% aqueous $NH_4OAc$ was added followed by conc. AcOH (3 drops) and the mixture was extracted with ethyl acetate; the organic layer was washed with brine, dried with $MgSO_4$ and the solvents removed in vacuo. To the residue in ethanol (1 c.c.) was added 2N NaOH (77 µL) and the solution freeze dried to yield the title compound.
$^1H$ NMR ($CD_3COCD_3$•DMSO) δ (ppm): 2.40-2.70 (m, 4H), 2.80 (s, 3H), 2.90 (s, 3H), 5.40 (s, 2H), 6.90-9.00 (m, 12H).

EXAMPLE 14

3,3′-(((3-((7-Chloro-2-quinolinyl)methoxy)phenyl)methylene)bis(thio))bis(benzoic acid), disodium salt

Step 1

Preparation of dimethyl 3,3′-(((3-((7-chloro-2-quinolinyl)methoxy)phenyl)methylene)bis(thio))bis(benzoate)

At room temperature (r.t.), $BF_3 \bullet Et_2O$ (270 µL, 3.2 equiv.) was slowly added to a mixture of methyl 3-mercaptobenzoate (Example 8, Step 7; 269 mg, 2.2 equiv.) and 3-((7-chloro-2-quinolinyl)methoxy)-benzaldehyde (EP 233,763, Example 16, Step 1; 204 mg) in $CH_2Cl_2$ (3.5 mL). The reaction mixture was stirred 2.5 hours and was quenched at 0° C with 25% aq. $NH_4OAc$. Extraction with EtOAc, drying over $Na_2SO_4$ and flash chromatography of the residue using EtOAc: toluene 5:95 yielded the title compound.
$^1H$ NMR ($CD_3COCD_3$) δ (ppm): 3.87 (s, 6H), 5.33 (s, 2H), 6.05 (s, 1H), 6.99 (d, 1H), 7.10-7.30 (m, 3H), 7.40 (t, 2H), 7.55-7.70 (m, 4H), 7.85 (d, 2H), 7.95-8.05 (m, 4H), 9.37 (d, 1H).

Step 2

Using the procedure of Example 8, Step 11, the diester of Step 1 was hydrolyzed to the diacid. The diacid was dissolved in ethanol and 2 equiv. of NaOH 1.000 N were added. The solvents were removed in vacuo and the residue was taken up in water and freeze dried.

| Anal. calc'd for $C_{31}H_{20}ClNO_5S_2Na_2 \bullet H_2O$: | | | | | | | | | | | | |
|---|---|---|---|---|---|---|---|---|---|---|---|---|
| | C, | 57.28; | H, | 3.41; | N, | 2.15; | S, | 9.86; | Cl, | 5.45; | Na, | 7.07 |
| Found: | C, | 57.18; | H, | 3.31; | N, | 2.00; | S, | 11.01; | Cl, | 5.34; | Na, | 6.35 |

EXAMPLE 23

3-(((4-Carboxyphenyl)thio)(3-(2-(7-chloro-2-quinolinyl)ethenyl)phenyl)methyl)benzoic acid, disodium salt

Step 1

Preparation of methyl 4-mercaptobenzoate

4-Mercaptobenzoic acid (J. Org. Chem., 1962, 27, 2835; 1.72g) and 98% $H_2SO_4$ (0.7 mL) were mixed together in MeOH (55 mL) for 4 days. At 0° C, 25% aq. $NH_4OAc$ (500 mL) was then added and the ester was extracted with EtOAc, dried over $Na_2SO_4$ and the residue was purified by filtration through silica using EtOAc:toluene 10:90.
$^1H$ NMR ($CDCl_3$) δ (ppm): 3.62 (s, 1H), 3.90 (s, 3H), 7.28 (d, 2H), 7.89 (d, 2H).

Step 2

Using the procedure of Example 10, but substituting methyl 3-mercaptobenzoate by methyl 4-mercaptobenzoate from Step 1, the title compound (free diacid) was obtained. It was then converted to the disodium salt using the procedure of Example 14, Step 2

| Anal. calc'd for $C_{32}H_{20}ClNO_4SNa_2 \bullet 2.8H_2O$: | | | | | | | | | | | | |
|---|---|---|---|---|---|---|---|---|---|---|---|---|
| | C, | 59.46; | H, | 3.99; | N, | 2.17; | S, | 4.96; | Cl, | 5.48; | Na, | 7.11 |
| Found: | C, | 59.40; | H, | 4.21; | N, | 2.10; | S, | 5.03; | Cl, | 5.59; | Na, | 6.37 |

EXAMPLE 24

3-((3-(2-(7-Chloro-2-quinolinyl)ethenyl)phenyl)((4-(dimethylaminocarbonyl)phenyl)thio)methyl)benzoic acid, sodium salt

Step 1

Preparation of N,N-dimethyl 4-mercaptobenzamide

To $P_2O_5$ (411 mg, 0.5 equiv.) in DMF (4 mL), 4-mercaptobenzoic acid (J. Org. Chem., 1962, 27, 2835; 883 mg) was added and the mixture was heated at reflux for 10 hours (see Monatsh Chem., 1968, 99, 1799). Water and EtOAc were added and the resulting mixture was stirred until complete decomposition of polyphosphoric acid. The organic layer was separated, dried over $Na_2SO_4$ and concentrated. The benzamide was purified by flash chromatography using EtOAc:toluene:AcOH 15:85:1 and 25:75:1.
$^1H$ NMR ($CDCl_3$) $\delta$ (ppm): 3.00 (br s, 3H), 3.10 (br s, 3H), 3.54 (br s, 1H), 7.22-7.38 (m, 4H).

Step 2

Using the procedure of Example 5, but substituting N,N-dimethyl 3-mercaptopropanamide by the thiol of Step 1, the title compound (free acid) was obtained (the reaction conditions for the alcohol substitution by the thiol were 0 °C 4 hours, then r.t. 30 min). It was then converted to the sodium salt as in Example 14, Step 2 (only one equiv. of NaOH was used).

| Anal. calc'd for $C_{34}H_{26}ClN_2O_3SNa \bullet 1.5\ H_2O$: | | | | | | | | | | | | |
|---|---|---|---|---|---|---|---|---|---|---|---|---|
| | C, | 65.02; | H, | 4.65; | N, | 4.46; | S, | 5.10; | Cl, | 5.64; | Na, | 3.66 |
| Found: | C, | 65.03; | H, | 4.61; | N, | 4.28; | S, | 5.56; | Cl, | 5.93; | Na, | 3.37 |

EXAMPLE 25

3-(2-((2-Carboxyethyl)thio)-2-(3-((7-chloro-2-quinolinyl)methoxy)phenyl)ethyl)benzoic acid

Step 1

Preparation of 1-methoxy-3-vinylbenzene

At -78 °C, BuLi 1.6 N (29.8 mL, 1.3 equiv.) was added dropwise to a suspension of methyltriphenylphosphonium bromide (18.4 g, 1.4 equiv.) in THF (130 mL) and the mixture was stirred at r.t. 30 min. At -78 °C, 3-methoxybenzaldehyde (4.5 mL) was then added and the mixture was stirred at r.t. 2 hours. After quenching with 25% aq. $NH_4OAc$, the title compound was extracted with EtOAc, dried over $Na_2SO_4$ and purified by filtration through silica using EtOAc:hexane 10:90.

Step 2

Preparation of (3-methoxyphenyl)oxirane

At 0 °C, m-chloroperbenzoic acid (82%, 10.6 g, 1.4 equiv.) was added to the product of Step 1 in $CH_2Cl_2$ (185 mL). The mixture was stirred at 0 °C for 30 min. and at r.t. for 2 hours. 10% Aq. $Na_2CO_3$ was then added, the phases were separated and the aqueous was layer extracted with ether. The combined

organic phases were dried over $Na_2SO_4$ and concentrated. The title compound was purified by distillation: B.P. 130-136° C/16 mm Hg.

Step 3

Preparation of 1-bromo-3-((diphenyl(2-methyl-2-propyl)silyloxy)methyl)benzene

3-Bromobenzyl alcohol (15.00 g 80.2 mmoles). tert-butylchlorodiphenylsilane (25 mL, 1.2 equiv.), 4-(dimethylamino)pyridine (1.06 g, 0.1 equiv.) and triethylamine (22 mL, 2 equiv.) were mixed together in $CH_2Cl_2$ (200 mL). After 2u hours of stirring, 25% Aq. $NH_4OAc$ was added and the phases were separated. The aqueous layer was extracted with EtOAc and the organic phases were combined, dried over $Na_2SO_4$ and concentrated. The title compound was purified by filtration through silica using ether:hexane 5:95. $^1H$ NMR ($CDCl_3$) $\delta$ (ppm): 1.10 (s, 9H), 4.73 (s, 2H), 7.14-7.30 (m, 2H), 7.32-7.53 (m, 8H), 7.67 (dd, 4H).

Step 4

Preparation of 1-(3-methoxyphenyl)-2-(3-((diphenyl (2-methyl-2-propyl)silyloxy)methyl)phenyl)ethanol

At -78° C, BuLi 1.6 M (47 mL) was added dropwise to the bromide of Step 3 (31.77 g, 75 mmoles) in THF (100 mL). The mixture was stirred at -20° C for 10 min. and was added, via a cannula, to a cooled (-20° C) suspension of CuCN (3.68 g) in THF (25 mL); a solution was obtained. (3-Methoxyphenyl)oxirane from Step 2 (1.8 g, 12 mmoles) was dissolved in THF (20 mL) and cooled to -78° C. It was then added. via a cannula, to the cooled (-20° C) solution of cuprate. The mixture was stirred at -20° C for 1 hour and at r.t. for 3 hours. Saturated $NH_4Cl$ containing 10% $NH_4OH$ was added and stirring was continued for 30 min. Water was then added and the desired compound was extracted with EtOAc, dried over $Na_2SO_4$ and purified by flash chromatography on silica using EtOAc:hexane 10:90, 15:85.and 25:75. (See also J. Am. Chem. Soc., 1982, 104, 2305 for the cuprate addition to epoxides.)
$^1H$ NMR ($CDCl_3$) $\delta$ (ppm): 1.10.(s, 9H), 1.94 (d, 1H, OH), 2.94 (dd, 1H), 3.04 (dd, 1H), 3.80 (s, 3H), 4.76 (s, 2H), 4.87 (m, 1H), 6.82 (br d, 1H), 6.90-6.98 (m, 2H), 7.12 (m, 1H), 7.17 (s, 1H), 7.25-7.30 (m, 3H), 7.33-7.47 (m, 6H), 7.70 (dd, 4H).

Step 5

Preparation of 1-chloro-1-(3-methoxyphenyl)-2-(3-((diphenyl(2-methyl-2-propyl)silyloxy)methyl)phenyl)ethane

To the benzyl alcohol of Step 4 (1.116 g, 2.25 mmoles) in $CH_2Cl_2$:$CCl_4$ 1:2 (10 mL) cooled to 0° C, trioctylphosphine (2.508 g, 3 equiv.) was added and the mixture was stirred at r.t. for 4 hours. Silica gel was added and the mixture was stirred 5 min. It was then poured onto a flash chromatography column and eluted with EtOAc:hexane 5:95. The title compound thus obtained was used as such for the next step.

Step 6

Preparation of methyl 3-((1-(3-methoxyphenyl)-2-(3-((diphenyl(2-methyl-2-propyl)silyloxy)methyl)phenyl) ethyl)thio)propanoate

The chloride of Step 5 was dissolved in $CH_3CN$ (15 mL). $CsCO_3$ (2.221 g) and methyl 3-mercaptopropanoate (340 µL) were added and the resulting mixture was stirred at 65° C for 2 hours and at reflux for 2 hours. EtOAc was added and the suspension was filtered through celite. Flash chromatography of the residue (after evaporation) on silica using EtOAc:hexane 7.5:92.5 and 10:90 yielded 1-(3-methoxyphenyl)-2-(3-((diphenyl(2-methyl-2-propyl)silyloxy)methyl)phenyl)ethene (the elimination product), followed by the title

compound.
$^1H$ NMR ($CDCl_3$) δ (ppm): 1.10 (s, 9H), 2.41 (t, 2H), 2.56 (t, 2H), 3.11 (d, 2H), 3.63 (s, 3H), 3.77 (s, 3H), 4.01 (t, 1H), 4.70 (s, 2H), 6.74 (dd, 1H), 6.83 (m, 2H), 6.94 (m, 1H), 7.04 (br s, 1H), 7.13-7.21 (m, 3H), 7.34-7.47 (m, 6H), 7.68 (d, 4H).

Step 7

Preparation of methyl 3-((1-(3-methoxyphenyl)-2-(3-(hydroxymethyl)phenyl)ethyl)thio)propanoate

The silyl ether of Step 6 (469 mg, 783 µmoles), $Bu_4NF$ (1.0 M in THF, 1.6 mL) and acetic acid (160 µL) were mixed together in THF (4 mL) for 8 hours. 25% Aq. $NH_4OAc$ was added and the title compound was extracted with EtOAc, dried over $Na_2SO_4$ and purified by flash chromatography on silica using EtOAc:toluene 20:80.
$^1H$ NMR ($CDCl_3$) δ (ppm): 1.83 (br s, 1H, OH), 2.43 (t, 2H), 2.53 (t, 2H), 3.12 (d, 2H), 3.65 (s, 3H), 3.80 (s, 3H), 4.02 (t, 1H), 4.62 (s, 2H), 6.78 (d, 1H), 6.80-6.90 (m, 2H), 7.00 (d, 1H), 7.08 (s, 1H), 7.12-7.25 (m, 3H).

Step 8

Preparation of methyl 3-(2-((2-(methoxycarbonyl)ethyl)thio)-2-(3-methoxyphenyl)ethyl)benzoate

Using the procedure of Example 1, Steps 3 and 4, the benzyl alcohol of Step 7 was oxidized to the title ester.
$^1H$ NMR ($CDCl_3$) δ (ppm): 2.43 (t, 2H), 2.56 (t, 2H), 3.14 (m, 2H), 3.63 (s, 3H), 3.79 (s, 3H), 3.90 (s, 3H), 4.03 (t, 1H), 6.78 (d, 1H), 6.80-6.88 (m, 2H), 7.17-7.30 (m, 3H), 7.80 (s, 1H), 7.85 (d, 1H).

Step 9

Preparation of methyl 3-(2-(3-hydroxyphenyl)-2-((2-(methoxycarbonyl)ethyl)thio)ethyl)benzoate

At -78 °C, $BBr_3$ (1.0 M in $CH_2Cl_2$, 2 mL, 4 equiv.) was added dropwise to a solution of the methyl ether of Step 8 (193 mg, 497 µmoles) and $Et_3N$ (14 µL, 0.2 equiv.) in $CH_2Cl_2$ (2 mL). The reaction mixture was stirred 1.5 hours at -20 °C and quenched with 25% aq. $NH_4OAc$.. Extraction with EtOAc, drying over $Na_2SO_4$ and flash chromatography of the residue on silica using EtOAc:toluene 7.5:92.5 and 12.5:87.5 yielded the title phenol.
$^1H$ NMR ($CDCl_3$) δ (ppm): 2.43 (t, 2H), 2.56 (t, 2H), 3.14 (m, 2H), 3.65 (s, 3H), 3.91 (s, 3H), 4.00 (t, 1H), 5.50 (br s, 1H, OH), 6.72 (d, 1H), 6.75-6.83 (m, 2H), 7.10-7.30 (m, 3H), 7.80 (s, 1H), 7.85 (d, 1H).

Step 10

Preparation of methyl 3-(2-(3-((7-chloro-2-quinolinyl)methoxy)phenyl)-2-((2-(methoxycarbonyl)ethyl) thio)-ethyl)benzoate

The phenol of Step 9 (142 mg, 379 µmoles), 2-(bromomethyl)-7-chloroquinoline (143 mg, 1.5 equiv.) and $K_2CO_3$ (milled, 105 mg, 2 equiv.) were mixed together in acetone (4 mL) and heated at reflux 8 hours. EtOAc was then added and the mixture was filtered through a small pad of silica gel. Flash chromatography of the residue using EtOAc:toluene 5:95 and 7.5:92.5 afforded the title compound.
$^1H$ NMR ($CD_3COCD_3$) δ (ppm): 2.42 (m, 2H), 2.53 (m, 2H), 3.20 (m, 2H), 3.58 (s, 3H), 3.85 (s, 3H), 4.27 (t, 1H), 5.37 (s, 2H), 6.90-7.00 (m, 2H), 7.13 (br s, 1H), 7.21 (t, 1H), 7.27-7.37 (m, 2H), 7.60 (dd, 1H), 7.70 (d, 1H), 7.74-7.80 (m, 2H), 8.02 (d, 1H), 8.05 (br s, 1H), 8.40 (d, 1H).

Step 11

Using the procedure of Example 8, Step 11, the diester of Step 10 was hydrolyzed to the title diacid. $^1$H NMR ($CD_3COCD_3$) δ (ppm): 2.42 (m 2H), 2.55 (m 2H), 3.22 (m, 2H), 4.30 (t, 1H), 5.37 (s, 2H). 6.92 (dd, 1H), 6.98 (d, 1H), 7.15 7.35 (m, 4H). 7.60 (dd, 1H). 7.70 (d, 1H), 7.80 (m. 2H), 8.01 (d, 1H), 8.05 (br s, 1H), 8.40 (d, 1H).

EXAMPLE 26

3-(1-(3-(2-(7-chloro-2-quinolinyl)ethenyl)phenyl)-1-((2-carboxy-4-pyridinyl)thio)methyl)benzoic acid

Step 1

Preparation of methyl 3-((3-(2-(7-chloro-2-quinolinyl)ethenyl)phenyl)hydroxymethyl)benzoate

At 0°C, AcCl (10 mL) was added to MeOH (80 mL) and the solution was stirred ≈ 30 min. at r.t. The hydroxyacid of Example 5, Step 1 (1.96 g, 4.7 mmoles) was then added and the mixture was stirred at r.t. 4 days. It was then poured into 25% aq. $NH_4OAc$ (400 mL), THF (100 mL) and EtOAc (150 mL). The phases were separated and the aqueous layer was extracted twice with EtOAc:THF 2:1. The combined extracts were dried over $Na_2SO_4$. Flash chromatography of the residue using EtOAc:toluene 10:90 and 20:80 yielded the title ester.

Step 2

Preparation of methyl 3-((3-(2-(7-chloro-2-quinolinyl)ethenyl)phenyl)chloromethyl)benzoate

To the hydroxyester of Step 1 (307 mg, 714 μmoles) in $CH_2Cl_2$:$CCl_4$ 3:2 (5 mL) was added dropwise trioctylphosphine (815 mg, 3 equiv.) and the resulting solution was stirred at r.t. 5 hours. 25% Aq. $NH_4OAc$ was then added and the title compound was extracted with EtOAc, dried over $Na_2SO_4$ and purified by flash chromatography on silica using EtOAc:toluene 2.5:97.5.
$^1$H NMR ($CD_3COCD_3$) δ (ppm): 3.88 (s, 3H), 6.58 (s, 1H), 7.43-7.60 (m, 5H) 7.73 (d, 1H), 7.80-8.01 (m 7H)-.8.20 (s, 1H), 8.33 (d, 1H).

Step 3

Preparation of methyl 3-(1-(3-(2-(7-chloro-2-quinolinyl)ethenyl)phenyl)-1-((2-carboxy-4-pyridinyl)thio) methyl)-benzoate

A mixture containing the chloride of Step 2 (166 mg, 370 μmoles), $Cs_2CO_3$ (61 mg, 5 equiv.), 2-carboxy-4-mercaptopyridine (84 mg, 2 equiv.) and 2,6-di-tert-butyl-4-methylphenol (53 mg, 0.5 equiv.) in $CH_3CN$ (4 mL) is stirred in the dark 20 hours. 25% Aq. $NH_4OAc$ was added and the product was extracted with EtOAc after acidification to pH 2, and purified by flash chromatography.

Step 4

Using the procedure of Example 8, Step 11, the ester of Step 3 is hydrolyzed to the title acid.

## EXAMPLE 27

3-(1-((2-carboxyphenyl)thio)-1-(3-(2-(7-chloro-2-quinolinyl)ethenyl)phenyl)methyl)benzoic acid, disodium salt

### Step 1

Preparation of methyl 2-mercaptobenzoate

A solution of HCl/MeOH was formed by the reaction of AcCl (35 mL) with MeOH (200 mL) at 0 °C. Thiosalicylic acid (9.91 g, 64.3 mmoles) was added to this solution and the mixture was stirred at r.t. 7 days. It was then poured into 25% aq. $NH_4OAc$ (2.5 l). Extraction with EtOAc drying over $Na_2SO_4$ and flash chromatography of the residue on silica using EtOAc:hexane 2.5:97.5 and 5:95 yielded the title ester.
$^1H$ NMR ($CDCl_3$) δ (ppm): 3.93 (s, 3H), 4.70 (s, 1H), 7.17 (m, 1H), 7.32 (m, 2H), 8.02 (d, 1H).

### Step 2

Using the procedure of Example 10, but substituting methyl 3-mercaptobenzoate for methyl 2-mercaptobenzoate from Step 1 and doing the hydrolysis Step 2 with NaOH at 50 °C 8 hours instead of with LiOH at r.t. 2 days, the title acid was obtained. The disodium salt was then prepared using the procedure of Example 14, Step 2.

| Anal. calc'd for $C_{32}H_{20}ClNO_4SNa_2 \bullet 3H_2O$: | | | | | | | | | | | | |
|---|---|---|---|---|---|---|---|---|---|---|---|---|
| | C, | 59.13; | H, | 4.03; | N, | 2.15; | S, | 4.93; | Cl, | 5.45; | Na, | 7.07 |
| Found: | C, | 59.11; | H, | 3.97; | N, | 2.21; | S, | 5.18; | Cl, | 5.54; | Na, | 6.15 |

## EXAMPLE 28

2-(3-(3-((7-chloro-2-quinolinyl)methoxy)phenyl)-3-((2 carboxyethyl)thio)propyl)benzoic acid

### Step 1

Preparation of methyl 2-(3-(3-methoxyphenyl)-2-propenyl)benzoate

Using the procedure of Example 1, Steps 1-4, with the modifications below, the title compound (cis:trans mixture) was prepared. In Step 1, 3-methoxybenzyl chloride was used in place of 3-(bromomethyl)benzonitrile and the phosphonium salt was formed at reflux of $CH_3CN$ for 19 hours.
$^1H$ NMR ($CDCl_3$) δ (ppm): 3.80-4.10 (m, 8H), 5.80, 6.40 and 6.55 (m, 2H, cis:trans mixture), 6.70-6.97 (m, 3H), 7.15-7.50 (m, 4H), 7.90 (d, 1H).

### Step 2

Preparation of methyl 2-(3-((2-(methoxycarbonyl)ethyl)thio)-3-(3-methoxyphenyl)propyl)benzoate

Using the procedure of Example 1, Step 5, but doing the reaction at -10 °C for 20 minutes, the

compound of Step 1 was converted to the title product.
$^1$H NMR ($CDCl_3$) $\delta$ (ppm): 2.12 (m, 2H), 2.46 (m, 2H), 2.59 (m, 2H), 2.88 (m, 1H), 3.04 (m, 1H), 3.67 (s, 3H), 3.84 (m, 7H), 6.80 (d, 1H), 6.90 (m, 2H), 7.17-7.28 (m, 3H), 7.40 (t, 1H), 7.87 (d, 1H).

Step 3

Using the procedure of Example 25, Steps 9-11, the preceding compound was converted to the title diacid.
$^1$H NMR ($CD_3COCD_3$) $\delta$ (ppm): 2.12 (m, 2H), 2.42 (m, 2H), 2.51 (m, 2H), 2.87 (m, 1H), 3.03 (m, 1H), 3.93 (t, 1H), 5.40 (s, 2H), 6.97 (d, 1H), 7.03 (d, 1H), 7.15-7.33 (m, 4H), 7.43 (t, 1H), 7.60 (d, 1H), 7.76 (d, 1H), 7.90 (d, 1H), 8.00 (d, 1H), 8.06 (s, 1H), 8.41 (d, 1H).

## EXAMPLE 29

2-(3-(3-((7-chloro-2-quinolinyl)methoxy)phenyl)-3-((3-dimethylamino-3-oxopropyl)thio)propyl)benzoic acid, sodium salt

Method A

Step 1

Preparation of methyl 2-(3-((3-dimethylamino-3-oxopropyl)thio)-3-(3-methoxyphenyl)propyl)benzoate

At -10°C, $AlCl_3$ (879 mg, 5.5 equiv.) was added to a solution containing N,N-dimethyl 3-mercaptopropanamide (191 mg, 1.2 equiv.), the styrene of Example 28, Step 1 (333 mg, 1.18 mmoles) and 2,6-di-tert-butyl-4-methylphenol (53 mg, 0.2 equiv.) in $CH_2Cl_2$ (12 mL). The resulting mixture was stirred in the dark at 0°C for 3 hours and was quenched with 25% aq. $NH_4OAc$. Extraction with EtOAc, drying over $Na_2SO_4$ and flash chromatography of the residue with acetone:toluene 10:90 and 15:85 yielded the title product, which was used as such for the next step.

Step 2

Preparation of methyl 2-(3-(3-((7-chloro-2-quinolinyl)methoxy)phenyl)-3-((3-dimethylamino-3-oxopropyl)thio)-propyl)benzoate

Using the procedures of Example 25, Steps 9 and 10, but avoiding the use of $Et_3N$ in Step 9, the product of Step 1 was converted to the title compound.
$^1$H NMR ($CD_3COCD_3$) $\delta$ (ppm): 2.08 (m, 2H), 2.40 (m, 2H), 2.52 (m, 2H), 2.75 (m, 1H), 2.80 (s, 3H), 2.90 (s, 3H), 2.94 (m, 1H), 3.80 (s, 3H), 3.93 (t, 1H), 5.48 (s, 2H), 6.98 (d, 1H), 7.02 (d, 1H), 7.15-7.33 (m, 4H), 7.43 (t, 1H), 7.60 (dd, 1H), 7.80 (m, 2H), 8.03 (d, 1H), 8.14 (br s, 1H), 8.50 (d, 1H).

Step 3

The ester of Step 2 (620 mg, 1.07 mmoles) was hydrolyzed with 3.3 N NaOH (3.3 mL, 10 equiv.) in THF:MeOH 1:1 (11 mL) at r.t. for 24 hours. 25% Aqueous $NH_4OAc$ was added and the solution was acidified with AcOH. Extraction with EtOAc, drying over $Na_2SO_4$ and flash chromatography of the residue on silica with acetone:toluene:AcOH 10:90:1 and 15:85:1 yielded the title acid. It was then converted to the sodium salt as in Example 14, Step 2, but only one equiv. of NaOH was used.

| Anal. calc'd for $C_{31}H_{30}ClN_2O_4SNa_2 \bullet 0.5H_2O$: | | | | | | | | | | | |
|---|---|---|---|---|---|---|---|---|---|---|---|
| | C, | 62.67; | H, | 5.26; | N, | 4.72; | S, | 5.40; | Cl, | 5.97; | Na, | 3.87 |
| Found: | C, | 62.86; | H, | 5.21; | N, | 4.53; | S, | 5.12; | Cl, | 5.87; | Na, | 3.62 |

Method B

Step 1

Preparation of 3-((7-chloro-2-quinolinyl)methoxy)benzenemethanol

3-((7 Chloro-2-quinolinyl)methoxy)benzaldehyde (EP 233,763, Example 16, Step 1; 9.60 g, 32.24 mmoles) was dissolved in EtOH:THF 3:1 (215 mL). At 0° C, $NaBH_4$ (1.21 g, 1 equiv.) was added and the mixture was stirred at r.t. 20 min. It was then poured into 25% aq. $NH_4OAc$ (500 mL) and EtOAc. Extractions with EtOAc, drying over $Na_2SO_4$ and filtration through silica yielded the benzyl alcohol, which was used as such in the next step.

Step 2

Preparation of 2-((3-(bromomethyl)phenoxy)methyl)-7-chloroquinoline

To the product of Step 1 and $CBr_4$ (13.90 g, 1.3 equiv.) in $CH_2Cl_2$ (160 mL) at 0° C, a solution of 1,2-bis(diphenylphosphino)ethane (DIPHOS, 8.37 g, 0.65 equiv.) in $CH_2Cl_2$ (75 mL) was added and the resulting mixture was stirred at 0° C for 45 min. and at r.t. for 30 min. Ether was then added and the mixture was filtered through a pad of silica and the silica was washed with EtOAc:toluene 20:80 to yield the pure benzylic bromide.

$^1H$ NMR ($CD_3COCD_3$) δ (ppm): 4.62 (s, 2H), 5.40 (s, 2H), 7.04 (d, 1H) 7.08 (d. 1H), 7.21 (s, 1H), 7.3 (t, 1H), 7.60 (dd, 1H), 7.76 (d, 1H), 8.02 (d, 1H), 8.05 (s, 1H), 8.42 (d, 1H).

Step 3

Formation of ((3-((7-chloro-2-quinolinyl)methoxy)phenyl)methyl)triphenyl-phosphonium bromide

The bromide of Step 2 (7.20 g, 22.3 mmoles) and triphenylphosphine (8.82 g, 1.5 equiv.) were heated at reflux in $CH_3CN$ (75 mL) for 8 hours. At r.t. ether was added and an oil separated, which crystallized on trituration. The solid was filtered and was swished with ether for 20 hours to yield the phosphonium salt.

$^1H$ NMR ($CD_3COCD_3/CD_3SOCD_3$) δ (ppm): 5.15 (s, 2H), 5.23 (d, 2H), 6.69 (d, 1H), 6.77 (s, l1H), 7.02 (d, 1H), 7.19 (t, 1H), 7.56 (d, 1H), 7.62-7.80 (m, 13H), 7.85-7.95 (m, 3H), 8.04 (s, 1H), 8.08 (d, 1H), 8.48 (d, 1H).

Step 4

Formation of 2-(3-(3-((7-chloro-2-quinolinyl)methoxy)phenyl-2-propenyl)benzenemethanol

At -10° C, potassium hexamethyldisilazide 0.65 M in toluene (21 mL, 1.8 equiv.) was added dropwise to a suspension of the phosphonium salt of Step 3 (8.457 g, 13.53 mmoles, 1.8 equiv.) in THF (70 mL) and the mixture was stirred at 0° C for 30 min. At -78° C, 1H-3-hydroxy-3,4-dihydrobenzo(c)pyran (1.141 g, 7.60 mmoles) in THF (14 mL) was added slowly. The mixture was allowed to warm to r.t. and was stirred for a further 3 hours. 25% Aqueous $NH_4OAc$ was added and the products were extracted with EtOAc, dried over

$Na_2SO_4$ and purified by flash chromatography on silica using EtOAc:toluene 10:90 and 15:85. The title compound was obtained as a cis:trans mixture and was used as such for the next step.
$^1$H NMR ($CD_3COCD_3$) δ (ppm): 3.60 (d, 2H), 4.55 and 4.72 (s, 2H), 5.37 (s, 2H), 5.75 and 6.35-6.57 (m 2H), 6.91 (d, 1H), 6.99 (d, 1H), 7.12-7.30 (m 5H), 7.43 (m, 1H) 7.60 (d, 1H), 7.73 (d, 1H) 8.00 (m, 2H), 8.40 (d, 1H).

Step 5

Preparation of 2-(3-(3-((7-chloro-2-quinolinyl)methoxy)phenyl)-2-propenyl)benzaldehyde

To a solution of the benzylic alcohol of Step 4 (2.899 g, 6.20 mmoles) in EtOAc (120 mL) was added portionwise activated $MnO_2$ (10.15 g, approx. 18 equiv.) and the reaction was followed by TLC (EtOAc:toluene 7.5:92.5). When the reaction was completed (approx. 2 hours), the mixture was filtered through silica, concentrated, and the title product was purified by flash chromatography on silica using EtOAc:toluene 2.5:97.5.
$^1$H NMR ($CD_3COCD_3$) δ (ppm): 4.00 (d, 2H), 5.35 (s, 2H), 5.72 and 6.30-6.60 (m, 2H, cis:trans mixture), 6.90-8.10 (m, 12H), 8.39 (d, 1H), 10.33 (s, 1H).

Step 6

Preparation of methyl 2-(3-(3-((7-chloro-2-quinolinyl)methoxy)phenyl)-2-propenyl)benzoate

Using the procedures of Example 1, Step 4, but dissolving the aldehyde of Step 5 in hot THF before doing the reaction, the title compound was obtained.
$^1$H NMR ($CD_3COCD_3$) δ (ppm): 3.70 and 3.82-3.95 (m, 5H, cis:trans mixture), 5.38 (2s, 2H), 5.70 and 6.47 (2m, 2H), 6.87-8.05 (m, 12H), 8.38 (2d, 1H).

Step 7

Using the procedure of Example 29, Method A, Steps 1 and 3, the product of Step 6 was converted to the title compound.

## EXAMPLE 30

2,2′-(((3-((7-chloro-2-quinolinyl)methoxy)phenyl)methylene)bis(thio))bis(benzoic acid), disodium salt

Step 1

Preparation of dimethyl 2,2′-(((3-((7-chloro-2-quinolinyl)methoxy)phenyl)methylene)bis(thio))bis(benzoate)

Using the procedure of Example 14, Step 1 but using methyl 2-mercaptobenzoate (Example 27, Step 1) instead of methyl 3-mercaptobenzoate, the title diester was obtained. It was used as such in the next step.

Step 2

The diester of Step 1 (603 mg, 980u μmoles) was stirred in MeOH:THF:$H_2O$ 7:5:3 (15 mL) with NaOH (1 mL of 10 N) at 50 °C for 5 hours and at r.t. 20 hours. The work up of the reaction and the formation of

the title compound was the same as Example 14, Step 2.

| Anal. calc'd for $C_{31}H_{20}ClNO_5S_2Na_2 \bullet 0.7H_2O$: | | | | | | | | | | | | |
|---|---|---|---|---|---|---|---|---|---|---|---|---|
| | C, | 57.75; | H, | 3.35; | N, | 2.17; | S, | 9.95; | Cl, | 5.50; | Na, | 7.13 |
| Found: | C, | 57.55; | H, | 3.39; | N, | 2.04; | S, | 9.78; | Cl, | 5.67; | Na, | 6.67 |

## EXAMPLE 31

2-chloro-5-((3-((7-chloro-2-quinolinyl)methoxy)phenyl)((3-dimethylamino-3-oxopropyl)thio)methyl)benzoic acid, sodium salt

### Step 1

Preparation of 2-chloro-5-((3-((7-chloro-2-quinolinyl)methoxy)phenyl)hydroxymethyl)benzoic acid

To a suspension of 5-bromo-2-chlorobenzoic acid (1.004 g, 4.26 mmoles) in THF (20 mL) at -100° C n-BuLi (5.3 mL of 1.6 M, 2 equiv.) was added dropwise and the mixture was stirred at -78° C for 1.2 hours. At -100° C, a solution of 3-((7-chloro-2-quinolinyl)methoxy)benzaldehyde (EP 233,763, Example 16, Step 1: 1.253 g, 4.21 mmoles) in THF (15 mL) was added dropwise and the mixture was stirred 2 hours at -78° C. AcOH (2 mL) was then added, followed by 25% aq. $NH_4OAc$. The product was extracted with EtOAc:THF 1:1, dried over $Na_2SO_4$ and purified by flash chromatography on silica with EtOAc:toluene:AcOH 30:70:1. $^1H$ NMR ($CD_3COCD_3$) $\delta$ (ppm): 5.34 (s, 2H), 5.87 (s, 1H), 6.95 (dd, 1H), 7.05 (d, 1H), 7.18 (s, 1H), 7.26 (t, 1H), 7.38 (d, 1H), 7.52 (dd, 1H), 7.62 (dd, 1H), 7.72 (d, 1H), 7.94 (d, 1H), 8.01 (d, 1H), 8.03 (s, 1H), 8.38 (d, 1H).

### Step 2

To a suspension of the alcohol of Step 1 (279 mg, 614 μmoles) in $CH_2Cl_2$ (6 mL) at 0° C, N,N-dimethyl 3-mercaptopropanamide (97 mg, 1.15 equiv.) was added, followed by $AlCl_3$ (580 mg, 7 equiv.). The mixture was stirred 2 hours at 0° C and was quenched with 25% ag. $NH_4OAc$, THF and AcOH. The product was extracted with EtOAc:THF 1:1, dried over $Na_2SO_4$ and purified by flash chromatography on silica using acetone:toluene:AcOH 10:90:1, 20:80:1 and 30:70:1 sequentially. The sodium salt was formed as in Example 14, Step 2, but only one equiv. of NaOH was used.

| Anal. calc'd for $C_{29}H_{25}Cl_2N_2O_4SNa \bullet 0.5H_2O$: | | | | | | | | | | | | |
|---|---|---|---|---|---|---|---|---|---|---|---|---|
| | C, | 58.01; | H, | 4.36; | N, | 4.67; | S, | 5.34; | Cl, | 11.81; | Na, | 3.83 |
| Found: | C, | 57.90; | H, | 4.62; | N, | 4.40; | S, | 5.56; | Cl, | 11.91; | Na, | 3.68 |

## EXAMPLE 32

2,2′-(((3-((7-chloro-2-quinolinyl)methoxy)phenyl)methylene)bis(thiomethyl))bis(benzoic acid), disodium salt

### Step 1

Preparation of ethyl 2-(mercaptomethyl)benzoate

$H_2S$ was bubbled through a solution of KOH (290 mg, 1.2 equiv.) in MeOH (4 mL) at 0 °C until saturation. A solution of ethyl 2-(chloromethyl)benzoate and ethyl 2-(bromomethyl)benzoate (Tetrahedron, 1966, 22, 2107; 1.006 g of a 1:2 mixture, 4.28 mmoles) in MeOH (3 mL) was then added dropwise at -20 °C. The mixture was warmed to 0 °C for l hour and to r.t. for 2 hours. $H_2S$ was occasionally bubbled into the reaction mixture during all this process. Addition of 25% aq. $NH_4OAc$, extraction with EtOAc, drying over $Na_2SO_4$ and distillation of the residue (80-86 °C/0.15 mm) yielded the title compound, which was used as such for the next step.

Step 2

Using the procedure of Example 14, Step 1, but replacing methyl 3-mercaptobenzoate by the thiol of Step 1, the diester of the title compound was obtained. It was then hydrolyzed using the procedure of Example 30, Step 2 to yield the title compound.

| Anal. calc'd for $C_{33}H_{24}ClNO_5S_2Na_2•0.5H_2O$: | | | | | | | | | | | | |
|---|---|---|---|---|---|---|---|---|---|---|---|---|
| | C, | 59.24; | H, | 3.77; | N, | 2.09; | S, | 9.58; | Cl, | 5.30; | Na, | 6.87 |
| Found: | C, | 59.34; | H, | 3.70; | N, | 2.09; | S, | 10.13; | Cl, | 5.04; | Na, | 6.64. |

EXAMPLE 33

2-((((3-((7-chloro-2-quinolinyl)methoxy)phenyl)((3-dimethylamino-3-oxopropyl)thio)methyl)thio)methyl)benzoic acid

Step 1

Preparation of N,N-dimethyl-3-(((acetylthio)(3-((7-chloro-2-quinolinyl)methoxy)phenyl)methyl)thio)-propanamide

Using the procedure of Example 14, Step 1, but using 1.1 equiv. of thiolacetic acid and 1.1 equiv. of N,N-dimethyl-3-mercaptopropanamide instead of 2.2 equiv. of methyl 3-mercaptobenzoate, and adding $BF_3•Et_2O$ at 0 °C instead of at r.t., the title compound was obtained. It was used as such for the next step.

Step 2

Preparation of ethyl 2-((((3-((7-chloro-2-quinolinyl)methoxy)phenyl)((3-dimethylamino-3-oxopropyl)thio)-methyl)thio)methyl)benzoate

At -78 °C, 1.26 M MeONa (410 µL, 1.1 equiv.) was added dropwise to a solution of the mixed dithioketal of Step 1 (230 mg, 470 µmoles) in THF (5 mL) and the mixture was stirred at -78 °C for 15 min. Then, a solution of ethyl 2-(chloromethyl)benzoate and ethyl 2-(bromomethyl)benzoate (Tetrahedron, 1966, 22, 2107; 222 mg of a 1:2 mixture, 2.13 equiv.) in THF (1 mL) was added. The mixture was stirred at -78 °C for 2.2 hours, then at -20 °C for 0.8 hour and at 0 °C for 0.5 hour. Addition of 25% aq. $NH_4OAc$, extraction with EtOAc, drying over $Na_2SO_4$ and flash chromatography of the residue on silica with EtOAc:toluene 40:60 yielded the title dithioketal.

$^1H$ NMR ($CD_3COCD_3$) δ (ppm): 1.33 (t, 3H), 2.45 (t, 2H), 2.60-2.80 (m, 2H), 2.82 (s, 3H), 2.89 (s, 3H), 4.07 (d, 1H), 4.15 (d, 1H), 4.30 (q, 2H), 4.90 (s, 1H), 5.37 (s, 2H), 6.98 (dd, 1H), 7.03 (d, 1H), 7.16 (m, 1H), 7.20-7.50 (m, 4H), 7.59 (d, 1H), 7.73 (d, 1H), 7.86 (d, 1H), 8.00 (d, 1H), 8.03 (s, 1H), 8.40 (d, 1H).

Step 3

Using the procedures of Example 8, Step 11, the title acid was prepared.
$^1$H NMR ($CD_3COCD_3$) δ (ppm): 2.58 (t, 2H), 2.70 (m, 1H), 2.80-2.95 (m, 1H), 2.88 (s, 3H), 2.94 (s, 3H), 4.11 (AB system, 2H), 4.98 (s, 1H), 5.40 (s, 2H), 6.99 (dd, 1H), 7.06 (d, 1H), 7.15-7.47 (m, 5H), 7.58 (d, 1H), 7.75 (d, 1H), 7.90 (d, 1H), 8.00 (d, 1H), 8.06 (s, 1H), 8.42 (d, 1H).

EXAMPLE 34

3-((3-(2-(7-Chloro-2-quinolinyl)ethenyl)phenyl)((4-(dimethylamino)-4-oxopropyl)thio)methyl)benzoic acid

Step 1

Preparation of methyl 3-((acetylthio)(3-(2-(7-chloro-2-quinolinyl)ethenyl)phenyl)methyl)benzoate

Using the procedure of Example 26, Step 3, but replacing 4-mercaptopyridine by thiolacetic acid (1.2 equiv.) and using 1 equiv. of 2,6-di-tert-butyl-4-methylphenol and 1.3 equiv. of $CsCO_3$, the title thiolester was prepared.
$^1$H NMR ($CD_3COCD_3$) δ (ppm): 2.38 (s, 3H), 3.87 (s, 3H), 6.08 (s, 1H), 7.45-7.55 (m, 5H), 7.64 (d. 1H), 7.70 (d, 1H), 7.75-8.00 (m, 6H), 8.09 (s, 1H), 8.30 (d, 1H).

Step 2

Preparation of methyl 3-((3-(2-(7-chloro-2-quinolinyl)ethenyl)phenyl)((4-(dimethylamino)-4-oxopropyl)thio)-methyl)benzoate

At -78°C, 1.26 M MeONa (970 μL, 4 equiv.) is added to a mixture of the thiolester of Step 1 (147 mg, 301 μmoles) and N,N-dimethyl-4-chloro butanamide (2.9 equiv.) in THF (3 mL). The mixture is stirred at 0°C for 45 min., at r.t. for 15 min and then at 50°C for 3 hours. Addition of 25% aq. $NH_4OAc$, extraction with EtOAc:THF 1:1, drying over $Na_2SO_4$ and flash chromatography of the residue on slica yields the title compound.

Step 3

The ester of Step 2 is hydrolyzed in THF:MeOH:$H_2O$ 2:4:1 (1 mL) with 3 equiv. of 10 N NaOH at r.t. for 23 hours. 25% aq. $NH_4OAc$ is added and the product is extracted with EtOAc, dried over $Na_2SO_4$ and purified by flash chromatography on silica.

EXAMPLE 35

2-(3-(3-((7-chloro-2-quinolinyl)methoxy)phenyl)-3-((3-((2-methyl-2-propyl)amino)-3-oxopropyl)thio)propyl)-benzoic acid, sodium salt

Step 1

Preparation of 3-((1-(3-((7-chloro-2-quinolinyl)methoxy)phenyl)-3-(2-(methylycarbonyl)phenyl)propyl)thio)-propanoic acid

To methyl 2-(3-(3-((7-chloro-2-quinolinyl)methoxy)phenyl)-2-propenyl)benzoate (Example 29, Method B, Step 6), 3-mercaptopropionic acid was added using the procedure of Example 29, Method A, Step 1, to yield the title compound.
$^1$H NMR ($CD_3COCD_3$) δ (ppm): 2.10 (m, 2H), 2.43 (m, 2H), 2.52 (m, 2H), 2.70-3.00 (m, 2H), 3.80 (s, 3H), 3.95 (t, 1H), 5.42 (s, 2H), 7.00 (m, 2H), 7.15-7.33 (m, 4H), 7.43 (t, IH), 7.58 (d, 1H), 7.77 (d, 1H), 7.80 (d, 1H), 7.97 (d, 1H), 8.04 (s, 1H), 8.39 (d, 1H).

Step 2

Preparation of methyl 2-(3-(3-((7-chloro 2-quinolinyl)methoxy)phenyl)-3-((3-((2-methyl-2-propyl)amino)-3-oxopropyl)thio)propyl)benzoate

To a solution of the acid of Step 1 (385 mg, 700 μmoles) in $CH_2Cl_2$:$CH_3CN$ 4:1 (20 mL) at 0° C, $Et_3N$ (290 μL, 2 equiv.) was added, followed by 2-chloro-1-methylpyridinium iodide (535 mg, 2 equiv.) and the reaction mixture was stirred at 0° C for 1.8 hours. Then, tert-butylamine was added and stirring was continued at r.t. 3 hours. Addition of 25% aq. $NH_4OAc$, extraction with EtOAc, drying over $Na_2SO_4$ and flash chromatography of the residue on silica, using EtOAc:toluene 20:80 and 25:75 sequentially yielded the title compound.
$^1$H NMR ($CD_3COCD_3$) δ (ppm): 1.30 (s, 9H), 2.07 (m, 2H), 2.25 (t, 2H), 2.50 (t, 2H), 2.70-3.00 (m, 2H), 3.80 (s, 3H), 3.90 (t, 1H), 5.32 (s, 2H), 6.70 (br,s, 1H, NH), 7.00 (m, 2H), 7.10-7.35 (m, 4H), 7.44 (t, 1H), 7.58 (d, 1H), 7.76 (d, 1H). 7.80 (d, 1H), 7.98 (d, 1H), 8.04 (s, 1H), 8.40 (d, 1H).

Step 3

Using the procedure of Example 29, Method A, Step 3, the ester of Step 2 was converted to the title sodium salt

| Anal. calc'd for $C_{33}H_{34}ClN_2O_4SNa_2 \bullet 0.5H_2O$: | | | | | | | | | | | | |
|---|---|---|---|---|---|---|---|---|---|---|---|---|
| | C, | 63.70; | H, | 5.67; | N, | 4.50; | S, | 5.15; | Cl, | 5.70; | Na, | 3.70 |
| Found: | C, | 63.51; | H, | 5.78; | N, | 4.07; | S, | 5.61; | Cl, | 5.48; | Na, | 3.60 |

EXAMPLE 36

2-(3-(3-(2-(7-chloro-2-quinolinyl)ethyl)phenyl)-3-((3-((2-methyl-2-propyl)amino)-3-oxopropyl)thio)propyl)-benzoic acid

Step 1

Preparation of methyl 2-(3-(3-(2-(7-chloro-2-quinolinyl)ethyl)phenyl)-2propenyl)benzoate

Using the procedure of Example 29, Method B, Steps 1-6, the aldehyde of Example 3, Step 2, was converted to the title product.

$^1$H NMR ($CD_3COCD_3$) δ (ppm): 3.10-3.35 (m, 4H), 3.72 and 3.88 (m, 5H; cis:trans mixture), 5.70 and 6.42 (m, 2H), 7.05-7.60 (m, 10H), 7.80-8.02 (m, 2H), 8.20 (m, 1H).

Step 2

The styrene of Step 1 was converted to the title acid using the procedure of Example 35. $^1$H NMR ($CD_3COCD_3$) δ (ppm): 1.28 (s, 9H), 2.10 (td, 2H), 2.25 (t, 2H), 2.50 (m, 2H), 2.90 (m, 2H), 3.15 (t, 2H), 3.32 (t, 2H), 3.86 (t, 1H), 6.80 (br s, 1H, NH), 7.00 (m, 1H), 7.10-7.35 (m, 5H), 7.40-7.53 (m, 3H), 7.90 (d, 2H), 8.02 (s, 1H), 8.25 (d, 1H).

## EXAMPLE 37

3-(3-(3-(2-(7-chloro-2-quinolinyl)ethyl)phenyl)-3-((3-dimethylamino)-3-oxopropyl)thio)propyl)benzoic acid

Step 1

Preparation of 3-(1,3-dioxolan-2-yl)benzaldehyde

Ethylene glycol (4.5 mL, 1.05 equiv.), isophthalaldehyde (10.201 g, 76 mmoles) and p-toluenesulfonic acid (560 mg) were heated at reflux in benzene (100 mL) for 8.5 hours. Water was continuously removed from the reaction mixture with a Dean-Stark apparatus. The solvent was then evaporated and the title compound was purified by flash chromatography on silica using EtOAc:toluene 2.5:92.5 and 5:95.
$^1$H NMR ($CDCl_3$) δ (ppm): 4.13 (m, 4H), 5.90 (s, 1H), 7.57 (t, 1H), 7.77 (d, 1H), 7.91 (d, 1H), 8.03 (s, 1H), 10.05 (s, 1H) ppm.

Step 2

Preparation of methyl 3-(1,3-dioxolan-2-yl)benzoate

Using the procedure of Example 1, Step 4, the aldehyde of Step 1 was oxidized to the title ester, which was used as such for the next step.

Step 3

Preparation of methyl 3-formylbenzoate

To the ketal of Step 2 (10.046 g, 45.7 mmoles) in THF:MeOH 3:2 (100 mL), 10 N aqueous HCl (80 mL) was added and the mixture was stirred for 1 hour. Addition of 25% aq. $NH_4OAc$, extraction with EtOAc, drying over $MgSO_4$ and flash chromatography of the residue on silica using EtOAc:toluene 2.5:97.5 yielded the title aldehyde.
$^1$H NMR ($CDCl_3$) δ (ppm): 4.00 (s, 3H), 7.67 (t, 1H), 8.10 (d, 1H), 8.32 (d, 1H), 8.54 (s, 1H), 10.08 (s, 1H).

Step 4

Preparation of methyl 3-(2-(trimethylsilyl)oxiranyl)benzoate

Using the procedure described in J. Am. Chem. Soc., 1977, 99, 4536, the product of Step 3 was converted to the title epoxysilane. In order to improve the yield, the anion of chloromethyltrimethylsilane was formed at -78° C for 1.2 hours and the aldehyde, dissolved in THF and cooled to -78° C, was added to this anion at -100° C.
$^1H$ NMR ($CD_2Cl_2$) δ (ppm): -0.17 and 0.15 (2s, 9H, mixture of cis:trans epoxyde), 2.32 and 2.55 (2d, 1H), 3.75 and 4.28 (2d, 1H), 3.90 (s, 3H), 7.37-7.60 (m, 2H), 7.90 - 8.05 (m, 2H).

Step 5

Preparation of methyl 3-(formylmethyl)benzoate

At 0° C, formic acid (6 mL) was added to a solution of the epoxysilane of Step 4 (605 mg, 2.42 mmoles) in THF:$H_2O$ 10:1 (6.6 mL) and the mixture was stirred at r.t. for 4 hours. The solvents were evaporated and the product was purified by flash chromatography on silica using EtOAc:hexane 15:85 and 20:80 sequentially.
$^1H$ NMR ($CDCl_3$) δ (ppm): 3.80 (s, 2H), 3.93 (s, 3H), 7.45 (m, 2H), 7.90 (s, 1H), 8.00 (d, 1H), 9.80 (s, 1H).

Step 6

Preparation of methyl 3-(3-(3-(2-(7-chloro-2 quinolinyl)ethyl)phenyl)-2-propenyl)benzoate

The title compound was obtained using the procedure of Example 29, Method B, Steps 4-6, with the following modifications to Step 4: 1.2 equiv. of phosphonium salt and 1.0 equiv. of KHMDS were used, and the aldehyde used was that of Example 37, Step 5.
$^1H$ NMR ($CDCl_3$) δ (ppm): 3.15 (m, 2H), 3.27 (m, 2H), 3.60 (d, 2H), 3.91 (s, 3H), 5.78 and 6.22-6.63 (2m, 2H, cis:trans mixture), 7.05-7.30 (m, 5H), 7.30-7.50 (m, 3H), 7.70 (2d, 1H), 7.83-8.10 (m, 4H).

Step 7

Using the procedure of Example 29, Method A, Step 1 and Step 3, the product of Step 6 was converted to the title compound.
$^1H$ NMR ($CD_3COCD_3$) δ (ppm): 2.10 (m, 2H), 2.37 (m, 2H), 2.48 (t, 2H), 2.60 (t, 2H), 2.80 (s, 3H), 2.86 (s, 3H), 3.18 (m, 2H), 3.32 (m, 2H), 3.80 (t, 1H), 7.15-7.30 (m, 4H), 7.40 (m, 3H), 7.50 (d, 1H), 7.80-7.92 (m, 3H), 8.04 (s, 1H), 8.22 (d, 1H).

EXAMPLE 38

2-((3-((7-chloro-2-quinolinyl)methoxy)phenyl)((3-dimethylamino-3-oxopropyl)thio)methyl)benzoic acid

Step 1

Preparation of 3-(3-((7-chloro-2-quinolinyl)methoxy)phenyl)-1-(3H)isobenzofuranone

At -100° C, 1.6 M BuLi (2.7 mL, 4.32 mmoles) was added dropwise to a suspension of 2 bromobenzoic acid (421 mg, 2.09 mmoles) in THF (8.5 mL) and the mixture was stirred at -78° C for 2.5 hours. At -100° C, a solution of 3-((7-chloro-2-quinolinyl) methoxy)benzaldehyde (EP 233,763, Example 16, Step 1; 498 mg, 1.67 mmoles) in THF (6 mL) was added and the mixture was stirred at -78° C for 45 min., then at 0° C for 15 min. AcOH was added at 0° C and the reaction mixture was poured into 1 N HCl and stirred overnight.

25% aq. $NH_4OAc$ was then added and the pH was adjusted to 5 by addition of 8 N KOH. Extraction wth EtOAc:THF 1:1, drying over $Na_2SO_4$ and flash chromatography of the residue using EtOAc:toluene 5:95 afforded the title lactone.

$^1$H NMR ($CD_3COCD_3$) $\delta$ (ppm): 5.38 (AB system, 2H), 6.58 (s, 1H), 7.00 (d, 1H), 7.10 (m, 2H), 7.30-7.45 (m, 2H), 7.55-7.75 (m, 4H), 7.88 (d, 1H), 8.02 (m, 2H), 8.38 (d, 1H).

Step 2

Preparation of 2-((3-((7-chloro-2-quinolinyl)methoxy)phenyl)hydroxymethyl)benzoic acid

To the lactone of Step 1 (188 mg, 468 µmoles) in THF:MeOH 1:1 (6 mL), 3.3 N NaOH (2 mL) was added. The mixture was stirred at r.t. for 3 days. A saturated solution of $NH_4Cl$ was added and the product was extracted with EtOAc, dried over $Na_2SO_4$ and concentrated. It was used as such for the next step.

Step 3

Using the procedure of Example 31, Step 2, the hydroxyacid of Step 2 was converted to the title compound.

$^1$H NMR ($CD_3COCD_3$) $\delta$ (ppm): 2.52 (m, 2H), 2.62 (m, 2H), 2.80 (s, 3H), 2.90 (s, 3H), 5.37 (s, 2H) 6.62 (s, 1H), 6.94 (d, 1H), 7.10 (d, 1H), 7.20-7.35 (m, 3H), 7.43 (t, 1H), 7.60 (dd, 1H), 7.68 (m, 2H). 7.89 (d, 1H), 8.00 (d, 1H), 8.05 (s, 1H), 8.38 (d, 1H).

EXAMPLE 39

2-(3-(3-((7-chloro-2-quinolinyl)methoxy)phenyl)-3-((3-oxo-3-(1-(tricyclo[3.3.1.1$^{3,7}$]decyl)amino)propyl)thio)-propyl)benzoic acid, sodium salt

Using the procedure of Example 35, with the modifications below, the title compound was synthesized. The modifications were only in Step 2, where 1) 1-adamantanamine hydrochloride (3 equiv.) was used instead of tert-butylamine, 2) 3 equiv. of $Et_3N$ were added with the adamantanamine and 3) 3 equiv. of each $Et_3N$ and 2-chloro-1-methylpyridinium iodide were used for the formation of the activated ester.

| Anal. calc'd for $C_{39}H_{40}ClN_2O_4SNa_2 \bullet 1.5H_2O$: | | | | | | | | | | |
|---|---|---|---|---|---|---|---|---|---|---|
| | C, | 65.21; | H, | 6.03; | N, | 3.90; | Cl, | 4.94; | Na, | 3.20 |
| Found: | C, | 65.24; | H, | 6.16; | N, | 3.70; | Cl, | 4.49; | Na, | 2.94 |

EXAMPLE 40

N,N-dimethyl 3-((1-(3-((7-chloro-2-quinolinyl)methoxy)phenyl)-3-(2-(1H-tetrazol 5-yl)phenyl)propyl)thio)-procanamide, sodium salt

Step 1

Preparation of 2-(3-(3-((7-chloro-2-quinolinyl)methoxy)phenyl)-2-propenyl)benzonitrile

To the aldehyde (Example 29, Method B, Step 5) (700 mg) were added formic acid (5 mL), sodium formate (360 mg, 1.8 equiv.) and hydroxylamine hydrochloride (230 mg, 1.15 equiv.). The resulting mixture was warmed at 95° C for 1 hr and then allowed to cool to room temperature. The reaction was quenched by the addition of saturated aqueous sodium bicarbonate, extracted with ether and dried with $Na_2SO_4$. The residue was purified by flash chromatography using 15% ethyl acetate in toluene to afford the title product.
$^1H$ NMR ($CD_3COCD_3$) $\delta$ (ppm): 3.75 (m, 2H), 5.38 and 5.43 (2s, 2H), 5.76 and 6.41 to 6.70 (m, 2H, cis:trans mixture), 6.90 to 7.46 (m, 13H).

Step 2

Preparation of N,N dimethyl 3-((1-(3-((7-chloro-2-quinolinyl)methoxy)phenyl)-3-(2-cyanophenyl)propyl)thio)-propanamide

Using the procedure of Example 29, Method A, Step 1, the cyanostyrene of Step 1 was converted to the title compound.
$^1H$ NMR ($CD_3COCD_3$) $\delta$ (ppm): 2.04 (q, 2H), 2.41 (m, 2H), 2.55 (m, 2H), 2.75 (m, 2H), 2.80 and 2.86 (2s, 6H), 3.96 (t, 1H), 5.40 (s, 2H), 6.95 to 8.06 (m, 12H), 8.40 (d, 1H).

Step 3

To the nitrile (Step 2) (300 mg) dissolved in $CH_2Cl_2$ (0.1 mL) was added tributyltin azide (122 mg). The $CH_2Cl_2$ was removed by a flow of nitrogen and the mixture warmed at 120° C. After 8 hrs, additional tributyltin azide was added (100 mg). After warming 4 hrs, the reaction mixture was purified by flash chromatography (20% acetone in toluene with 1% of acetic acid) to give the corresponding tetrazole.

To the tetrazole (278 mg) dissolved in ethanol (10 mL) was added sodium hydroxide 1 M (473 µL, 1 equiv.) and the solution was freeze dried to give the title compound.
$^1H$ NMR ($CD_3COCD_3$) $\delta$ (ppm): 2.15 (m, 2H), 2.41 (m, 2H), 2.53 (m, 2H), 2.83 and 2.91 (2s, 6H), 3.15 (m, 2H), 3.86 (t, 1H), 5.38 (s, 2H), 6.88 to 8.05 (m, 12H), 8.36 (d, 1H).

| Anal. calc'd for $C_{31}H_{30}ClN_6O_2SNa{\bullet}2.5H_2O$: | | | | | | | | | | | | |
|---|---|---|---|---|---|---|---|---|---|---|---|---|
| | C, | 56.92; | H, | 5.39; | Cl, | 5.42; | N, | 12.84; | S, | 4.90; | Na, | 3.51 |
| Found: | C, | 56.80; | H, | 5.43; | Cl, | 5.33; | N, | 12.70;, | S, | 5.42; | Na, | 3.55 |

EXAMPLE 41

N,N-dimethyl 3-((1-(3-((7-chloro-2-quinolinyl)methoxy)phenyl)-3-(2-((1H-tetrazol-5-yl)methyl)phenyl)propyl)-thio)propanamide, sodium salt

Step 1

Preparation of 2-((3-(3-(2-(bromomethyl)phenyl)-1-propenyl)phenoxy)methyl)-7-chloroquinoline

Using the procedure of Example 29, Method B Step 2, the benzyl alcohol of Example 29, Method B, Step 4 was converted to the title compound.
$^1H$ NMR ($CD_3COCD_3$) $\delta$ (ppm): 3.75 (m, 2H), 4.66 (s, 2H), 5.36 (s, 2H), 5.80 and 6.46 to 6.63 (m, 2H, cis:trans mixture) 6.91 to 8.05 (m, 12H), 8.36 (d, 1H).

Step 2

Preparation of 7-chloro-2-((3-(3-(2-(cyanomethyl)phenyl)-1-propenyl)phenoxy)methyl)quinoline

To the benzyl bromide (Step 1) (930 mg) dissolved in ethanol (13.5 mL) and water (2.7 mL) was added NaCN (611 mg, 6.5 equiv.). The resulting suspension was stirred at 60° C for 1 hr and acetone (4 mL) was added. The mixture was warmed at 80° C for 2 hr then cooled to 40° C for 18 hr. The resulting solution was then poured in an ethyl acetate/25% aqueous ammonium acetate mixture, extracted with ethyl acetate and dried. The residue was purified by flash chromatography using 3% ethyl acetate in toluene to give the title compound.
$^1$H NMR ($CD_3COCD_3$) δ (ppm): 3.63 (m, 2H), 3.96 (m, 2H), 5.35 (m, 2H), 5.70 and 6.41 to 6.61 (m, 2H, cis:trans mixture), 6.86 to 8.05 (m, 12H) 8.50 (2d, 1H).

Step 3

Preparation of N,N-dimethyl 3-((1-(3-((7-chloro-2-quinolinyl)methoxy)phenyl)-3-(2-(cyanomethyl)phenyl) propyl)thio)propanamide

Using the procedure of Example 29, Method A, Step 1, the benzyl nitrile of Step 2 was converted to the title compound.
$^1$H NMR ($CD_3COCD_3$) δ (ppm): 2.11 (m, 2H), 2.41 (m, 2H), 2.55 (m, 2H), 2.75 (m, 2H), 2.83 and 2.90 (2s, 6H), 3.83 (s, 2H), 3.96 (t, 1H), 5.41 (s, 2H), 6.96 to 8.06 (m, 12H), 8.41 (d, 1H).

Step 4

Using the procedure of Example 40, Step 3, the benzyl nitrile of Step 3 was converted to the title compcund (except that the formation of the tetrazole was completed within 4 hrs).
$^1$H NMR ($CD_3COCD_3$) δ (ppm): 2.10 (m, 2H), 2.45 (m, 2H), 2.60 (m, 2H), 2.76 (m, 2H), 2.85 and 2 93 (2s, 6H), 3.95 (t, 1H), 4.08 (s, 2H), 6.91 to 8.08 (m, 12H), 8.41 (d, 1H).

| Anal. calc'd for $C_{32}H_{32}ClN_6O_2SNa \bullet 3H_2O$: | | | | | | | | | | | | |
|---|---|---|---|---|---|---|---|---|---|---|---|---|
| | C, | 56.76; | H, | 5.66; | Cl, | 5.24; | N, | 12.41; | S, | 4.73; | Na, | 3.39 |
| Found: | C, | 56.54; | H, | 5.63; | Cl, | 5.74; | N, | 12.14; | S, | 5.45; | Na, | 3.38 |

EXAMPLE 42

2-(3-(3-((7-Chloro-2-quinolinyl)methoxy)phenyl)-3-((3-dimethylamino-3-oxopropyl)thio)propyl)benzeneacetic acid, sodium salt

Step 1

Preparation of methyl 2-(3-(3-((7-chloro 2-quinolinyl)methoxy)phenyl)-3-((3-dimethylamino-3-oxopropyl)thio)-propyl)benzeneacetate

A solution of benzyl nitrile (Example 41, Step 3) (400 mg) dissolved in methanol (6 mL) saturated with HCl gas was stirred at 60° C for 5 hrs. The solvent was removed under reduced pressure and flash chromatography (40% ethyl acetate in toluene) of the resulting residue gave the title compound.

$^1H$ NMR ($CD_3COCD_3$) δ (ppm): 2.10 (m, 2H), 2.41 (m, 2H), 2.55 (m, 2H), 2.66 (m, 2H), 2.83 and 2.91 (2s, 6H), 3.58 (2s, 5H), 3.96 (t, 1H), 5.36 (s, 2H), 6.95 to 8.08 (m, 12H), 8.41 (d, 1H).

Step 2

To the ester (Step 1) (360 mg) dissolved in methanol (20 mL) and water (5 mL) was added $K_2CO_3$ (200 mg). After 2 hr at room temperature NaOH 10 N (500 µL) was added. After a period of 18 hrs the methanol was removed at reduced pressure and the resulting residue was partitioned between ethyl acetate and water (pH 5 with acetic acid). The organic phase was collected, dried and evaporated to give the acid.

The acid (280 mg) was dissolved in ethanol and treated with NaOH IM (484 µL, 1 equiv.). The solution was then freeze dried to give the title compound.

$^1H$ NMR ($CD_3COCD_3$) δ (ppm): 2.21 (m, 2H), 2.36 (m, 2H), 2.50 (m, 2H), 2.61 (m, 2H), 2.83 and 2.90 (2s, 6H), 3.45 (s, 2H), 2.93 (t, 1H), 5.43 (s, 2H), 6.91 to 8.16 (m, 12H), 8.38 (d, 1H).

| Anal. calc'd for $C_{32}H_{32}ClN_2O_4SNa \bullet 3H_2O$: | | | | | | | | | | |
|---|---|---|---|---|---|---|---|---|---|---|
| | C, | 60.00; | H, | 5.76; | Cl, | 5.53; | N, | 4.37; | S, | 5.00 |
| Found: | C, | 60.02; | H, | 5.83; | Cl, | 5.92; | N, | 4.17; | S, | 5.05 |

## EXAMPLE 43

3-((1-(3-((7-Chloro-2-quinolinyl)methoxy)phenyl)-3-(2-(dimethylaminocarbonyl)phenyl)propyl)thio)propanoic acid, sodium salt

Step 1

Preparation of N,N-dimethyl 2-(3-(3-((7-chloro-2-quinolinyl)methoxy)phenyl)-2propenyl)benzamide

To the ester (Example 29, Method B, Step 6) (500 mg) dissolved in $CH_2Cl_2$ (5 mL) was added a solution of dimethylaluminum dimethylamide (0.8 M, 7 mL, 5 equiv.) in toluene. The solution was stirred in a sealed tube at 60 °C. After 36 hrs the reaction mixture was poured in ethyl acetate (50 mL), and HCl (10%, 20 mL) was then added at 0 °C. After extraction with ethyl acetate, drying ($Na_2SO_4$) and evaporation, the resulting mixture was purified by flash chromatography with 25% ethyl acetate in toluene to give the title compound.

$^1H$ NMR ($CD_3COCD_3$) δ (ppm): 2.75 and 2.86 (4s, 6H), 3.38 to 3.66 (m, 2H), 5.38 (2d, 2H), 5.78 and 6.30 to 6.55 (m, 2H, cis:trans mixture), 6.86 to 8.08 (m, 12H), 8.40 (d, 1H).

Step 2

Using the procedure of Example 29, Method A, Step 1, but using methyl 3 mercaptopropanoate instead of N,N-dimethyl 3-mercaptopropanamide, the styrene amide of Step 1 was converted the thioether.

The ester was hydrolyzed using the procedure of Example 42, Step 2 to give the title compound.

1H NMR ($CD_3COCD_3$) δ (ppm): 2.10 (m, 2H), 2.20 (m, 2H), 2.36 (m, 2H), 2.45 (m, 2H), 2.50 and 2.86 (2s, 6H), 3.76 (t, 1H), 5.33 (s, 2H), 6.83 to 7.83 (m, 12H), 8.25 (d, 1H).

| Anal. calc'd for $C_{31}H_{30}ClN_2O_4SNa \bullet 2,5H_2O$: | | | | | | | | | | | |
|---|---|---|---|---|---|---|---|---|---|---|---|
| | C, | 59.09; | H, | 5.60; | Cl, | 5.62; | N, | 4.44; | S, | 5.08; | Na, | 3.65 |
| Found: | C, | 59.54; | H, | 5.32; | Cl, | 5.84; | N, | 4.58; | S, | 5.93; | Na, | 3.89 |

## EXAMPLE 44

N,N-dimethyl 3-((1-(3-((7-chloro-2-quinolinyl)methoxy)phenyl)-1-(3-((1H-tetrazol-5-yl)methyl)phenyl)methyl)-thio)propanamide, sodium salt

### Step 1

Preparation of 1-(3-((7-chloro-2-quinolinyl)methoxy)phenyl)-1-(3-(((2-methyl-2-propyl)diphenylsilyloxy)-methyl)phenyl)methanol

To the bromide from Example 25, Step 3 (2 g) dissolved in THF (9.7 mL) was added magnesium (128 mg, 1.1 equiv.) followed by few drops of 1,2-dibromoethane. The mixture was warmed to 60° C until most of the magnesium has been consumed. The solution (5.7 mL) was then added to a solution of 3-((7-chloro-2-quinolinyl)methoxy)benzaldehyde (EP 233,763, Example 16, Step 1) (500 mg) in THF (9.7 mL) at 0° C. After 1 hr 25% aqueous ammonium acetate solution was added and the mixture extracted with ethyl acetate. dried and evaporated. The residue was purified by flash chromatography with 3% ethyl acetate in toluene to yield the title compound.

$^1H$ NMR ($CD_3COCD_3$) $\delta$ (ppm): 1.04 (s, 9H), 4.75 (s, 2H), 4.86 (d, 1H), 5.80 (s, 1H), 6.78 to 8.08 (m, 22H), 8.36 (d, 1H).

### Step 2

Preparation of 7-chloro-2-((3-((3-(((2-methyl-2-propyl)diphenylsilyloxy)methyl)phenyl)chloromethyl)phenoxy)-methyl)quinoline

Using the procedure of Example 26, Step 2, the benzyl alcohol of Step 1 was converted to the title compound, which was used as such for the next step.

### Step 3

Preparation of N,N-dimethyl 3-((1-(3-((7-chloro-2-quinolinyl)methoxy)phenyl)-1-(3-(((2-methyl-2-propyl)-diphenylsilyloxy)methyl)phenyl)methyl)thio)propanamide

To the benzyl chloride of Step 2 (1.6 g) dissolved in $CH_3CN$ (10 mL) were added N,N-dimethyl 3-mercaptopropanamide (500 mL, 1.5 equiv.) and $Cs_2CO_3$ (1.6 g). The reaction mixture was stirred at 65° C for 4 hrs then diluted with ethyl acetate and water, and the organic phase was dried and evaporated. The crude mixture was purified by flash chromatography with 25% ethyl acetate in toluene to afford the title compound.

$^1H$ NMR ($CD_3COCD_3$) $\delta$ (ppm): 0.95 (s, 9H), 2.46 (m, 2H), 2.56 (m, 2H), 2.78 and 2.86 (2s, 6H), 3.91 (s, 2H), 5.33 (s, 1H), 5.38 (s, 2H), 6.90 to 8.08 (m, 22H), 8.36 (d, 1H).

### Step 4

Preparation of N,N-dimethyl 3-((1-(3-((7-chloro-2-quinolinyl)methoxy)phenyl)-1-(3-(hydroxymethyl)phenyl)-methyl)thio)propanamide

To the silyl ether (Step 3) (1.4 g) in THF (9 mL) at 0 °C was added a solution of n-tetrabutylammonium fluoride 1 M in THF (3.6 mL, 2. equiv.).

The resulting solution was allowed to warm to room temperature for a few hours. Then 25% aqueous ammonium acetate was added and the product was extracted with ethyl acetate, dried and evaporated. The residue was purified by flash chromatography (50% ethyl acetate in toluene followed by pure acetone) to give the title compound.

$^1H$ NMR ($CD_3COCD_3$) δ (ppm): 2.50 (m, 2H), 2.60 (m, 2H), 2.83 and 2.91 (2s, 6H), 4.16 (t, 1H), 4.56 (d, 2H), 5.35 (s, 1H), 5.43 (s, 2H), 6.91 to 8.08 (m, 12H), 8.40 (d, 1H).

Step 5

Preparation of N,N-dimethyl 3-((1-(3-((7-chloro-2-quinolinyl)methoxy)phenyl)-1-(3-(cyanomethyl)phenyl) methyl)thio)propanamide

To the alcohol (Step 4) (549 mg) in $CH_2Cl_2$ (6 mL) at -78 °C were added triethylamine (293 µL, 2 equiv.) and methanesulfonyl chloride (122 µL, 1.5 equiv.). Then the mixture was allowed to warm to -10 °C and 25% ammonium acetate was added, the mixture was extracted with ethyl acetate, dried and evaporated. The crude mixture was purified by flash chromatography (20% acetone in ethyl acetate) to give the mesylate.

To the mesylate (600 mg) dissolved in DMSO (2.4 mL) at r.t. was added NaCN (240 mg). Then after 4 hr the DMSO was evaporated and the crude mixture was purified by flash chromatography (5 % ethyl acetate in toluene) to afford the title compound.

$^1H$ NMR ($CD_3COCD_3$) δ (ppm): 2.55 (m, 2H). 2.61 (m, 2H) 2.83 and 2.91 (2s, 6H), 3.93 (s 2H), 5.41 (s, 2H), 5.43 (s 1H) 6.91 to 8.05 (m, 12H), 8.38 (d 1H).

Step 6

Using the procedure of Example 40. Step 3, the benzyl nitrile of Step 5 was converted to the title compound (except that the formation of the tetrazole was completed within 4 hrs).

$^1H$ NMR ($CD_3COCD_3$) δ (ppm): 2.46 (m, 2H), 2.55 (m, 2H), 2.76 and 2.80 (2s, 6H), 4.05 (s, 2H), 5.25 (s, 1H), 5.28 (s, 2H), 6.83 to 8.00 (m, 12H), 8.33 (d, 1H).

| Anal. calc'd for $C_{30}H_{28}ClN_6OSNa \bullet 6H_2O$: | | | | | | | | | | | | |
|---|---|---|---|---|---|---|---|---|---|---|---|---|
| | C, | 52.44; | H, | 5.87; | Cl, | 5.16; | N, | 12.23; | S, | 4.67; | Na, | 3.35 |
| Found: | C, | 52.52; | H, | 5.53; | Cl, | 5.33; | N, | 12.14; | S, | 5.20; | Na, | 3.71 |

EXAMPLE 45

3-(((3-Carboxyphenyl)thio)(3-((7-chloro-2-quinolinyl)methoxy)phenyl)methyl)benzoic acid, disodium salt

Step 1

Preparation of methyl3-(((3-(methoxycarbonyl)phenyl)thio)(3-((7-chloro-2-quinolinyl)methoxy)phenyl)methyl) benzoate

To a r.t. solution of the alcohol of Example 9, Step 1 (445 mg) in $CCl_4$ (10 c.c.) and $CH_2Cl_2$ (30 c.c.) was added tri-n-octylphosphine (1.3 g) and the mixture reacted for 1.5 hr. The reaction mixture was filtered through a plug of $SiO_2$ and the intermediate chloride isolated after removal of the solvent. This chloride (155 mg) was taken in $CH_3CN$ (5 c.c.), methyl 3-mercaptobenzoate (84 mg) was added followed by dry cesium carbonate (163 mg) and the mixture was heated at 75° C for 0.5 hr. After cooling, ethyl acetate (10 c.c.) was added and the organic layer was washed with 25% $NH_4OAc$ (5 c.c.), brine, dried with $MgSO_4$ and the solvent removed in vacuo to yield the title compound after purification by chromatography.
$^1H$ NMR ($CDCl_3$) $\delta$ (ppm): 3.70-3.90 (2s, 6H), 5.20 (s, 2H), 5.45 (s, 1H), 6.70-8.05 (m, 17H).

Step 2

To a 0° C solution of the ester of Step 1 (144 mg) in tetrahydrofuran (4 c.c.) and methanol (1 c.c.) was added 2 N NaOH (370 µL) and the mixture was kept at 0° C for 2 days. 25% $NH_4OAc$ (5 c.c.) and acetic acid (3 drops) were added and the product was extracted in ethyl acetate (3x 5 c.c.). The organic layer was washed with brine and the solvents were removed in vacuo. The residue (126 mg) was taken in $H_2O$ (2 c.c.) containing 2 N NaOH (228 µL) and the solution was freeze dried to yield the title compound.
$^1H$ NMR (DMSO-$d_6$ containing 10% $CD_3COCD_3$) $\delta$ (ppm): 5.30 (s, 2H), 5.80 (s, 1H), 6.85-8.50 (m, 17H).

## Claims

1. A compound of the formula:

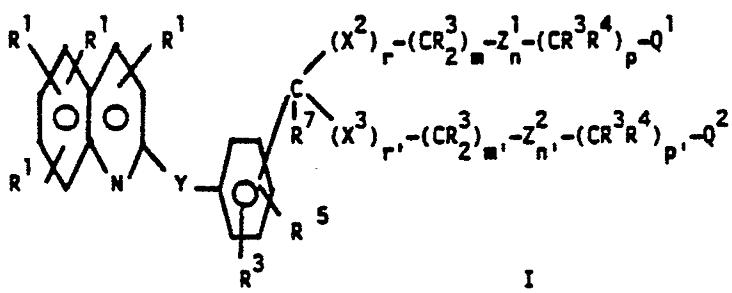

I

wherein:
$R^1$ is H, halogen, $C_1$-$C_8$ alkyl, $C_2$-$C_8$ alkenyl, $C_2$-$C_8$ alkynyl, $-CF_3$, $-SR^2$, $-S(O)R^2$, $-S(O)_2R^2$, $-NR^3R^3$, $-OR^3$, $-COOR^3$, $-(C=O)R^3$, $-C(OH)R^3R^3$, -CN, $-NO_2$, $-N_3$, substituted or unsubstituted phenyl, substituted or unsubstituted benzyl, substituted or unsubstituted 2-phenethyl, or substituted or unsubstituted pyridyl;
$R^2$ is $C_1$-$C_8$ alkyl, $C_2$-$C_8$ alkenyl, $C_2$-$C_8$ alkynyl, $-CF_3$, substituted or unsubstituted phenyl, substituted or unsubstituted benzyl, or substituted or unsubstituted 2-phenethyl;
$R^3$ is H or $R^2$;
$R^4$ is H, halogen, $-NO_2$, -CN, $-OR^3$, $-SR^3$, $NR^3R^3$, or $C_1$-$C_8$ alkyl;
$CR^3R^4$ may be the radical of a naturally occurring amino acid;
$R^5$ is H, halogen, $-NO_2$, $-N_3$, -CN, $-SR^2$, $-NR^3R^3$, $-OR^3$, $C_1$-$C_8$ alkyl or $-(C=O)R^3$;
$R^6$ is $-(CH_2)_s-C(R^7R^7)-(CH_2)_s-R^8$ or $-CH_2CONR^{12}R^{12}$;
$R^7$ is H or $C_1$-$C_4$ alkyl;
$R^8$ is A) a monocyclic or bicyclic heterocyclic radical containing from 3 to 12 nuclear carbon atoms and 1 or 2 nuclear hetero atoms selected from N, S or O and with each ring in the heterocyclic radical being formed of 5 or 6 atoms, or
B) the radical $W-R^9$;
$R^9$ contains up to 21 carbon atoms and is (1) a hydrocarbon radical or (2) an acyl radical of an organic acyclic or monocyclic carboxylic acid containing not more than I heteroatom in the ring;
$R^{10}$ is $-SR^{11}$, $-OR^{12}$, or $-NR^{12}R^{12}$;
$R^{11}$ is $C_1$-$C_6$ alkyl, $-(C=O)R^{14}$, unsubstituted phenyl, or unsubstituted benzyl;
$R^{12}$ is H, $R^{11}$, or two $R^{12}$ groups joined to the same N may form a ring of 5 or 6 members containing up to two heteroatoms chosen from O, S or N;

$R^{13}$ is $C_1$-$C_8$ alkyl, $C_2$-$C_8$ alkenyl, $C_2$-$C_8$ alkynyl, $-CF_3$, or unsubstituted phenyl, benzyl, or 2-phenethyl;
$R^{14}$ is H or $R^{13}$;
$R^{15}$ is $R^3$ or halogen;
$R^{16}$ is H, $C_1$-$C_4$ alkyl, or OH;
m and m′ are independently 0-8;
n and n′ are independently 0 or 1 but not both 0;
p and p′ are independently 0-8;
m + n + p is 1-10 when $X^2$ is O, S, S(O), or $S(O)_2$;
m + n + p is 0-10 when $X^2$ is $CR^3R^{16}$;
m′ + n′ + p′ is 1-10 when $X^3$ is O, S, S(O), or $S(O)_2$; m′ + n′ + p′ is 0-10 when $X^3$ is $CR^3R^{16}$;
r is O or 1 when $Z^1$ is HET ($-R^3$, $-R^5$);
r is 1 when $Z^1$ is $-CONR^3$;
r′ is O or 1 when $Z^2$ is HET($-R^3$, $R^5$);
r′ is 1 when $Z^2$ is $CONR^3$;
s is 0-3;
$Q^1$ and $Q^2$ are independently $-COOR^3$, tetrazole, $-COOR^6$, $-CONHS(O)_2R^{13}$, -CN, $-CONR^{12}R^{12}$, -CHO, $-CH_2OH$, $-COCH_2OH$, $-NHS(O)_2R^{13}$; or if $Q^1$ or $Q^2$ is COOH and $R^4$ is -OH, -SH, or $-NHR^3$ then $Q^1$ or $Q^2$ and $R^4$ and the carbons through which they are attached may form a heterocyclic ring by loss of water;
W is O, S, or $NR^3$;
$X^1$ is O, S, -S(O)-, $-S(O)_2-$, $-NR^3$, or $-CR^3R^3-$;
$X^2$ and $X^3$ are independently O, S, S(O), $S(O)_2$, or $CR^3R^{16}$;
Y is $-CR^3=CR^3-$, -C≡C-, $-CR^3R^3-X^1-$, $-X^1-CR^3R^3-$, $-CR^3R^3-X^1-CR^3R^3-$,

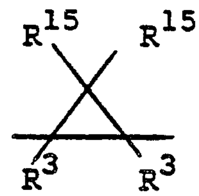,

O O
C=O, $-NR^3-$ C -, - C $-NR^3-$, O, S, or $NR^3$;
$Z^1$ and $Z^2$ are independently $-CONR^3-$ or $-HET(-R^3-R^5)-$provided that at least one of them is $-HET(-R^3-R^5)-$;
HET is

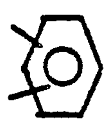, 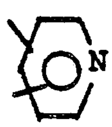, or 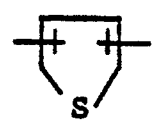;

and the pharmaceutically acceptable salts thereof.

2. A compound of Claim 1 wherein the substituents are as folows:

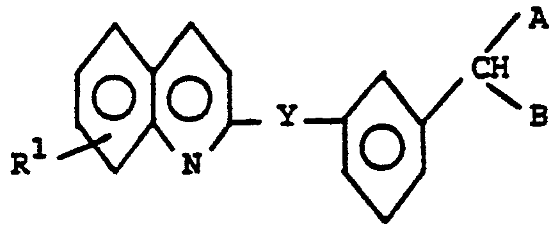

I'

| $R^1$ | Y | A | B |
|---|---|---|---|
| 7-Cl | $-CH=CH-$ | $-S(CH_2)_2CO_2H$ | $-CH_2CH_2$(1,2-Phe)$CO_2H$* |
| 7-Cl | $-CH=CH-$ | $-S(CH_2)_2C(O)N(CH_3)_2$ | $-CH_2CH_2$(1,2-Phe)$CO_2H$ |
| 7-Cl | $-CH_2CH_2-$ | $-S(CH_2)_2C(O)N(CH_3)_2$ | -(1,3-Phe)$CO_2H$ |
| 7-Cl | $-CH=CH-$ | $-S(CH_2)_2C(O)N(CH_3)_2$ | -(2,5-Thio)$CO_2H$ |
| 7-Cl | $-CH=CH-$ | $-S(CH_2)_2C(O)N(CH_3)_2$ | -(1,3-Phe)$CO_2H$ |
| 7-Cl | $-CH=CH-$ | $-S(CH_2)_2C(O)N(CH_3)_2$ | -(1,4-Phe)$CO_2H$ |
| 7-Cl | $-CH=CH-$ | $-S(CH_2)_2CO_2H$ | -(1,3-Phe)$CO_2H$ |
| 7-Cl | $-CH=CH-$ | -S-(1,3-Phe)$CO_2H$ | $-(CH_2)_2CH(CH_3)CH_2CO_2H$ |
| 7-Cl | $-CH_2O-$ | $-S(CH_2)_2C(O)N(CH_3)_2$ | -(1,3-Phe)$CO_2H$ |
| 7-Cl | $-CH=CH-$ | -S(1,3-Phe)$CO_2H$ | -(1,3-Phe)$CO_2H$ |
| 7-Cl | $-CH_2O-$ | $-S(CH_2)_2C(O)NH$-t-Bu | -(1,3-Phe)$CO_2H$ |
| 7-Cl | $-CH_2O-$ | $-S(CH_2)_2C(O)N(CH_3)_2$ | -(3,5-Pye)$CO_2H$ |
| 7-Cl | $-CH=CH-$ | $-S(CH_2)_2C(O)N(CH_3)_2$ | -(3,5-Pye)$CO_2H$ |
| 7-Cl | $-CH_2O-$ | -S-(1,3-Phe)$CO_2H$ | -S-(1,3-Phe)$CO_2H$ |
| 7-Cl | $-CH=CH-$ | -S-(1,3-Phe)$CO_2H$ | -S-(1,3-Phe)$CO_2H$ |
| 7-Cl | $-CH=CH-$ | -S-(1,4-Phe)$CO_2H$ | -S-(1,4-Phe)$CO_2H$ |
| 7-Cl | $-CH=CH-$ | -S-(1,4-Phe)$CO_2H$ | -(2,6-Pye)$CO_2H$ |
| 7-$OCH_3$ | $-CH=CH-$ | $-S(CH_2)_2CO_2H$ | -(1,3-Phe)$CO_2H$ |
| 6-$CF_3$ | $-CH=CH-$ | $-S(CH_2)_2CO_2H$ | -(1,3-Phe)$CO_2H$ |
| 7-$CF_3$ | $-CH=CH-$ | $-S(CH_2)_2CO_2H$ | -(1,4-Phe)$CO_2H$ |
| 6-$SO_2CH_3$ | $-CH=CH-$ | $-S(CH_2)_2CO_2H$ | -(1,3-Phe)$CO_2H$ |
| H | $-CH=CH-$ | $-S(CH_2)_2CO_2H$ | -(1,3-Phe)$CO_2H$ |

| $R^1$ | Y | A | B |
|---|---|---|---|
| 7-Cl | -CH=CH- | $-S(1,4-Phe)CO_2H$ | $-(1,3-Phe)CO_2H$ |
| 7-Cl | -CH=CH- | $-S(1,4-Phe)C(O)N(CH_3)_2$ | $-(1,3-Phe)CO_2H$ |
| 7-Cl | $-CH_2O-$ | $-S(CH_2)_2CO_2H$ | $-CH_2(1,3-Phe)CO_2H$ |
| 7-Cl | -CH=CH- | $-S(4,2-Pye)CO_2H$ | $-(1,3-Phe)CO_2H$ |
| 7-Cl | -CH=CH- | $-S(1,2-Phe)CO_2H$ | $-(1,3-Phe)CO_2H$ |
| 7-Cl | $-CH_2O-$ | $-S(CH_2)_2CO_2H$ | $-(CH_2)_2(1,2-Phe)CO_2H$ |
| 7-Cl | $-CH_2O-$ | $-S(CH_2)_2C(O)NMe_2$ | $-(CH_2)_2(1,2-Phe)CO_2H$ |
| 7-Cl | $-CH_2O-$ | $-S(1,2-Phe)CO_2H$ | $-S(1,2-Phe)CO_2H$ |
| 7-Cl | $-CH_2O-$ | $-(CH_2)_2C(O)N(CH_3)_2$ | $-(1,3-(4-Cl-Phe))CO_2H$ |
| 7-Cl | $-CH_2O-$ | $-SCH_2(1,2-Phe)CO_2H$ | $-SCH_2(1,2-Phe)CO_2H$ |
| 7-Cl | $-CH_2O-$ | $-SCH_2(1,2-Phe)CO_2H$ | $-S(CH_2)_2C(O)N(CH_3)_2$ |
| 7-Cl | -CH=CH- | $-S(CH_2)_3C(O)N(CH_3)_2$ | $-(1,3-Phe)CO_2H$ |
| 7-Cl | $-CH_2O-$ | $-S(CH_2)_2C(O)NH-t-Bu$ | $-(CH_2)_2(1,2-Phe)CO_2H$ |
| 7-Cl | $-CH_2CH_2-$ | $-S(CH_2)_2C(O)NH-t-Bu$ | $-(CH_2)_2(1,2-Phe)CO_2H$ |
| 7-Cl | $-CH_2CH_2-$ | $-S(CH_2)_2C(O)N(CH_3)_2$ | $-(CH_2)_2(1,3-Phe)CO_2H$ |
| 7-Cl | $-CH_2O-$ | $-S(CH_2)_2C(O)N(CH_3)_2$ | $-(1,2-Phe)CO_2H$ |
| 7-Cl | $-CH_2O-$ | $-S(CH_2)_2C(O)NH(1-adamantyl)$ | $-(CH_2)_2(1,2-Phe)CO_2H$ |
| 7-Cl | $-CH_2O-$ | $-S(CH_2)_2C(O)N(CH_3)_2$ | $-(CH_2)_2(1,2-Phe)CN_4H$* |
| 7-Cl | $-CH_2O-$ | $-S(CH_2)_2C(O)N(CH_3)_2$ | $-(CH_2)_2(1,2-Phe)CH_2CN_4H$ |
| 7-Cl | $-CH_2O-$ | $-S(CH_2)_2C(O)N(CH_3)_2$ | $-(CH_2)_2(1,2-Phe)CH_2CO_2H$ |
| 7-Cl | $-CH_2O-$ | $-S(CH_2)_2CO_2H$ | $-(CH_2)_2(1,2-Phe)C(O)N(CH_3)_2$ |
| 7-Cl | $-CH_2O-$ | $-S(CH_2)_2C(O)N(CH_3)_2$ | $-(1,3-Phe)CH_2CN_4H$ |
| 7-Cl | $-CH_2O-$ | $-S(1,3-Phe)CO_2H$ | $-(1,3-Phe)CO_2H$ |
| 7-Cl | $-CH_2O-$ | $-S(CH_2)_2CO_2H$ | $-(1,3-Phe)CH_2C(O)N(CH_3)_2$ |
| 7-Cl | $-CH_2O-$ | $-S(CH_2)_2CO_2H$ | $-(1,3-Phe)CH_2C(O)NH-t-Bu$ |
| 7-Cl | $-CH_2O-$ | $-S(CH_2)_2CN_4H$ | $-(1,3-Phe)CH_2C(O)N(CH_3)_2$ |
| 7-Cl | $-CH_2O-$ | $-S(CH_2)_2CN_4H$ | $-(1,3-Phe)CH_2C(O)NH-t-Bu$ |
| 7-Cl | $-CH_2O-$ | $-SCH_2C(O)N(CH_3)_2$ | $-(1,3-Phe)CH_2CN_4H$ |
| 7-Cl | $-CH_2O-$ | $-SCH_2C(O)NH-t-Bu$ | $-(1,3-Phe)CH_2CN_4H$ |
| 7-Cl | $-CH_2O-$ | $-S(CH_2)_2C(O)N(CH_3)_2$ | $-(1,2-Phe)CH_2CN_4H$ |
| 7-Cl | $-CH_2O-$ | $-S(CH_2)_2C(O)NH-t-Bu$ | $-(1,2-Phe)CH_2CN_4H$ |
| 7-Cl | $-CH_2O-$ | $-SCH_2CO_2H$ | $-(CH_2)_2(1,2-Phe)C(O)N(CH_3)_2$ |
| 7-Cl | $-CH_2O-$ | $-SCH_2CO_2H$ | $-(CH_2)_2(1,2-Phe)C(O)NH-t-Bu$ |
| 7-Cl | $-CH_2O-$ | $-S(CH_2)_2CO_2H$ | $-(CH_2)_2(1,3-Phe)C(O)N(CH_3)_2$ |
| 7-Cl | $-CH_2O-$ | $-S(CH_2)_2CO_2H$ | $-(CH_2)_2(1,3-Phe)C(O)NH-t-Bu$ |
| 7-Cl | $-CH_2O-$ | $-SCH_2CO_2H$ | $-(CH_2)_2(1,3-Phe)C(O)N(CH_3)_2$ |
| 7-Cl | $-CH_2O-$ | $-SCH_2CO_2H$ | $-(CH_2)_2(1,3-Phe)C(O)NH-t-Bu$ |
| 7-Cl | $-CH_2O-$ | $-SCH_2CN_4H$ | $-(CH_2)_2(1,3-Phe)C(O)N(CH_3)_2$ |
| 7-Cl | $-CH_2O-$ | $-SCH_2CN_4H$ | $-(CH_2)_2(1,3-Phe)C(O)NH-t-Bu$ |
| H | $-CH_2O-$ | $-S(CH_2)_2CO_2H$ | $-(CH_2)_2(1,2-Phe)C(O)N(CH_3)_2$ |
| 6,7-diCl | $-CH_2O-$ | $-S(CH_2)_2CO_2H$ | $-(CH_2)_2(1,2-Phe)C(O)N(CH_3)_2$ |
| $7-S(O)_2Me$ | $-CH_2O-$ | $-S(CH_2)_2CO_2H$ | $-(CH_2)_2(1,2-Phe)C(O)N(CH_3)_2$ |
| $6-OCH_3$ | $-CH_2O-$ | $-S(CH_2)_2CO_2H$ | $-(CH_2)_2(1,2-Phe)C(O)N(CH_3)_2$ |
| $6-CH(CH_3)_2$ | $-CH_2O-$ | $-S(CH_2)_2CO_2H$ | $-(CH_2)_2(1,2-Phe)C(O)N(CH_3)_2$ |
| 7-Cl | $-CH_2O-$ | $-S(CH_2)_2CO_2H$ | $-(CH_2)_2(1,2-Phe)C(O)NH-t-Bu$ |
| 7-Cl | $-CH_2O-$ | $-S(CH_2)_2CO_2H$ | $-(CH_2)_2(1,2-Phe)C(O)N(CH_2)_5$* |

| $R^1$ | Y | A | B |
|---|---|---|---|
| 7-Cl | $-CH_2O-$ | $-S(CH_2)_2CO_2H$ | $-(CH_2)_2(1,2\text{-Phe})C(O)N((CH_2)_2O(CH_2)_2)$* |
| 7-Cl | $-CH_2O-$ | $-S(CH_2)_2CO_2H$ | $-(CH_2)_2(1,2\text{-Phe})C(O)NH\text{-}1\text{-adamantyl}$ |
| 7-Cl | $-CH_2O-$ | $-S(CH_2)_2CO_2H$ | $-(CH_2)_2(1,2\text{-Phe})C(O)NHCH_2Ph$* |
| 7-Cl | $-CH_2O-$ | $-S(CH_2)_2CO_2H$ | $-(CH_2)_2(1,2\text{-Phe})C(O)H\text{-}1\text{-naphthyl}$ |
| 7-Cl | $-CH_2O-$ | $-S(CH_2)_2CN_4H$ | $-(CH_2)_2(1,2\text{-Phe})C(O)N(CH_3)_2$ |
| 7-Cl | $-CH_2O-$ | $-S(CH_2)_2CN_4H$ | $-(CH_2)_2(1,2\text{-Phe})C(O)NH\text{-}t\text{-}Bu$ |
| 7-Cl | $-CH_2O-$ | $-SCH_2CH(CH_3)CO_2H$ | $-(CH_2)_2(1,2\text{-Phe})C(O)N(CH_3)_2$ |
| 7-Cl | $-CH_2O-$ | $-SCH_2CH(CH_3)CO_2H$ | $-(CH_2)_2(1,2\text{-Phe})C(O)NH\text{-}t\text{-}Bu$ |
| 7-Cl | $-CH_2O-$ | $-S(CH_2)_2CO_2H$ | $-(CH_2)_2(1,2\text{-}(4\text{-Br-Phe}))C(O)N(CH_3)_2$ |
| 7-Cl | $-CH_2O-$ | $-S(CH_2)_2CO_2H$ | $-(CH_2)_2(1,2\text{-}(4\text{-}SCH_3\text{-Phe}))C(O)N(CH_3)_2$ |
| 7-Cl | $-CH_2O-$ | $-S(CH_2)_2CO_2H$ | $-(CH_2)_2(1,2\text{-}(4\text{-}S(O)_2CH_3\text{-Phe}))(CH_3)_2$ |
| 7-Cl | $-CH_2O-$ | $-S(CH_2)_2CO_2H$ | $-(CH_2)_2(1,2\text{-}(5\text{-Br-Phe}))C(O)N(CH_3)_2$ |
| 7-Cl | $-CH_2O-$ | $-S(CH_2)_2CO_2H$ | $-(CH_2)_2(1,2\text{-}(5\text{-}SCH_3\text{-Phe}))C(O)N(CH_3)_2$ |
| 7-Cl | $-CH_2O-$ | $-S(CH_2)_2CO_2H$ | $-(CH_2)_2(1,2\text{-}(5\text{-}S(O)_2CH_3\text{-Phe}))C(O)N(CH_3)_2$ |
| 7-Cl | $-CH_2O-$ | $-S(CH_2)_2C(O)N(CH_3)_2$ | $-(CH_2)_2(1,2\text{-Phe})C(O)NHS(O)_2CH_3$ |
| 7-Cl | $-CH_2O-$ | $-S(CH_2)_2C(O)NHS(O)_2CF_3$ | $-(CH_2)_2(1,2\text{-Phe})C(O)N(CH_3)_2$ |
| 7-Cl | $-CH_2O-$ | $-S(CH_2)_2C(O)N(CH_3)_2$ | $-(1,3\text{-Phe})CH_2C(O)NHS(O)_2Ph$ |
| 7-Cl | $-CH_2O-$ | $-OCH_2CN_4N$ | $-(CH_2)_2(1,2\text{-Phe})C(O)N(CH_3)_2$ |
| 7-Cl | $-CH_2O-$ | $-OCH_2CO_2H$ | $-(CH_2)_2(1,2\text{-Phe})C(O)N(CH_3)_2$ |
| 7-Cl | $-CH_2O-$ | $-OCH(CH_3)CO_2H$ | $-(CH_2)_2(1,2\text{-Phe})C(O)N(CH_3)_2$ |
| 7-Cl | $-CH_2O-$ | $-O(CH_2)_2C(O)N(CH_3)_2$ | $-(1,3\text{-Phe})CH_2CN_4H$ |
| 7-Cl | $-CH_2O-$ | $-O(CH_2)_2C(O)N(CH_3)_2$ | $-(1,3\text{-Phe})CH(CH_3)CN_4H$ |
| 7-Cl | $-CH_2CH_2-$ | $-S(CH_2)_2CO_2H$ | $-(CH_2)_2(1,2\text{-Phe})C(O)N(CH_3)_2$ |
| 7-Cl | $-CH_2CH_2-$ | $-SCH_2CH(CH_3)CO_2H$ | $-(CH_2)_2(1,2\text{-Phe})C(O)N(CH_3)_2$ |
| 7-Cl | $-CH_2CH_2-$ | $-SCH_2CO_2H$ | $-(CH_2)_2(1,2\text{-Phe})C(O)N(CH_3)_2$ |
| 7-Cl | $-CH_2CH_2-$ | $-SCH(CH_3)CO_2H$ | $-(CH_2)_2(1,2\text{-Phe})C(O)N(CH_3)_2$ |
| 7-Cl | $-CH_2O-$ | $-S(CH_2)_2CO_2H$ | $-(CH_2)_2(1,2\text{-Phe})C(O)N(C_2H_5)_2$ |
| 7-Cl | $-CH_2O-$ | $-S(CH_2)_2CO_2H$ | $-(CH_2)_2(1,2\text{-Phe})C(O)NHCH_3$ |

3. A compound of Claim 2 which is:
2-(3-(3-(2-(7-chloroquinolin-2-yl)ethenyl)phenyl)-3-(2-carboxyethylthio)propyl)benzoic acid, disodium salt;
2-(3-(3-(2-(7-chloroquinolin-2-yl)ethenyl)phenyl)-3-(2-(dimethylcarbamoyl)ethylthio)propyl)benzoic acid, sodium salt;
3-(3-(2-(7-chloroquinolin-2-yl)ethyl)phenyl)-(2-(dimethylcarbamoyl)ethylthio)methyl)benzoic acid, sodium salt;
5-((3-(2-(7-chloroquinolin-2-yl)ethenyl)phenyl)(2-(dimethylcarbamoyl)ethylthio)methyl)thiophene-2-carboxylic acid;
3((3-(2-(7-chloroquinolin-2-yl)ethenyl)phenyl)(2-(dimethylcarbamoyl)ethylthio)methyl)benzoic acid;
4-((3-(2-(7-chloroquinolin-2-yl)ethenyl)phenyl)(2-(dimethylcarbamoyl)ethylthio)methyl)benzoic acid;
3-((2-carboxyethylthio)(3-(2-(7-chloroquinolin 2-yl)ethenyl)phenyl)benzoic acid;
6-(3-carboxyphenylthio)-6-(3-(2-(7-chloroquinolin-2-yl)ethenyl)phenyl)-3-methylhexanoic acid;
3-((3-((7-chloroquinolin-2-ylmethyl)oxy)phenyl)(2-(dimethylcarbamoyl)ethylthio)methyl)benzoic acid, sodium salt;
3-((3-carboxyphenylthio)(3-(2-(7-chloroquinolin-2-yl)ethenyl)phenyl)methyl)benzoic acid;
3-((3-((7-chloroquinolin-2-ylmethyl)oxy)phenyl)(2-(t-butylcarbamoyl)ethylthio)methyl)benzoic acid, sodium salt;
5-((3-((7-chloro-2-quinolinyl)methoxy)phenyl)((3-dimethylamino-3-oxopropyl)thio)methyl)-3-pyridinecarboxylic acid, sodium salt;
3,3′-(((3-((7-Chloro-2-quinolinyl)methoxy)phenyl)methylene)bis(thio))bis(benzoic acid), disodium salt;
3-(((4-Carboxyphenyl)thio)(3-(2-(7-chloro-2-quinolinyl)ethenyl)phenyl)methyl)benzoic acid, disodium salt;
3-((3-(2-(7-Chloro-2-quinolinyl)ethenyl)phenyl)((4-(dimethylaminocarbonyl)phenyl)thio)methyl)benzoic acid, sodium salt;
3-(2-((2-Carboxyethyl)thio)-2-(3-((7-chloro-2-quinolinyl)methoxy)phenyl)ethyl)benzoic acid;
3-(1-(3-(2-(7-chloro-2-quinolinyl)ethenyl)phenyl)-1-((2-carboxy-4-pyridinyl)thio)methyl)benzoic acid;
3-(1-((2-carboxyphenyl)thio)-1-(3-(2-(7-chloro-2-quinolinyl)ethenyl)phenyl)methyl)benzoic acid, disodium salt;

2-(3-(3-((7-chloro-2-quinolinyl)methoxy)phenyl)-3-((2-carboxyethyl)thio)propyl)benzoic acid;
2-(3-(3-((7-chloro-2 quinolinyl)methoxy)phenyl)-3-((3-dimethylamino-3-oxopropyl)thio)propyl)benzoic acid, sodium salt;
2,2′-(((3-((7-chloro-2-quinolinyl)methoxy)phenyl)methylene)bis(thio))bis(benzoic acid), disodium salt;
2-chloro-5-((3-((7-chloro-2-quinolinyl)methoxy)phenyl)((3-dimethylamino-3-oxopropyl)thio)methyl)benzoic acid, sodium salt;
2,2′-(((3-((7-chloro-2-quinolinyl)methoxy)phenyl)methylene)bis(thiomethyl))bis(benzoic acid), disodium salt;
2-((((3-((7-chloro-2-quinolinyl)methoxy)phenyl)((3-dimethylamino-3-oxopropyl)thio)methyl)thio)methyl)benzoic acid;
3-((3-3-(2-(7-Chloro-2-quinolinyl)ethenyl)phenyl)((4-(dimethylamino)-4-oxopropyl)thio)methyl)benzoic acid;
2-(3-((7-chloro-2-quinolinyl)methoxy)phenyl)-3-((3-((2-methyl-2-propyl)amino)-3-oxopropyl)thio)propyl)-benzoic acid, sodium salt;
2-(3-(2-(7-chloro-2-quinolinyl)ethyl)phenyl)-3-((3-((2-methyl-2-propyl)amino)-3-oxopropyl)thio)propyl)benzoic acid;
3(3-(3-(2-(7-chloro-2-quinolinyl)ethyl)phenyl)-3-((3-dimethylamino)-3-oxopropyl)thio)propyl)benzoic acid;
2-((3-((7-chloro-2-quinolinyl)methoxy)phenyl)((3-dimethylamino-3-oxopropyl)thio)methyl)benzoic acid;
2-(3-(3-((7-chloro-2-quinolinyl)methoxy)phenyl)-3-((3-oxo-3-(1-(tricyclo[3.3.1.$1^{3,7}$]decyl)amino)propyl)thio)-propyl)benzoic acid, sodium salt;
N,N-dimethyl-3-((1-(3-((7-chloro-2-quinolinyl)methoxy)phenyl)-3-(2-(1H-tetrazol-5-yl)phenyl)propyl)thio)-propanamide, sodium salt;
N,N-dimethyl-3-((1-(3-((7-chloro-2-quinolinyl)methoxy)phenyl)-3-(2-((1H-tetrazol-5-yl)methyl)phenyl)propyl)-thio)propanamide, sodium salt;
2-(3-(3-((7-Chloro-2-quinolinyl)methoxy)phenyl)-3-((3-dimethylamino-3-oxopropyl)thio)propyl)benzeneacetic acid, sodium salt;
3-((1-(3-((7-Chloro-2-quinolinyl)methoxy)phenyl)-3-(2-(dimethylaminocarbonyl)phenyl)propyl)thio)propanoic acid, sodium salt;
N,N-dimethyl-3-((1-(3-((7-chloro-2-quinolinyl)methoxy)phenyl)-1-(3-((1H-tetrazol-5-yl)methyl)phenyl)methyl)-thio)propanamide, sodium salt; or
3-(((3-Carboxyphenyl)thio)(3-((7-chloro-2-quinolinyl)methoxy)phenyl)methyl)benzoic acid, disodium salt.

4. A compound of Claim 1 wherein:
$R^1$ is H, halogen, $C_1$-$C_8$ alkyl, $-CF_3$, $SR^2$, $-S(O)R^2$, $-S(O)_2R^2$, $-OR^3$, or -CN;
$R^2$ is $C_1$-$C_8$ alkyl or $-CF_3$;
$R^4$ is H, $-OR^3$, $-SR^3$, $NR^3R^3$, or $C_1$-$C_8$ alkyl;
$CR^3R^4$ may be the radical of a naturally occurring amino acid;
$R^5$ is H, halogen, -CN, $-SR^2$, $-OR^3$, $C_1$-$C_8$ alkyl, or $-(C=O)R^3$;
$R^{13}$ is $C_1$-$C_8$ alkyl, $-CF_3$, or unsubstituted phenyl, benzyl, or 2-phenethyl;
m and m′ are independently 0-4;
p and p′ are independently 0-4;
m + n + p is 1-10 when $X^2$ is O or S;
m′ + n′ + p′ is 1-10 when $X^3$ is O or S;
$Q^1$ and $Q^2$ are independently $-COOR^3$, tetrazole, $-COOR^6$, $-CONHS(O)_2R^{13}$, $-CONR^{12}R^{12}$, $-NHS(O)_2R^{13}$; or if $Q^1$ or $Q^2$ is COOH and $R^4$ is -OH, -SH, or $-NHR^3$ then $Q^1$ or $Q^2$ and $R^4$ and the carbons through which they are attached may form a heterocyclic ring by loss of water;
W is O, S, or NH;
$X^1$ is O, S, $-NR^3$, or $-CR^3R^3-$;
$X^2$ and $X^3$ are independently O, S, or $CR^3R^{16}$;
Y is $-CR^3=CR^3-$, $-C\equiv C-$, $-CR^3R^3-X^1-$, or $-X^1-CR^3R^3-$;
and the pharmaceutically acceptable salts thereof.

5. A compound of Claim 1 wherein:
$R^1$ is H, halogen, $C_1$-$C_8$ alkyl, $-CF_3$, $-SR^2$, $-S(O)R^2$, $-S(O)_2R^2$, $-OR^3$, or -CN;
$R^2$ is $C_1$-$C_8$ alkyl or $-CF_3$;
$R^4$ is H, $-OR^3$, $-SR^3$, $NR^3R^3$, or $C_1$-$C_8$ alkyl;
$CR^3R^4$ is not the radical of a naturally occurring amino acid;
$R^5$ is H, halogen, -CN, $-SR^2$, $-OR^3$, $C_1$-$C_8$ alkyl, or $-(C=O)R^3$;
$R^{13}$ is $C_1$-$C_8$ alkyl, $-CF_3$, or unsubstituted phenyl, benzyl, or 2-phenethyl;
m and m′ are independently 0-4;
p and p′ are independently 0-4;
m + n + p is 1-10 when $X^2$ is O or S;

$m' + n' + p$ is 1-10 when $X^3$ is O or S;
$Q^1$ and $Q^2$ are independently $-COOR^3$, tetrazole, or $-CONR^{12}R^{12}$;
W is O, S, or NH;
$X^1$ is O, S, $-NR^3$, or $-CR^3R^3-$;
$X^2$ and $X^3$ are independently O, S, or $CR^3R^{16}$;
Y is -CH=CH-;
$Z^1$ and $Z^2$ are HET($-R^3-R^5$);
and the pharmaceutically acceptable salts thereof.

6. A compound of the formula:

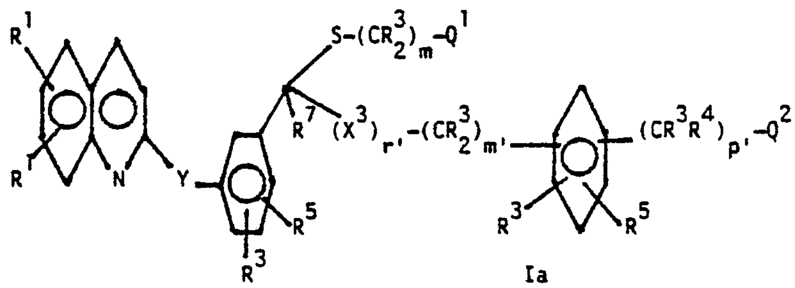

Ia

wherein:
$R^4$ is H or $C_1$-$C_8$ alkyl;
$CR^3R^4$ is not the radical of a naturally occurring amino acid;
m is 1-4;
$m'$ is 0-4;
$p'$ is 0-4;
$r'$ is 0 or 1;
$Q^1$ and $Q^2$ are independently $-COOR^3$, tetrazole, $-CONHS(O)_2R^{13}$, or $-CONR^{12}R^{12}$;
$X^3$ is S or $CR^3R^{16}$;
Y is -CH=CH- or $-CH_2O-$;
and the rest of the definitions are as in Claim 4.

7. A pharmaceutical composition comprising a therapeutically effective amount of a compound of Claim 1 and a pharmaceutically acceptable carrier.

8. The pharmaceutical composition of Claim 7 additionally comprising an effective amount of a second active ingredient selected from the group consisting of non-steroidal anti-inflammatory drugs; peripheral analgesic agents; cyclooxygenase inhibitors; leukotriene antagonists; leukotriene bisynthesis inhibitors; $H_2$-receptor antagonists; antihistaminic agents; prostaglandin antagonists; thromboxane antagonists; thromboxane synthetase inhibitors; and ACE antagonists.

9. A pharmaceutical composition according to Claim 8, wherein the second active ingredient is a non-steroidal anti-inflammatory drug.

10. A pharmaceutical composition of Claim 9, wherein the weight ratio of said compound of Claim 1 to said second active ingredient ranges from about 1000:1 to 1:1000.

11. The use of a compound of Claim 1 for the preparation of a medicament useful for preventing the synthesis, the action, or the release of SRS-A or leukotrienes in a mammal.

12. The use of a compound of Claim 1 for the preparation of a medicament useful for inducing cytoprotection in a mammal.

13. The use of a compound of Claim 1 for the preparation of a medicament useful for treating inflammatory diseases of the eye.